# EUROPÄISCHE PATENTSCHRIFT

(11) **EP 0 831 705 B1**
(45) Veröffentlichungstag und Bekanntmachung des Hinweises auf die Patenterteilung: **29.08.2001**
(21) Anmeldenummer: 96918634.5
(22) Anmeldetag: 28.05.1996
(51) Int. Cl.: A01N 43/90, A01N 43/653, C07D 249/10, C07D 401/04

(54) **N-ARYL-1,2,4-TRIAZOLIN-5-ONE**
N-ARYL-1,2,4-TRIAZOLIN-5-ONES
N-ARYLE-1,2,4-TRIAZOLIN-5-ONES

(30) Priorität: 09.06.1995 DE 19521162
(43) Veröffentlichungstag der Anmeldung: 01.04.1998
(73) Patentinhaber: BAYER AG, 51368 Leverkusen (DE)
(72) Erfinder: LINKER, Karl-Heinz, D-51377 Leverkusen (DE); FINDEISEN, Kurt, D-51375 Leverkusen (DE); HAAS, Wilhelm, D-50259 Pulheim (DE); LENDER, Andreas, D-42327 Wuppertal (DE); MÜLLER, Klaus-Helmut, D-40593 Düsseldorf (DE); SCHALLNER, Otto, D-40789 Monheim (DE); ERDELEN, Christoph, D-42799 Leichlingen (DE); TURBERG, Andreas, D-40699 Erkrath (DE); MENCKE, Norbert, D-51381 Leverkusen (DE)
(86) Internationale Anmeldenummer: EP9602287
(87) Internationale Veröffentlichungsnummer: WO9641535

(56) Entgegenhaltungen:
- EP-A- 0 000 014
- EP-A- 0 597 360
- EP-A- 0 610 733
- EP-A- 0 617 026
- DE-A- 2 724 819

## Beschreibung

Die vorliegende Erfindung betrifft neue N-Aryl-1,2,4-triazolin-5-one, Verfahren zu deren Herstellung und deren Verwendung zur Bekämpfung von tierischen Schädlingen, insbesondere von Insekten, Spinnentieren und Nematoden, die in der Landwirtschaft, in Forsten, im Vorrats- und Materialschutz sowie auf dem Hygienesektor vorkommen.

Es sind bereits bestimmte N-Aryl-1,2,4-triazolin-5-on-Derivate bekannt geworden (vgl. CA 71, 1969, 217 70g und CA 94, 1981, 121 413m). Ein Einsatz dieser Stoffe zur Bekämpfung von Schädlingen wird jedoch nicht beschrieben.

Weiterhin ist bereits bekannt, daß bestimmte substituierte N-Aryl-1,2,4-triazolin-5-on-Derivate herbizide Eigenschaften aufweisen (vgl. z.B. EP-A-0 610 733 und EP-A-0 617 026). Über eine insektizide Wirkung der aus der EP-A-0 617 026 bekannten Verbindungen ist nichts bekannt. Die aus der EP-A-0 610 733 bekannten Verbindungen sollen in entsprechenden Aufwandmengen eine blattinsektizide und akarizide Wirksamkeit aufweisen. Die insektizide Wirkung dieser Verbindungen ist jedoch, insbesondere bei niedrigen Aufwandmengen und Konzentrationen nicht immer zufriedenstellend.

Es wurden nun neue N-Aryl-1,2,4-triazolin-5-one der Formel (Ia) gefunden, 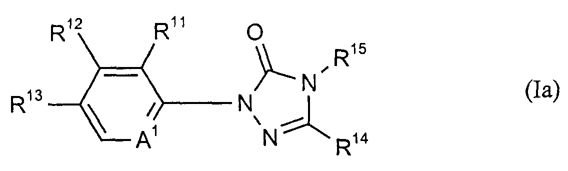 in welcher
- A¹: für Stickstoff oder die Gruppe CR^{O} steht,
- R^{O}: für Wasserstoff, Halogen, Nitro, Cyano, Hydroxy, gegebenenfalls durch Halogen, Cyano, Nitro, Alkoxy oder Amino substituiertes Alkyl, gegebenenfalls substituiertes Cycloalkyl oder den Rest -CX¹-NY¹¹Y¹² steht,
- R¹¹: für Halogen, Nitro, Cyano, jeweils gegebenenfalls durch Halogen, Cyano, Nitro, Alkoxy oder Amino substituiertes Alkyl oder Alkoxy, gegebenenfalls substituiertes Cycloalkyl oder den Rest -CX¹-NY¹¹Y¹² steht,
- R¹²: für Wasserstoff, Halogen, gegebenenfalls durch Halogen, Cyano, Nitro, Alkoxy oder Amino substituiertes Alkyl oder gegebenenfalls substituiertes Cycloalkyl steht,
- R¹³: für Halogenalkyl, Halogenalkoxy oder den Rest -S(O)ₙ¹ R¹⁶ steht,
- R¹⁴: für Halogen, Cyano, jeweils gegebenenfalls durch Halogen, Cyano, Nitro, Alkoxy oder Amino substituiertes Alkyl oder Alkoxy, für Alkenyl, Alkenyloxy, Alkinyl, Hydroxy, Mercapto, jeweils gegebenenfalls substituiertes Cycloalkyl oder Cycloalkylalkyl, jeweils gegebenenfalls substituiertes Aryl, Aryloxyalkyl oder Aralkyl oder den Rest -S(O)ₙ¹R¹⁶ steht,
- R¹⁵: für Wasserstoff, Cyano, Amino, jeweils gegebenenfalls durch Halogen, Cyano, Nitro, Alkoxy oder Amino substituiertes Alkyl oder Alkoxy, für Alkenyl, Alkenyloxy, Alkinyl, Alkinyloxy, jeweils gegebenenfalls substituiertes Cycloalkyl oder Cycloalkylalkyl, jeweils gegebenenfalls subsituiertes Aryl oder Aralkyl oder einen der Reste -S(O)ₙ¹R¹⁶, -NR¹⁷R¹⁸ oder -N=CR¹⁹R²⁰ steht,
- R¹⁶: für gegebenenfalls durch Halogen, Cyano, Nitro, Alkoxy oder Amino substituiertes Alkyl, für Alkenyl, Alkandienyl, Alkinyl, gegebenenfalls substituiertes Cycloalkyl oder gegebenenfalls substituiertes Aryl steht,
- R¹⁷ und R¹⁸: gemeinsam mit dem Stickstoffatom, an das sie gebunden sind, für einen gegebenenfalls substituierten Heterocyclus stehen, der gegebenenfalls ein oder mehrere weitere Heteroatome enthält,
- R¹⁹: für Wasserstoff, gegebenenfalls durch Halogen, Cyano, Nitro, Alkoxy oder Amino substituiertes Alkyl, für Alkoxy, gegebenenfalls substituiertes Cycloalkyl oder jeweils gegebenenfalls subsituiertes Aryl oder Heterocyclyl steht,
- R²⁰: für Wasserstoff, gegebenenfalls durch Halogen, Cyano, Nitro, Alkoxy oder Amino substituiertes Alkyl, für Alkoxy, gegebenenfalls substituiertes Cycloalkyl oder jeweils gegebenenfalls subsituiertes Aryl oder Heterocyclyl steht,
- X¹: für Sauerstoff oder Schwefel steht,
- Y¹¹: für Wasserstoff oder Alkyl steht,
- Y¹²: für Wasserstoff oder Alkyl steht und
- n¹: für eine der Zahlen 0, 1 oder 2 steht,
mit Ausnahme der Verbindungen

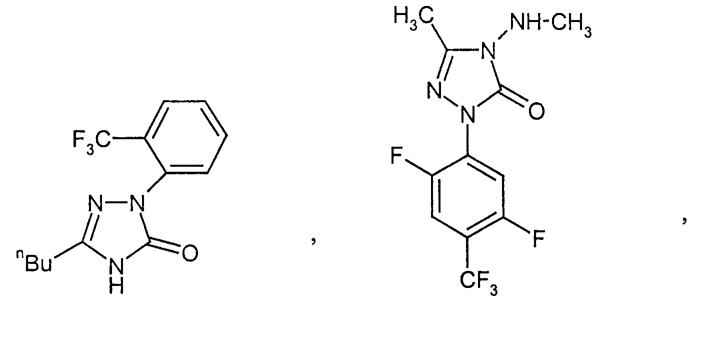

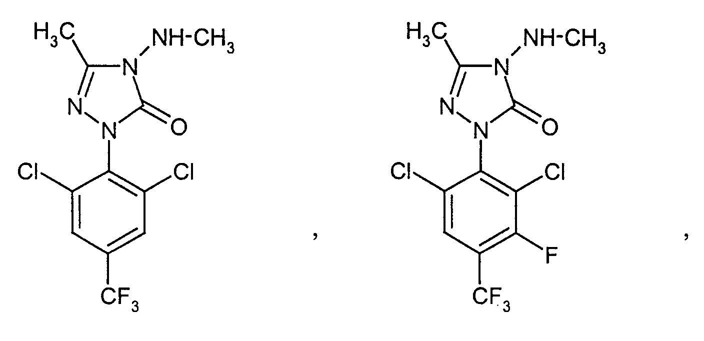

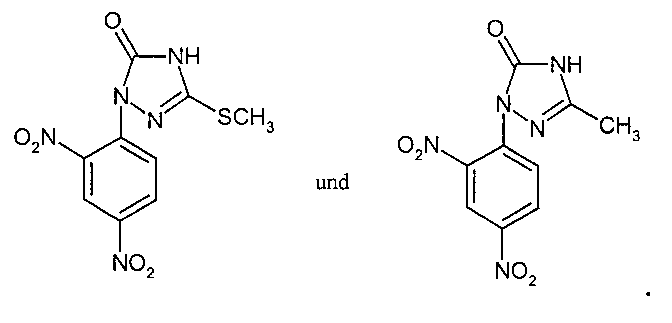

Die Verbindungen der Formel (Ia) können in Abhängigkeit von der Art der Substituenten als geometrische und/oder optische Isomere oder Isomerengemische unterschiedlicher Zusammensetzung vorliegen. Die Erfindung betrifft sowohl die reinen Isomeren als auch die Isomerengemische.

Weiterhin wurde gefunden, daß man
a) N-Aryl-1,2,4-triazolin-5-one der Formel (Ia) 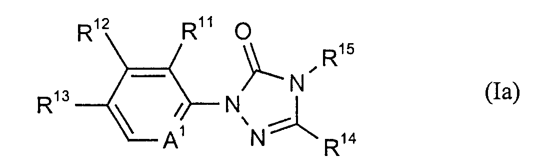 in welcher
   - R¹¹, R¹², R¹³, R¹⁴, R¹⁵ und A¹: die oben angegebene Bedeutung haben,
   erhält, wenn man
   1H-Triazolinone der Formel (II) 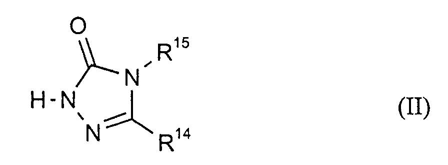 in welcher
   - R¹⁴ und R¹⁵: die oben angegebene Bedeutung haben,
   mit Halogen-Derivaten der Formel (III) 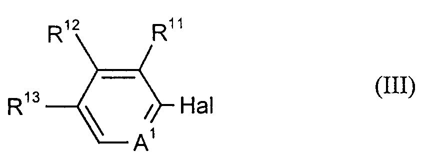 in welcher
   - R¹¹, R¹², R¹³ und A¹: die oben angegebene Bedeutung haben und
   - Hal: für Halogen steht,
   gegebenenfalls in Gegenwart eines Verdünnungsmittels und gegebenenfalls
   in Gegenwart eines Reaktionshilfsmittels umsetzt;
   oder
b) N-Aryl-1,2,4-triazolin-5-one der Formel (Ib) 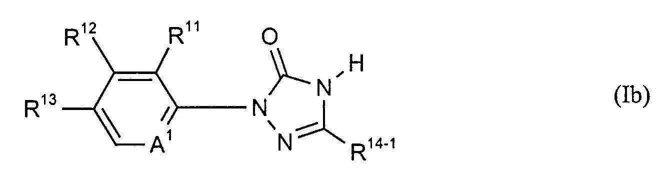 in welcher
   - R¹¹, R¹², R¹³ und A¹: die oben angegebene Bedeutung haben und
   - R¹⁴⁻¹: für Cyano, jeweils gegebenenfalls durch Halogen, Cyano, Nitro, Alkoxy oder Amino substituiertes Alkyl oder Alkoxy, für Alkenyl, Alkenyloxy, Alkinyl, jeweils gegebenenfalls substituiertes Cycloalkyl oder Cycloalkylalkyl oder jeweils gegebenenfalls substituiertes Aryl, Aryloxyalkyl oder Aralkyl steht,
   erhält, wenn man
   Hydrazin-Derivate der Formel (IV) 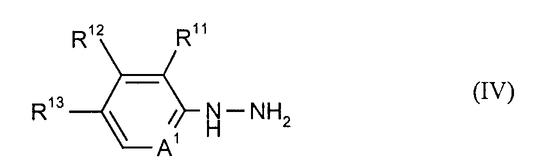 in welcher
   - R¹¹, R¹², R¹³ und A¹: die oben angegebene Bedeutung haben,
   mit Iminocarbonestern der Formel (V) 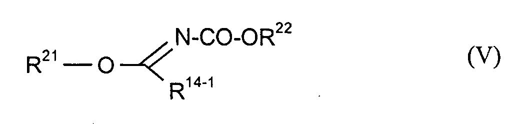 in welcher
   - R¹⁴⁻¹: die oben angegebene Bedeutung hat und
   - R²¹ und R²²: unabhängig voneinander für Alkyl (bevorzugt C₁-C₈-Alkyl) stehen,
   in Gegenwart eines Verdünnungsmittels umsetzt;
   oder
c) N-Aryl-1,2,4-triazolin-5-one der Formel (Ic) 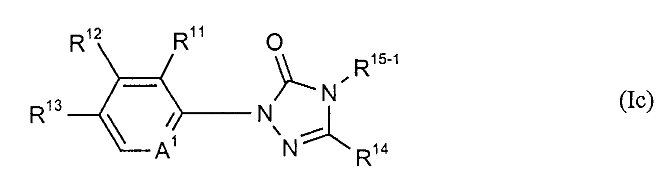 in welcher
   - R¹¹, R¹², R¹³, R¹⁴ und A¹: die oben angegebene Bedeutung haben und
   - R¹⁵⁻¹: für Cyano, gegebenenfalls durch Halogen, Cyano, Nitro, Alkoxy oder Amino substituiertes Alkyl, für Alkenyl, Alkinyl, jeweils gegebenenfalls subsituiertes Cycloalkyl und Cycloalkylalkyl oder gegebenenfalls substituiertes Aryl steht,
   erhält, wenn man
   N-Aryl-1,2,4-triazolin-5-one der Formel (Id) 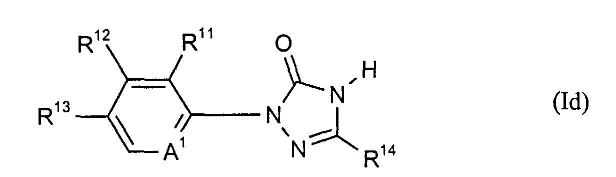 in welcher
   - R¹¹, R¹², R¹³, R¹⁴ und A¹: die oben angegebene Bedeutung haben,
   mit Alkylierungsmitteln der Formel (VI)

   E-R¹⁵⁻¹ (VI)

   in welcher
   - R¹⁵⁻¹: die oben angegebene Bedeutung hat und
   - E: für eine elektronenanziehende Abgangsgruppe steht,
   gegebenenfalls in Gegenwart eines Verdünnungsmittels und gegebenenfalls in Gegenwart eines Reaktionshilfsmittels umsetzt.

Die erfindungsgemäßen neuen N-Aryl-1,2,4-triazolin-5-one sind durch die Formel (Ia) allgemein definiert.

Bevorzugte Substituenten bzw. Bereiche der in den oben und nachstehend erwähnten Formeln aufgeführten Reste werden im folgenden erläutert.
- A¹: steht bevorzugt für Stickstoff oder die Gruppe CR^{O}.
- R^{O}: steht bevorzugt für Wasserstoff, Fluor, Chlor, Brom, Nitro, Cyano, Hydroxy, gegebenenfalls durch Fluor, Chlor, Cyano, Nitro oder C₁-C₂-Alkoxy substituiertes C₁-C₄-Alkyl, gegebenenfalls durch Fluor, Chlor, Brom, C₁-C₄-Alkyl, C₁-C₄-Alkoxy, C₁-C₄-Halogenalkyl oder C₁-C₄-Halogenalkoxy substituiertes C₃-C₆-Cycloalkyl, oder für einen der Reste -CONH₂, -CSNH₂, - CO-NH-C₁-C₄-Alkyl, -CS-NH-C₁-C₄-Alkyl, -CO-N(C₁-C₄-Alkyl)₂ oder -CS-N(C₁-C₄-Alkyl)₂.
- R¹¹: steht bevorzugt für Fluor, Chlor, Brom, Nitro, Cyano, jeweils gegebenenfalls durch Fluor, Chlor, Cyano, Nitro oder C₁-C₂-Alkoxy substituiertes C₁-C₄-Alkyl oder C₁-C₄-Alkoxy, gegebenenfalls durch Fluor, Chlor, Brom, C₁-C₄-Alkyl, C₁-C₄-Alkoxy, C₁-C₄-Halogenalkyl oder C₁-C₄-Halogenalkoxy substituiertes C₃-C₆-Cycloalkyl, oder für einen der Reste -CONH₂, -CSNH₂, -CO-NH-C₁-C₄-Alkyl, -CS-NH-C₁-C₄-Alkyl, -CO-N(C₁-C₄-Alkyl)₂ oder -CS-N(C₁-C₄-Alkyl)₂.
- R¹²: steht bevorzugt für Wasserstoff, Fluor, Chlor, C₁-C₄-Alkyl oder C₁-C₄-Halogenalkyl.
- R¹³: steht bevorzugt für C₁-C₄-Halogenalkyl, C₁-C₄-Halogenalkoxy oder für einen der Reste -S-C₁-C₄-Halogenalkyl, -SO-C₁-C₄-Halogenalkyl oder -SO₂-C₁-C₄-Halogenalkyl.
- R¹⁴: steht bevorzugt für Halogen, Cyano, jeweils gegebenenfalls durch Fluor, Chlor, Cyano, Nitro, C₁-C₄-Alkoxy oder Amino substituiertes C₁-C₄-Alkyl oder C₁-C₄-Alkoxy, für C₃-C₆-Alkenyl, C₃-C₆-Alkenyloxy, C₃-C₆-Alkinyl, Hydroxy, Mercapto, jeweils gegebenenfalls durch Fluor, Chlor, Brom, C₁-C₄-Alkyl, C₁-C₄-Alkoxy, C₁-C₄-Halogenalkyl oder C₁-C₄-Halogenalkoxy substituiertes C₃-C₆-Cycloalkyl oder C₃-C₆-Cycloalkyl-C₁-C₄-alkyl, jeweils gegebenenfalls durch Halogen, Cyano, Nitro, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio oder gegebenenfalls durch Halogen oder C₁-C₄-Alkyl substituiertes Phenyl substituiertes Phenyl, Phenyloxy-C₁-C₄-alkyl oder Phenyl-C₁-C₄-alkyl oder für den Rest -S(O)ₙ¹R¹⁶.
- R¹⁵: steht bevorzugt für Wasserstoff, Cyano, Amino, jeweils gegebenenfalls durch Fluor, Chlor, Cyano, Nitro, C₁-C₄-Alkoxy oder Amino substituiertes C₁-C₄-Alkyl oder C₁-C₄-Alkoxy, für C₃-C₆-Alkenyl, C₃-C₆-Alkenyloxy, C₃-C₆-Alkinyl, C₃-C₆-Alkinyloxy, jeweils gegebenenfalls durch Fluor, Chlor, Brom, C₁-C₄-Alkyl, C₁-C₄-Alkoxy, C₁-C₄-Halogenalkyl oder C₁-C₄-Halogenalkoxy substituiertes C₃-C₆-Cycloalkyl oder C₃-C₆-Cycloalkyl-C₁-C₄-alkyl, gegebenenfalls durch Halogen, Cyano, Nitro, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio oder gegebenenfalls durch Halogen oder C₁-C₄-Alkyl substituiertes Phenyl substituiertes Phenyl, gegebenenfalls durch C₁-C₄-Alkyl substituiertes Benzyl oder für einen der Reste -S(O)ₙ¹R¹⁶, -NR¹⁷R¹⁸ oder -N=CR¹⁹R²⁰.
- R¹⁶: steht bevorzugt für gegebenenfalls durch Fluor, Chlor, Cyano oder Nitro substituiertes C₁-C₄-Alkyl, für C₃-C₆-Alkenyl, C₃-C₆-Alkandienyl, C₃-C₆-Alkinyl, gegebenenfalls durch Fluor, Chlor, Brom, C₁-C₄-Alkyl, C₁-C₄-Alkoxy, C₁-C₄-Halogenalkyl oder C₁-C₄-Halogenalkoxy substituiertes C₃-C₆-Cycloalkyl, oder gegebenenfalls durch Halogen, Cyano, Nitro, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio oder gegebenenfalls durch Halogen oder C₁-C₄-Alkyl substituiertes Phenyl substituiertes Phenyl.
- n¹: steht bevorzugt für eine der Zahlen 0, 1 oder 2.
- R¹⁷ und R¹⁸: stehen gemeinsam bevorzugt mit dem Stickstoffatom, an das sie gebunden sind, für einen gegebenenfalls durch Halogen, C₁-C₄-Alkyl, C₁-C₄-Alkoxy oder C₁-C₄-Alkylthio substituierten, gesättigten fünf- bis siebengliedrigen Heterocyclus mit zusätzlich zum Stickstoffatom 2 oder 3 gleichen oder verschiedenen Heteroatomen aus der Reihe Stickstoff, Sauerstoff und Schwefel.
- R¹⁹: steht bevorzugt für Wasserstoff, gegebenenfalls durch Fluor, Chlor, Cyano, Nitro, Amino oder C₁-C₄-Alkoxy substituiertes C₁-C₄-Alkyl oder für C₁-C₄-Alkoxy.
- R²⁰: steht bevorzugt für Wasserstoff, gegebenenfalls durch Fluor, Chlor, Cyano, Nitro, Amino oder C₁-C₄-Alkoxy substituiertes C₁-C₄-Alkyl oder für C₁-C₄-Alkoxy,
mit Ausnahme der Verbindungen 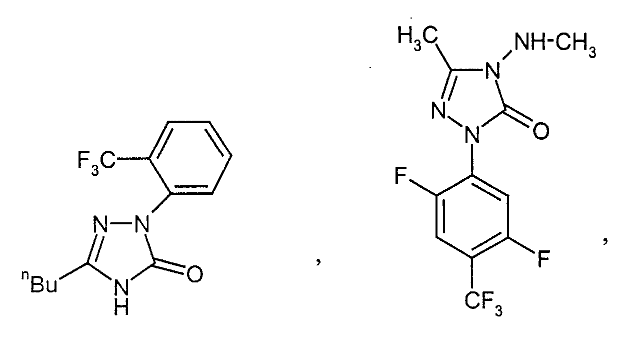 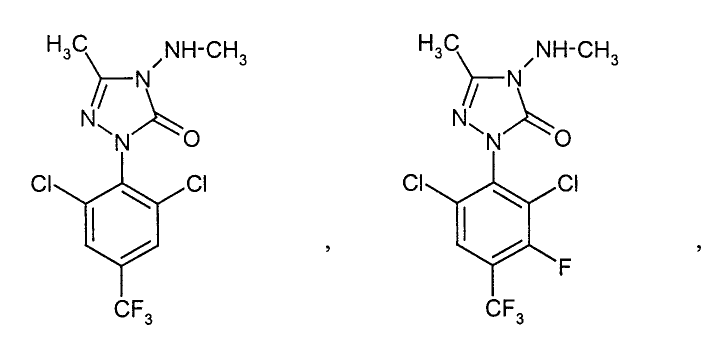 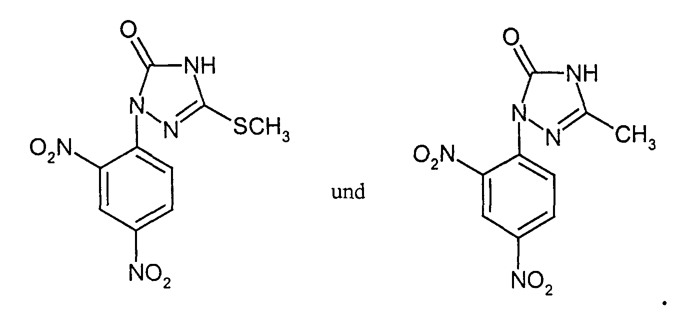
- A¹: steht besonders bevorzugt für Stickstoff oder die Gruppe CR^{O}.
- R^{O}: steht besonders bevorzugt für Wasserstoff, Fluor, Chlor, Brom, Nitro, Cyano, Hydroxy, Methyl, Ethyl, n- oder i-Propyl, Methoxymethyl, Trifluormethyl, Difluormethyl, Chlordifluormethyl, Dichlorfluormethyl oder für einen der Reste -CONH₂, -CSNH₂, -CONHCH₃, -CSNHCH₃, -CON(CH₃)₂ oder -CSN(CH₃)₂.
- R¹¹: steht besonders bevorzugt für Fluor, Chlor, Brom, Nitro, Cyano, Methyl, Ethyl, n- oder i-Propyl, Methoxymethyl, Trifluormethyl, Difluormethyl, Chlordifluormethyl, Dichlorfluormethyl, Trifluormethoxy, Difluormethoxy, Trifluorethoxy oder für einen der Reste -CONH₂, -CSNH₂, -CONHCH₃, -CSNHCH₃, -CON(CH₃)₂ und -CSN(CH₃)₂.
- R¹²: steht besonders bevorzugt für Wasserstoff, Fluor, Chlor, Methyl oder Trifluormethyl.
- R¹³: steht besonders bevorzugt für Trifluormethyl, Difluormethyl, Chlordifluormethyl, Dichlorfluormethyl, Trifluormethoxy, Difluormethoxy, Chlordifluormethoxy, Trifluormethylsulfenyl, Trifluormethylsulfinyl oder Trifluormethylsulfonyl.
- R¹⁴: steht besonders bevorzugt für Chlor, Brom, Cyano, Methyl, Ethyl, n- oder i-Propyl, n-, i-, s- oder t-Butyl, Methoxy, Ethoxy, n- oder i-Propoxy, n-, i-, s-oder t-Butoxy, Methoxymethyl, Methoxyethyl, Ethoxymethyl, Ethoxyethyl, Trifluormethyl, Difluormethyl, Chlordifluormethyl, Dichlorfluormethyl, Trifluorethyl, Tetrafluorethyl, Pentafluorethyl, Fluorpropyl, Trifluormethoxy, Difluormethoxy, Chlordifluormethoxy, Dichlorfluormethoxy, Trifluorethoxy, Tetrafluorethoxy, Pentafluorethoxy, Cyanmethyl, Allyl, Allyloxy, Propargyl, Hydroxy, Mercapto, jeweils gegebenenfalls durch Fluor, Chlor, Brom, Methyl, Ethyl, Methoxy, Trifluormethyl oder Trifluormethoxy substituiertes Cyclopropyl, Cyclopropylmethyl, Cyclopentyl, Cyclopentylmethyl, Cyclohexyl oder Cyclohexylmethyl, jeweils gegebenenfalls durch Fluor, Chlor, Methyl, Methoxy, Trifluormethyl, Trifluormethoxy, Methylthio, Trifluormethylthio, Nitro oder gegebenenfalls durch Fluor, Chlor oder Methyl substituiertes Phenyl substituiertes Phenyl, Phenoxymethyl oder Benzyl oder für den Rest -S(O)ₙ¹R¹⁶.
- R¹⁵: steht besonders bevorzugt für Wasserstoff, Cyano, Amino, Methyl, Ethyl, n-oder i-Propyl, n-, i-, s- oder t-Butyl, Methoxy, Ethoxy, n- oder i-Propoxy, n-, i-, s- oder t-Butoxy, Methoxymethyl, Methoxyethyl, Ethoxymethyl, Ethoxyethyl, Difluormethyl, Chlordifluormethyl, Dichlorfluormethyl, Trifluorethyl, Fluorpropyl, Difluormethoxy, Chlordifluormethoxy, Dichlorfluormethoxy, Cyanmethyl, Allyl, Allyloxy, Propargyl, Propargyloxy, Butinyloxy, jeweils gegebenenfalls durch Fluor, Chlor, Brom, Methyl, Ethyl, Methoxy, Trifluormethyl oder Trifluormethoxy substituiertes Cyclopropyl, Cyclopropylmethyl, Cyclopentyl, Cyclopentylmethyl, Cyclohexyl oder Cyclohexylmethyl, gegebenenfalls durch Fluor, Chlor, Methyl, Methoxy, Trifluormethyl, Trifluormethoxy, Methylthio, Trifluormethylthio, Cyano, Nitro sowie gegebenenfalls durch Fluor, Chlor oder Methyl substituiertes Phenyl substituiertes Phenyl, für gegebenenfalls durch Methyl, Ethyl, n- oder i-Propyl, n-, i-, s- oder t-Butyl substituiertes Benzyl oder für einen der Reste -S(O)ₙ¹R¹⁶, -NR¹⁷R¹⁸ oder -N=CR¹⁹R²⁰.
- R¹⁶: steht besonders bevorzugt für Methyl, Ethyl, n- oder i-Propyl, n-, i-, s- oder t-Butyl, Trifluormethyl, Difluormethyl, Chlordifluormethyl, Dichlorfluormethyl, Trifluorethyl, Tetrafluorethyl, Pentafluorethyl, Fluorpropyl, Allyl, Allenyl, Propargyl, jeweils gegebenenfalls durch Fluor, Chlor, Brom, Methyl, Ethyl, Methoxy, Trifluormethyl oder Trifluormethoxy substituiertes Cyclopropyl, Cyclopentyl oder Cyclohexyl, oder gegebenenfalls durch Fluor, Chlor, Methyl, Methoxy, Trifluormethyl, Trifluormethoxy, Methylthio, Trifluormethylthio, Cyano, Nitro oder gegebenenfalls durch Fluor, Chlor oder Methyl substituiertes Phenyl substituiertes Phenyl.
- n¹: steht besonders bevorzugt für eine der Zahlen 0, 1 oder 2.
- R¹⁷ und R¹⁸: stehen besonders bevorzugt mit dem Stickstoffatom, an das sie gebunden sind, für jeweils gegebenenfalls durch Methyl, Ethyl, Methoxy oder Ethoxy substituiertes Piperidinyl, Piperazinyl oder Morpholinyl.
- R¹⁹: steht besonders bevorzugt für Wasserstoff, Methyl, Ethyl, n- oder i-Propyl, n-, i-, s- oder t-Butyl, Methoxy oder Ethoxy.
- R²⁰: steht besonders bevorzugt für Wasserstoff, Methyl, Ethyl, n- oder i-Propyl, n-, i-, s- oder t-Butyl, Methoxy oder Ethoxy,
mit Ausnahme der Verbindungen

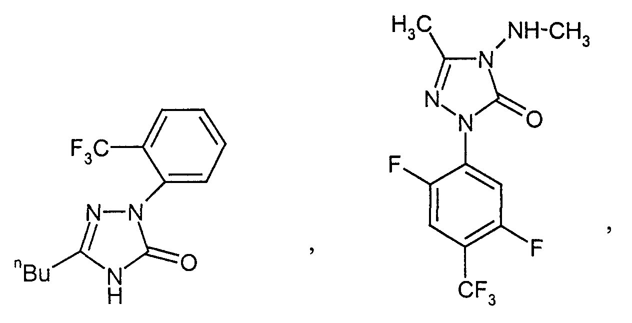

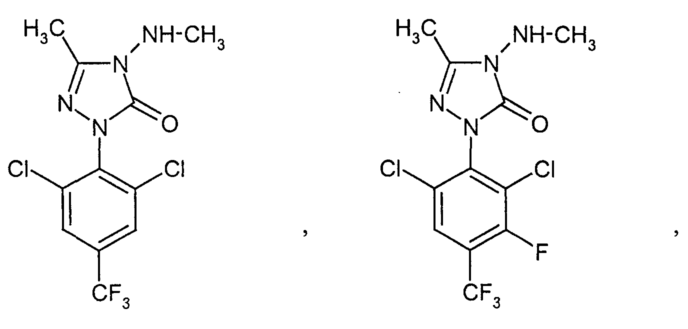

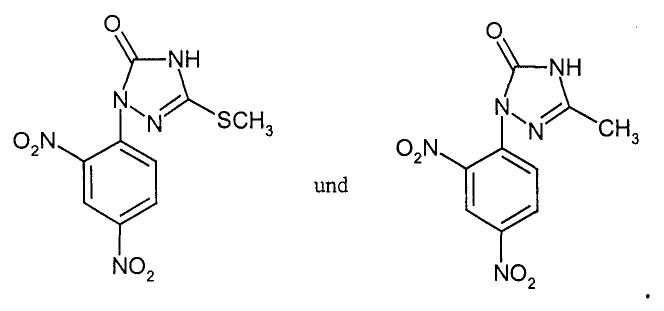

Die oben aufgeführten allgemeinen oder in Vorzugsbereichen aufgeführten Restedefinitionen bzw. Erläuterungen gelten für die Endprodukte und für die Ausgangs- und Zwischenprodukte entsprechend. Diese Restedefinitionen können untereinander, also auch zwischen den jeweiligen Vorzugsbereichen, beliebig kombiniert werden.

Erfindungsgemäß bevorzugt werden die Verbindungen der Formel (Ia), in welchen eine Kombination der vorstehend als bevorzugt (vorzugsweise) aufgeführten Bedeutungen vorliegt.

Erfindungsgemäß besonders bevorzugt werden die Verbindungen der Formel (Ia), in welchen eine Kombination der vorstehend als besonders bevorzugt aufgeführten Bedeutungen vorliegt.

In den oben und nachstehend aufgeführten Restedefinitionen sind Kohlenwasserstoffreste, wie Alkyl oder Alkenyl - auch in Verbindungen mit Heteroatomen wie Alkoxy oder Alylthio - soweit möglich jeweils geradkettig oder verzweigt.

Beispiele für die neuen erfindungsgemäßen Verbindungen sind in den Tabellen 1a bis 80d aufgeführt:

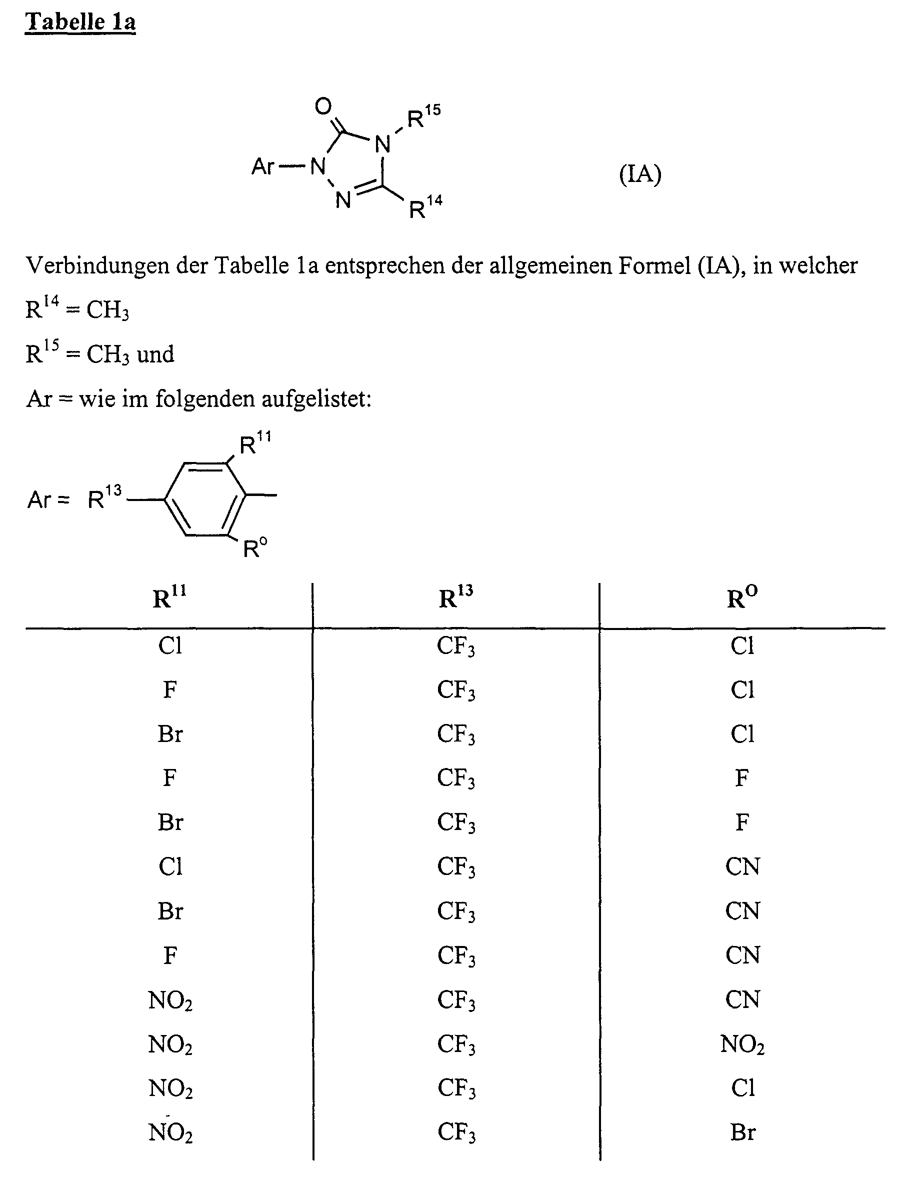

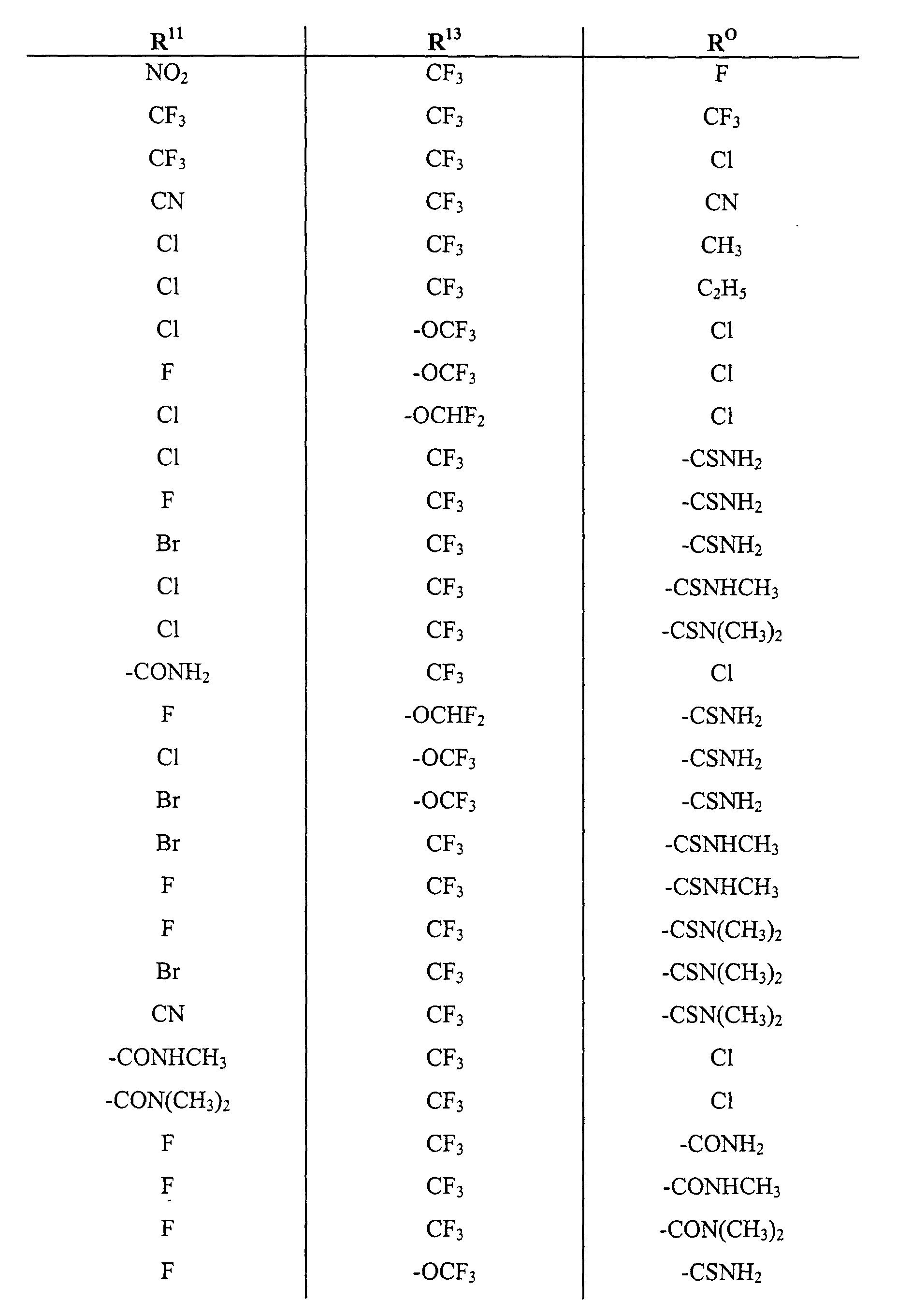

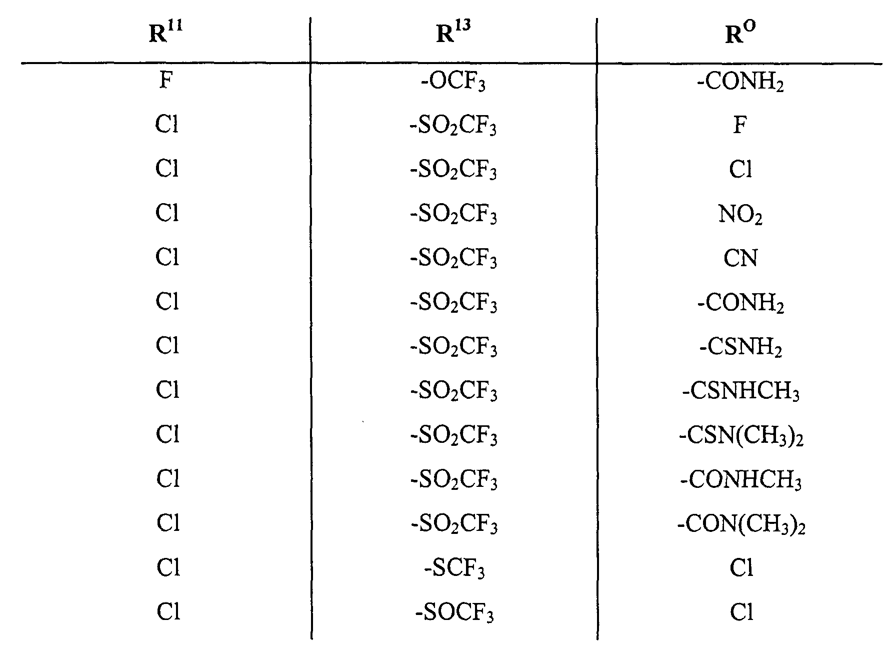

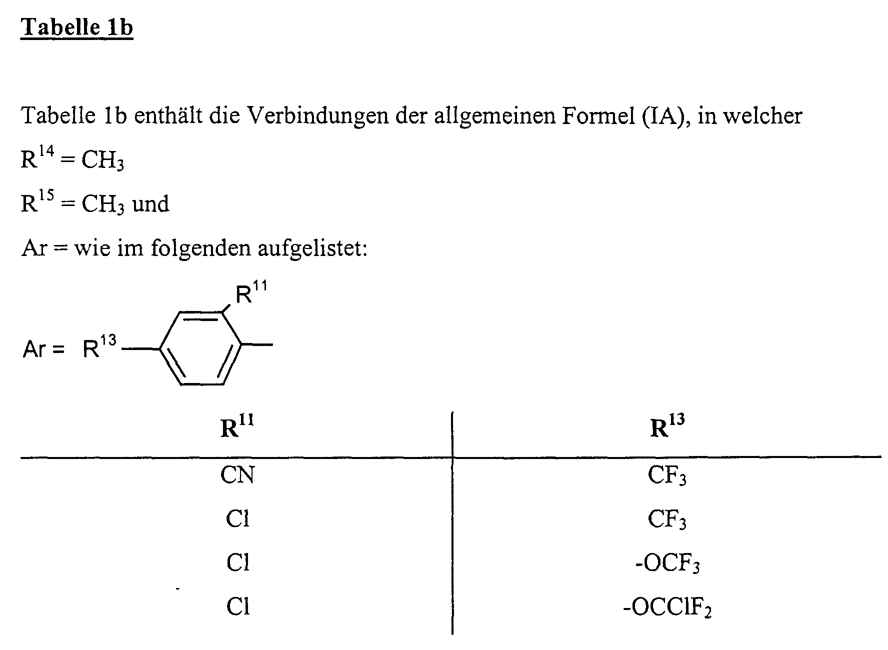

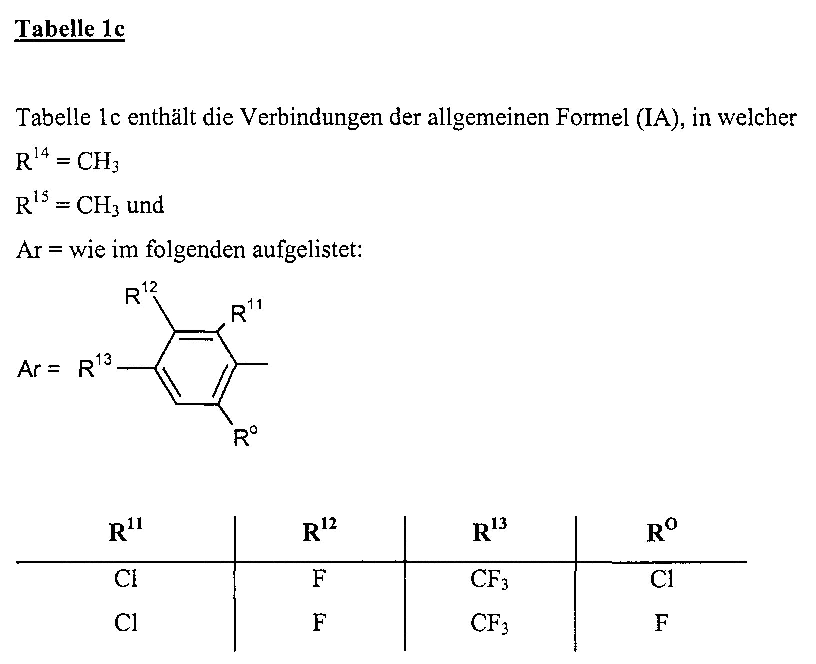

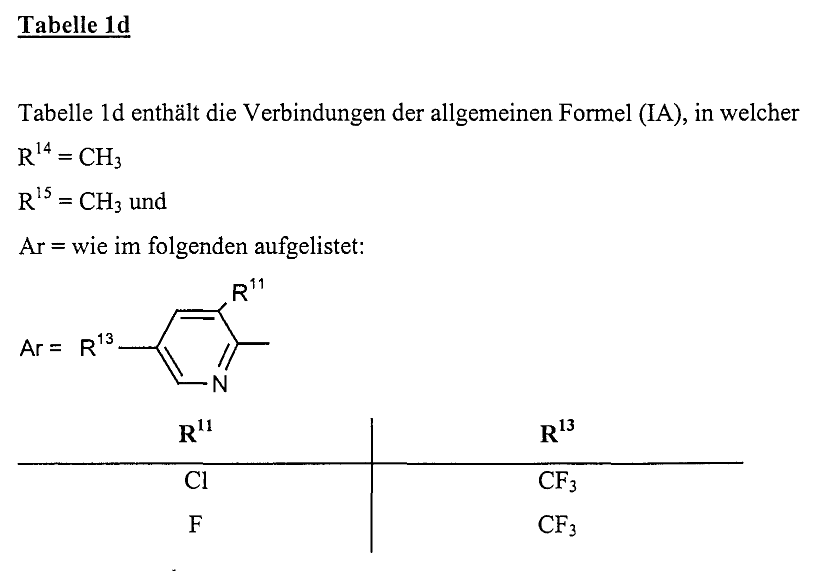

### Tabellen 2a bis 2d

Tabellen 2a bis 2d enthalten die Verbindungen der allgemeinen Formel (IA), in welcher
R¹⁴ = C₂H₅
R¹⁵ = CH₃ und
Ar = wie in den Tabellen 1a bis 1d aufgelistet.

### Tabellen 3a bis 3d

Tabellen 3a bis 3d enthalten die Verbindungen der allgemeinen Formel (IA), in welcher
R¹⁴ = C₃H₇-i
R¹⁵ = CH₃ und
Ar = wie in den Tabellen 1a bis 1d aufgelistet.

### Tabellen 4a bis 4d

Tabellen 4a bis 4d enthalten die Verbindungen der allgemeinen Formel (IA), in welcher
R¹⁴ = C₃H₇-n
R¹⁵ = CH₃ und
Ar = wie in den Tabellen 1a bis 1d aufgelistet.

### Tabellen 5a bis 5d

Tabellen 5a bis 5d enthalten die Verbindungen der allgemeinen Formel (IA), in welcher
R¹⁴ = C₄H₉-n
R¹⁵ = CH₃ und
Ar = wie in den Tabellen 1a bis 1d aufgelistet.

### Tabellen 6a bis 6d

Tabellen 6a bis 6d enthalten die Verbindungen der allgemeinen Formel (IA), in welcher
R¹⁴ = 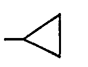
R¹⁵ = CH₃ und
Ar = wie in den Tabellen 1a bis 1d aufgelistet.

### Tabellen 7a bis 7d

Tabellen 7a bis 7d enthalten die Verbindungen der allgemeinen Formel (IA), in welcher
R¹⁴= 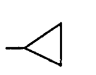
R¹⁵ = C₂H₅ und
Ar = wie in den Tabellen 1a bis 1d aufgelistet.

### Tabellen 8a bis 8d

Tabellen 8a bis 8d enthalten die Verbindungen der allgemeinen Formel (IA), in welcher
R¹⁴ = 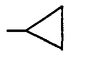
R¹⁵ = C₃H₇-i und
Ar = wie in den Tabellen 1a bis 1d aufgelistet.

### Tabellen 9a bis 9d

Tabellen 9a bis 9d enthalten die Verbindungen der allgemeinen Formel (IA), in welcher
R¹⁴ = 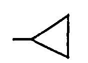
R¹⁵ = 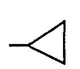 und
Ar = wie in den Tabellen 1a bis 1d aufgelistet.

### Tabellen 10a bis 10d

Tabellen 10a bis 10d enthalten die Verbindungen der allgemeinen Formel (IA), in welcher
R¹⁴ = C₃H₇-i
R¹⁵ = 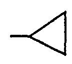 und
Ar = wie in den Tabellen 1a bis 1d aufgelistet.

### Tabellen 11a bis 11d

Tabellen 11 a bis 11d enthalten die Verbindungen der allgemeinen Formel (IA), in welcher
R¹⁴ = C₄H₉-t
R¹⁵ = 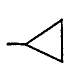 und
Ar = wie in den Tabellen 1a bis 1d aufgelistet.

### Tabellen 12a bis 12d

Tabellen 12a bis 12d enthalten die Verbindungen der allgemeinen Formel (IA), in welcher
R¹⁴ = 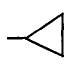
R¹⁵ = -CH₂CH₂OCH₃ und
Ar = wie in den Tabellen 1a bis 1d aufgelistet.

### Tabellen 13a bis 13d

Tabellen 13a bis 13d enthalten die Verbindungen der allgemeinen Formel (IA), in welcher
R¹⁴ = CF₃
R¹⁵ = H und
Ar = wie in den Tabellen 1a bis 1d aufgelistet.

### Tabellen 14a bis 14d

Tabellen 14a bis 14d enthalten die Verbindungen der allgemeinen Formel (IA), in welcher
R¹⁴ = CF₃
R¹⁵ = -SCFCl₂ und
Ar = wie in den Tabellen 1a bis 1d aufgelistet.

### Tabellen 15a bis 15d

Tabellen 15a bis 15d enthalten die Verbindungen der allgemeinen Formel (IA), in welcher
R¹⁴ = 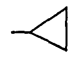
R¹⁵ = -OCH₃ und
Ar = wie in den Tabellen 1a bis 1d aufgelistet.

### Tabellen 16a bis 16d

Tabellen 16a bis 16d enthalten die Verbindungen der allgemeinen Formel (IA), in welcher
R¹⁴ = 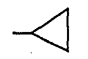
R¹⁵ = -OC₂H₅ und
Ar = wie in den Tabellen 1a bis 1d aufgelistet.

### Tabellen 17a bis 17d

Tabellen 17a bis 17d enthalten die Verbindungen der allgemeinen Formel (IA), in welcher
R¹⁴ = 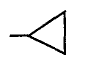
R¹⁵ = -OCH₂CH=CH₂ und
Ar = wie in den Tabellen 1a bis 1d aufgelistet.

### Tabellen 18a bis 18d

Tabellen 18a bis 18d enthalten die Verbindungen der allgemeinen Formel (IA), in welcher
R¹⁴ = 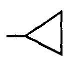
R¹⁵ = -NH₂ und
Ar = wie in den Tabellen 1a bis 1d aufgelistet.

### Tabellen 19a bis 19d

Tabellen 19a bis 19d enthalten die Verbindungen der allgemeinen Formel (IA), in welcher
R¹⁴ = 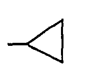
R¹⁵ = -CHF₂ und
Ar = wie in den Tabellen 1a bis 1d aufgelistet.

### Tabellen 20a bis 20d

Tabellen 20a bis 20d enthalten die Verbindungen der allgemeinen Formel (IA), in welcher
R¹⁴ = CF₃
R¹⁵ = C₃H₇-i und
Ar = wie in den Tabellen 1a bis 1d aufgelistet.

### Tabellen 21a bis 21d

Tabellen 21a bis 21d enthalten die Verbindungen der allgemeinen Formel (IA), in welcher
R¹⁴ = CF₃
R¹⁵ = CH₃ und
Ar = wie in den Tabellen 1a bis 1d aufgelistet.

### Tabellen 22a bis 22d

Tabellen 22a bis 22d enthalten die Verbindungen der allgemeinen Formel (IA), in welcher
R¹⁴ = CF₃
R¹⁵ = C₂H₅ und
Ar = wie in den Tabellen 1a bis 1d aufgelistet.

### Tabellen 23a bis 23d

Tabellen 23a bis 23d enthalten die Verbindungen der allgemeinen Formel (IA), in welcher
R¹⁴ = CF₃
R¹⁵ = C₄H₉-t und
Ar = wie in den Tabellen 1a bis 1d aufgelistet.

### Tabellen 24a bis 24d

Tabellen 24a bis 24d enthalten die Verbindungen der allgemeinen Formel (IA), in welcher
R¹⁴ = CF₃
R¹⁵ = 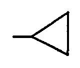 und
Ar = wie in den Tabellen 1a bis 1d aufgelistet.

### Tabellen 25a bis 25d

Tabellen 25a bis 25d enthalten die Verbindungen der allgemeinen Formel (IA), in welcher
R¹⁴ = CF₃
R¹⁵ = 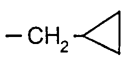 und
Ar = wie in den Tabellen 1a bis 1d aufgelistet.

### Tabellen 26a bis 26d

Tabellen 26a bis 26d enthalten die Verbindungen der allgemeinen Formel (IA), in welcher
R¹⁴ = CHF₂
R¹⁵ = 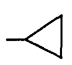 und
Ar = wie in den Tabellen 1a bis 1d aufgelistet.

### Tabellen 27a bis 27d

Tabellen 27a bis 27d enthalten die Verbindungen der allgemeinen Formel (IA), in welcher
R¹⁴ = CHF₂
R¹⁵ = CN und
Ar = wie in den Tabellen 1a bis 1d aufgelistet.

### Tabellen 28a bis 28d

Tabellen 28a bis 28d enthalten die Verbindungen der allgemeinen Formel (IA), in welcher
R¹⁴ = CF₃
R¹⁵ = CN und
Ar = wie in den Tabellen 1a bis 1d aufgelistet.

### Tabellen 29a bis 29d

Tabellen 29a bis 29d enthalten die Verbindungen der allgemeinen Formel (IA), in welcher
R¹⁴ = SCHF₂
R¹⁵ = C₃H₇-i und
Ar = wie in den Tabellen 1a bis 1d aufgelistet.

### Tabellen 30a bis 30d

Tabellen 30a bis 30d enthalten die Verbindungen der allgemeinen Formel (IA), in welcher
R¹⁴ = CF₃
R¹⁵ = -OCH₃ und
Ar = wie in den Tabellen 1a bis 1d aufgelistet.

### Tabellen 31a bis 31d

Tabellen 31a bis 31d enthalten die Verbindungen der allgemeinen Formel (IA), in welcher
R¹⁴ = CF₃
R¹⁵ = -CH₂CH₂OCH₃ und
Ar = wie in den Tabellen 1a bis 1d aufgelistet.

### Tabellen 32a bis 32d

Tabellen 32a bis 32d enthalten die Verbindungen der allgemeinen Formel (IA), in welcher
R¹⁴ = CF₃
R¹⁵ = 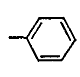 und
Ar = wie in den Tabellen 1a bis 1d aufgelistet.

### Tabellen 33a bis 33d

Tabellen 33a bis 33d enthalten die Verbindungen der allgemeinen Formel (IA), in welcher
R¹⁴ = CF₃
R¹⁵ = 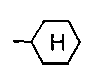 und
Ar = wie in den Tabellen 1a bis 1d aufgelistet.

### Tabellen 34a bis 34d

Tabellen 34a bis 34d enthalten die Verbindungen der allgemeinen Formel (IA), in welcher
R¹⁴ = CF₃
R¹⁵ = 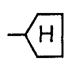 und
Ar = wie in den Tabellen 1a bis 1d aufgelistet.

### Tabellen 35a bis 35d

Tabellen 35a bis 35d enthalten die Verbindungen der allgemeinen Formel (IA), in welcher
R¹⁴ = -OCH₂CF₃
R¹⁵ = 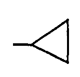 und
Ar = wie in den Tabellen 1a bis 1d aufgelistet.

### Tabellen 36a bis 36d

Tabellen 36a bis 36d enthalten die Verbindungen der allgemeinen Formel (IA), in welcher
R¹⁴ = 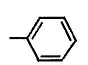
R¹⁵ = 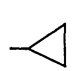 und
Ar = wie in den Tabellen 1a bis 1d aufgelistet.

### Tabellen 37a bis 37d

Tabellen 37a bis 37d enthalten die Verbindungen der allgemeinen Formel (IA), in welcher
R¹⁴= -CF₂CF₃
R¹⁵ = 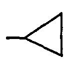 und
Ar = wie in den Tabellen 1a bis 1d aufgelistet.

### Tabellen 38a bis 38d

Tabellen 38a bis 38d enthalten die Verbindungen der allgemeinen Formel (IA), in welcher
R¹⁴= -CF₂CF₃
R¹⁵ = C₄H₉-t und
Ar = wie in den Tabellen 1a bis 1d aufgelistet.

### Tabellen 39a bis 39d

Tabellen 39a bis 39d enthalten die Verbindungen der allgemeinen Formel (IA), in welcher
R¹⁴= -CF₂CF₃
R¹⁵ = C₃H₇-i und
Ar = wie in den Tabellen 1a bis 1d aufgelistet.

### Tabellen 40a bis 40d

Tabellen 40a bis 40d enthalten die Verbindungen der allgemeinen Formel (IA), in welcher
R¹⁴= -CF₂CF₃
R¹⁵ = C₂H₅ und
Ar = wie in den Tabellen 1a bis 1d aufgelistet.

### Tabellen 41a bis 41d

Tabellen 41a bis 41d enthalten die Verbindungen der allgemeinen Formel (IA), in welcher
R¹⁴= -CF₂CF₃
R¹⁵ = CH₃ und
Ar = wie in den Tabellen 1a bis 1d aufgelistet.

### Tabellen 42a bis 42d

Tabellen 42a bis 42d enthalten die Verbindungen der allgemeinen Formel (IA), in welcher
R¹⁴= -CF₂CHF₂
R¹⁵ = CH₃ und
Ar = wie in den Tabellen 1a bis 1d aufgelistet.

### Tabellen 43a bis 43d

Tabellen 43a bis 43d enthalten die Verbindungen der allgemeinen Formel (IA), in welcher
R¹⁴= -CF₂CHF₂
R¹⁵ = C₂H₅ und
Ar = wie in den Tabellen 1a bis 1d aufgelistet.

### Tabellen 44a bis 44d

Tabellen 44a bis 44d enthalten die Verbindungen der allgemeinen Formel (IA), in welcher
R¹⁴= -CF₂CHF₂
R¹⁵ = C₃H₇-i und
Ar = wie in den Tabellen 1a bis 1d aufgelistet.

### Tabellen 45a bis 45d

Tabellen 45a bis 45d enthalten die Verbindungen der allgemeinen Formel (IA), in welcher
R¹⁴= -CF₂CHF₂
R¹⁵ = C₄H₉-t und
Ar = wie in den Tabellen 1a bis 1d aufgelistet.

### Tabellen 46a bis 46d

Tabellen 46a bis 46d enthalten die Verbindungen der allgemeinen Formel (IA), in welcher
R¹⁴= -CF₂CHF₂
R¹⁵ = C₃H₇-n und
Ar = wie in den Tabellen 1a bis 1d aufgelistet.

### Tabellen 47a bis 47d

Tabellen 47a bis 47d enthalten die Verbindungen der allgemeinen Formel (IA), in welcher
R¹⁴= -CF₂CHF₂
R¹⁵ = 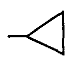 und
Ar = wie in den Tabellen 1a bis 1d aufgelistet.

### Tabellen 48a bis 48d

Tabellen 48a bis 48d enthalten die Verbindungen der allgemeinen Formel (IA), in welcher
R¹⁴= CF₃
R¹⁵ = -CH₂CH₂CH₂F und
Ar = wie in den Tabellen 1a bis 1d aufgelistet.

### Tabellen 49a bis 49d

Tabellen 49a bis 49d enthalten die Verbindungen der allgemeinen Formel (IA), in welcher
R¹⁴= C₃H₇-i
R¹⁵ = -CH₂CH₂CH₂F und
Ar = wie in den Tabellen 1a bis 1d aufgelistet.

### Tabellen 50a bis 50d

Tabellen 50a bis 50d enthalten die Verbindungen der allgemeinen Formel (IA), in welcher
R¹⁴ = 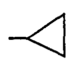
R¹⁵ = -CH₂CH₂CH₂F und
Ar = wie in den Tabellen 1a bis 1d aufgelistet.

### Tabellen 51a bis 51d

Tabellen 51 a bis 51 d enthalten die Verbindungen der allgemeinen Formel (IA), in welcher
R¹⁴ = CN
R¹⁵ = -CH₂CH₂CH₂F und
Ar = wie in den Tabellen 1a bis 1d aufgelistet.

### Tabellen 52a bis 52d

Tabellen 52a bis 52d enthalten die Verbindungen der allgemeinen Formel (IA), in welcher
R¹⁴ = CN
R¹⁵ = -CHF₂ und
Ar = wie in den Tabellen 1a bis 1d aufgelistet.

### Tabellen 53a bis 53d

Tabellen 53a bis 13d enthalten die Verbindungen der allgemeinen Formel (IA), in welcher
R¹⁴ = CN
R¹⁵ = 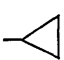 und
Ar = wie in den Tabellen 1a bis 1d aufgelistet.

### Tabellen 54a bis 54d

Tabellen 54a bis 54d enthalten die Verbindungen der allgemeinen Formel (IA), in welcher
R¹⁴ = Br
R¹⁵ = 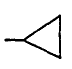 und
Ar = wie in den Tabellen 1a bis 1d aufgelistet.

### Tabellen 55a bis 55d

Tabellen 55a bis 55d enthalten die Verbindungen der allgemeinen Formel (IA), in welcher
R¹⁴ = Cl
R¹⁵ = 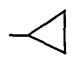 und
Ar = wie in den Tabellen 1a bis 1d aufgelistet.

### Tabellen 56a bis 56d

Tabellen 56a bis 56d enthalten die Verbindungen der allgemeinen Formel (IA), in welcher
R¹⁴ = Cl
R¹⁵ = -CHF₂ und
Ar = wie in den Tabellen 1a bis 1d aufgelistet.

### Tabellen 57a bis 57d

Tabellen 57a bis 57d enthalten die Verbindungen der allgemeinen Formel (IA), in welcher
R¹⁴ = Cl
R¹⁵ = -CH₃ und
Ar = wie in den Tabellen 1a bis 1d aufgelistet.

### Tabellen 58a bis 58d

Tabellen 58a bis 58d enthalten die Verbindungen der allgemeinen Formel (IA), in welcher
R¹⁴ = Cl
R¹⁵ = -C₂H₅ und
Ar = wie in den Tabellen 1a bis 1d aufgelistet.

### Tabellen 59a bis 59d

Tabellen 59a bis 59d enthalten die Verbindungen der allgemeinen Formel (IA), in welcher
R¹⁴ = Cl
R¹⁵ = -C₃H₇-i und
Ar = wie in den Tabellen 1a bis 1d aufgelistet.

### Tabellen 60a bis 60d

Tabellen 60a bis 60d enthalten die Verbindungen der allgemeinen Formel (IA), in welcher
R¹⁴ = Br
R¹⁵ = -C₃H₇-i und
Ar = wie in den Tabellen 1a bis 1d aufgelistet.

### Tabellen 61a bis 61d

Tabellen 61a bis 61d enthalten die Verbindungen der allgemeinen Formel (IA), in welcher
R¹⁴ = Br
R¹⁵ = -C₄H₉-t und
Ar = wie in den Tabellen 1a bis 1d aufgelistet.

### Tabellen 62a bis 62d

Tabellen 62a bis 62d enthalten die Verbindungen der allgemeinen Formel (IA), in welcher
R¹⁴ = SCHF₂
R¹⁵ = 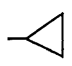 und
Ar = wie in den Tabellen 1a bis 1d aufgelistet.

### Tabellen 63a bis 63d

Tabellen 63a bis 63d enthalten die Verbindungen der allgemeinen Formel (IA), in welcher
R¹⁴ = OCF₂H
R¹⁵ = 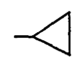 und
Ar = wie in den Tabellen 1a bis 1d aufgelistet.

### Tabellen 64a bis 64d

Tabellen 64a bis 64d enthalten die Verbindungen der allgemeinen Formel (IA), in welcher
R¹⁴ = OCHF₂
R¹⁵ = C₄H₉-t
Ar = wie in den Tabellen 1a bis 1d aufgelistet.

### Tabellen 65a bis 65d

Tabellen 65a bis 65d enthalten die Verbindungen der allgemeinen Formel (IA), in welcher
R¹⁴ = -OCH₃
R¹⁵ = 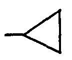 und
Ar = wie in den Tabellen 1a bis 1d aufgelistet.

### Tabellen 66a bis 66d

Tabellen 66a bis 66d enthalten die Verbindungen der allgemeinen Formel (IA), in welcher
R¹⁴ = -OC₂H₅
R¹⁵ = 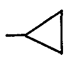 und
Ar = wie in den Tabellen 1a bis 1d aufgelistet.

### Tabellen 67a bis 67d

Tabellen 67a bis 67d enthalten die Verbindungen der allgemeinen Formel (IA), in welcher
R¹⁴ = -OC₃H₇-i
R¹⁵ = 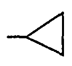 und
Ar = wie in den Tabellen 1a bis 1d aufgelistet.

### Tabellen 68a bis 68d

Tabellen 68a bis 68d enthalten die Verbindungen der allgemeinen Formel (IA), in welcher
R¹⁴ = -SCH₃
R¹⁵ = 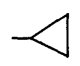 und
Ar = wie in den Tabellen 1a bis 1d aufgelistet.

### Tabellen 69a bis 69d

Tabellen 69a bis 69d enthalten die Verbindungen der allgemeinen Formel (IA), in welcher
R¹⁴ = -SO₂CH₃
R¹⁵ = 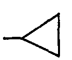 und
Ar = wie in den Tabellen 1a bis 1d aufgelistet.

### Tabellen 70a bis 70d

Tabellen 70a bis 70d enthalten die Verbindungen der allgemeinen Formel (IA), in welcher
R¹⁴ = -SC₃H₇-i
R¹⁵ = 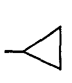 und
Ar = wie in den Tabellen 1a bis 1d aufgelistet.

### Tabellen 71a bis 71d

Tabellen 71a bis 71d enthalten die Verbindungen der allgemeinen Formel (IA), in welcher
R¹⁴ = C₄H₉-t
R¹⁵ = CN und
Ar = wie in den Tabellen 1a bis 1d aufgelistet.

### Tabellen 72a bis 72d

Tabellen 72a bis 72d enthalten die Verbindungen der allgemeinen Formel (IA), in welcher
R¹⁴ = -SC₂H₅
R¹⁵ = -CN und
Ar = wie in den Tabellen 1a bis 1d aufgelistet.

### Tabellen 73a bis 73d

Tabellen 73a bis 73d enthalten die Verbindungen der allgemeinen Formel (IA), in welcher
R¹⁴=CF₃
R¹⁵ = -CHF₂ und
Ar = wie in den Tabellen 1a bis 1d aufgelistet.

### Tabellen 74a bis 74d

Tabellen 74a bis 74d enthalten die Verbindungen der allgemeinen Formel (IA), in welcher
R¹⁴ = CF₃
R¹⁵ = -CH₂CN und
Ar = wie in den Tabellen 1a bis 1d aufgelistet.

### Tabellen 75a bis 75d

Tabellen 75a bis 75d enthalten die Verbindungen der allgemeinen Formel (IA), in welcher
R¹⁴ = CF₃
R¹⁵ = 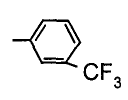 und
Ar = wie in den Tabellen 1a bis 1d aufgelistet.

### Tabellen 76a bis 76d

Tabellen 76a bis 76d enthalten die Verbindungen der allgemeinen Formel (IA), in welcher
R¹⁴ = CF₃
R¹⁵ = 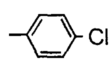 und
Ar = wie in den Tabellen 1a bis 1d aufgelistet.

### Tabellen 77a bis 77d

Tabellen 77a bis 77d enthalten die Verbindungen der allgemeinen Formel (IA), in welcher
R¹⁴ = CF₃
R¹⁵ = -N=CHOC₂C₅ und
Ar = wie in den Tabellen 1a bis 1d aufgelistet.

### Tabellen 78a bis 78d

Tabellen 78a bis 78d enthalten die Verbindungen der allgemeinen Formel (IA), in welcher
R¹⁴ = CF₃
R¹⁵ = 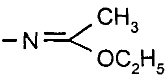 und
Ar = wie in den Tabellen 1a bis 1d aufgelistet.

### Tabellen 79a bis 79d

Tabellen 19a bis 19d enthalten die Verbindungen der allgemeinen Formel (IA), in welcher
R¹⁴ = CF₃
R¹⁵ = 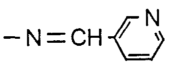 und
Ar = wie in den Tabellen 1a bis 1d aufgelistet.

### Tabellen 80a bis 80d

Tabellen 80a bis 80d enthalten die Verbindungen der allgemeinen Formel (IA), in welcher
R¹⁴ = CF₃
R¹⁵ = -SO₂CF₂CH₃
Ar = wie in den Tabellen 1a bis 1d aufgelistet.

Verwendet man beispielsweise 2,6-Dichlor-4-trifluormethyl-fluorbenzol und 3-Cyano-4-cyclopropyl-lH-1,2,4-triazolin-5-on als Ausgangsstoffe, so kann der Reaktionsablauf des erfindungsgemäßen Verfahrens (a) durch das folgende Formelschema wiedergegeben werden: 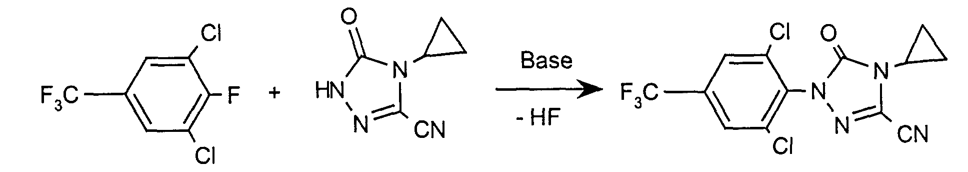 Verwendet man beispielsweise 2,6-Dinitro-4-trifluormethyl-phenyl-hydrazin und N-Methoxycarbonyl-acetimid-säureester als Ausgangsstoffe, so kann der Reaktionsablauf des erfindungsgemäßen Verfahrens (b) durch das folgende Formelschema wiedergegeben werden: 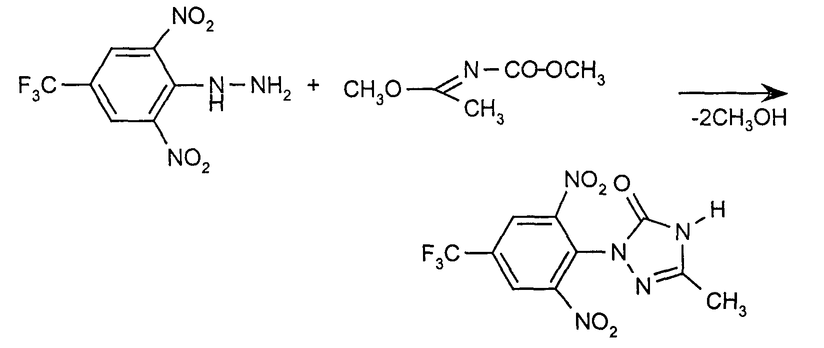 Verwendet man beispielsweise 1-(2,6-Dichlor-4-trifluormethyl-phenyl)-3-methyl-(4H)-1,2,4-triazolin-5-on und Chlordifluormethan als Ausgangsstoffe, so kann der Reaktionsablauf des erfindungsgemäßen Verfahrens (c) durch das folgende Formelschema wiedergegeben werden: 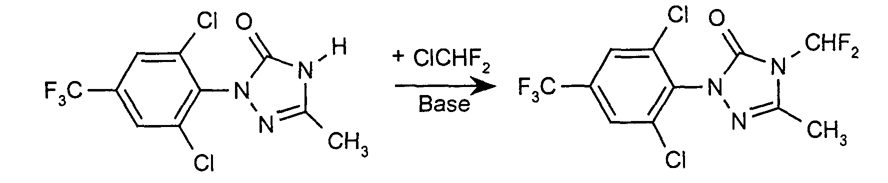 Die zur Durchführung des erfindungsgemäßen Verfahrens (a) als Ausgangsstoffe benötigten 1H-Triazolinone sind durch die Formel (II) allgemein definiert. In dieser Formel (II) stehen R¹⁴ und R¹⁵ vorzugsweise und besonders bevorzugt für diejenigen Reste, die bereits im Zusammenhang mit der Beschreibung der erfindungsgemäßen Verbindungen der Formel (Ia) als bevorzugt bzw. besonders bevorzugt für diese Substituenten genannt wurden.

Die 1H-Triazolinone der Formel (II) sind bekannt oder erhältlich nach bekannten Verfahren (vergl. z.B. EP 283 876; EP 305 844; EP 341 489; EP 415 196; EP 422 469; EP 425 948; EP 431 291; EP 507 171; EP 513 621; EP 534 266; Chem. Ber. 102, 755-766 [1969]; Liebigs Ann. Chem. 343, 24 [1905]). Teilweise sind sie auch Gegenstand einer eigenen Patentanmeldung, die noch nicht veröffentlicht ist (vgl. Deutsche Patentanmeldung P 4 435 547 vom 05.10.1994).

Beispiele für die IH-Triazolinone der Formel (II) sind in den Tabellen (II-1) bis (II-49) aufgeführt:

### Tabelle (II-1)

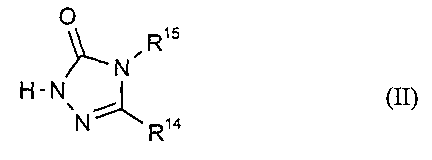

Verbindungen der Tabelle (II-1) entsprechen der allgemeinen Formel (II), in welcher
R¹⁴ = CH₃ und
R¹⁵ = wie im folgenden aufgelistet:
   H, CH₃, C₂H₅, n-C₃H₇, i-C₃H₇, n-C₄H₉, i-C₄H₉, s-C₄H₉, t-C₄H₉, CHF₂, CClF₂, CHClF,
   CH₂CH₂CH₂F, CH₂CF₃, CHF=CClF, CH₂CH₂-O-CH₃, CH₂CH₂-O-CH₂CH₃, CH₂CH=CH₂, CH₂CH₂CH₂-O-CH₃, CH₂CN, CN, CH₂C≡CH, O-CH₃, O-C₂H₅, O-CH₂CH=CH₂, O-CH(CH₃)C≡CH, SCFCl₂, SO₂CF₂CH₃, N=CHOC₂H₅, N=C(CH₃)OC₂H₅, NH₂, 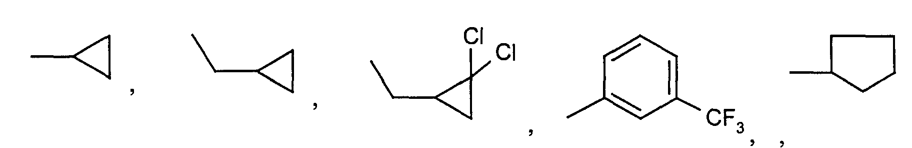 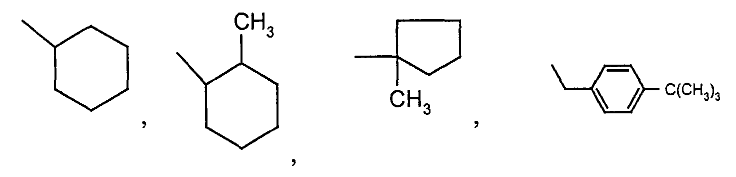 sowie

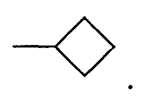

### Tabelle (II-2)

Tabelle (II-2) enthält die Verbindungen der allgemeinen Formel (II), in welcher
R¹⁴ = C₂H₅ und
R¹⁵ = wie in Tabelle (II-1) aufgelistet.

### Tabelle (II-3)

Tabelle (II-3) enthält die Verbindungen der allgemeinen Formel (II), in welcher
R¹⁴ = C₃H₇-i und
R¹⁵ = wie in Tabelle (II-1) aufgelistet.

### Tabelle (II-4)

Tabelle (II-4) enthält die Verbindungen der allgemeinen Formel (II), in welcher
R¹⁴ = C₃H₇-n und
R¹⁵ = wie in Tabelle (II-1) aufgelistet.

### Tabelle (II-5)

Tabelle (II-5) enthält die Verbindungen der allgemeinen Formel (II), in welcher
R¹⁴ = C₄H₉-n und
R¹⁵ = wie in Tabelle (II-1) aufgelistet.

### Tabelle (II-6)

Tabelle (II-6) enthält die Verbindungen der allgemeinen Formel (II), in welcher
R¹⁴ = C₄H₉-i und
R¹⁵ = wie in Tabelle (II-1) aufgelistet.

### Tabelle (II-7)

Tabelle (II-7) enthält die Verbindungen der allgemeinen Formel (II), in welcher
R¹⁴ = C₄H₉-t und
R¹⁵ = wie in Tabelle (II-1) aufgelistet.

### Tabelle (II-8)

Tabelle (II-8) enthält die Verbindungen der allgemeinen Formel (II), in welcher
R¹⁴ = C₄H₉-s und
R¹⁵ = wie in Tabelle (II-1) aufgelistet.

### Tabelle (II-9)

Tabelle (II-9) enthält die Verbindungen der allgemeinen Formel (II), in welcher
R¹⁴ = 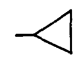 und
R¹⁵ = wie in Tabelle (II-1) aufgelistet.

### Tabelle (II-10)

Tabelle (II-10) enthält die Verbindungen der allgemeinen Formel (II), in welcher
R¹⁴ = CF₃ und
R¹⁵ = wie in Tabelle (II-1) aufgelistet.

### Tabelle (II-11)

Tabelle (II-11) enthält die Verbindungen der allgemeinen Formel (II), in welcher
R¹⁴ = CHF₂ und
R¹⁵ = wie in Tabelle (II-1) aufgelistet.

### Tabelle (II-12)

Tabelle (II-12) enthält die Verbindungen der allgemeinen Formel (II), in welcher
R¹⁴ = CClF₂ und
R¹⁵ = wie in Tabelle (II-1) aufgelistet.

### Tabelle (II-13)

Tabelle (II-13) enthält die Verbindungen der allgemeinen Formel (II), in welcher
R¹⁴ = CF₂CF₃ und
R¹⁵ = wie in Tabelle (II-1) aufgelistet.

### Tabelle (II-14)

Tabelle (II-14) enthält die Verbindungen der allgemeinen Formel (II), in welcher
R¹⁴ = CF₂CHF₂ und
R¹⁵ = wie in Tabelle (II-1) aufgelistet.

### Tabelle (II-15)

Tabelle (II-15) enthält die Verbindungen der allgemeinen Formel (II), in welcher
R¹⁴ = 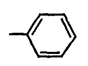 und
R¹⁵ = wie in Tabelle (II-1) aufgelistet.

### Tabelle (II-16)

Tabelle (II-16) enthält die Verbindungen der allgemeinen Formel (II), in welcher
R¹⁴ = CN und
R¹⁵ = wie in Tabelle (II-1) aufgelistet.

### Tabelle (II-17)

Tabelle (II-17) enthält die Verbindungen der allgemeinen Formel (II), in welcher
R¹⁴ = -OCH₂CF₃ und
R¹⁵ = wie in Tabelle (II-1) aufgelistet.

### Tabelle (II-18)

Tabelle (II-18) enthält die Verbindungen der allgemeinen Formel (II), in welcher
R¹⁴ = -OCHF₂ und
R¹⁵ = wie in Tabelle (II-1) aufgelistet.

### Tabelle (II-19)

Tabelle (II-19) enthält die Verbindungen der allgemeinen Formel (II), in welcher
R¹⁴ = Br und
R¹⁵ = wie in Tabelle (II-1) aufgelistet.

### Tabelle (II-20)

Tabelle (II-20) enthält die Verbindungen der allgemeinen Formel (II), in welcher
R¹⁴ = 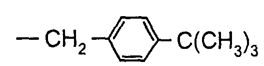 und
R¹⁵ = wie in Tabelle (II-1) aufgelistet.

### Tabelle (II-21)

Tabelle (II-21) enthält die Verbindungen der allgemeinen Formel (II), in welcher
R¹⁴ = Cl und
R¹⁵ = wie in Tabelle (II-1) aufgelistet.

### Tabelle (II-22)

Tabelle (II-22) enthält die Verbindungen der allgemeinen Formel (II), in welcher
R¹⁴ = OCH₃ und
R¹⁵ = wie in Tabelle (II-1) aufgelistet.

### Tabelle (II-23)

Tabelle (II-23) enthält die Verbindungen der allgemeinen Formel (II), in welcher
R¹⁴ = -OC₂H₅ und
R¹⁵ = wie in Tabelle (II-1) aufgelistet.

### Tabelle (II-24)

Tabelle (II-24) enthält die Verbindungen der allgemeinen Formel (II), in welcher
R¹⁴ = -OC₃H₇-i und
R¹⁵ = wie in Tabelle (II-1) aufgelistet.

### Tabelle (II-25)

Tabelle (II-25) enthält die Verbindungen der allgemeinen Formel (II), in welcher
R¹⁴ = -SCH₃ und
R¹⁵ = wie in Tabelle (II-1) aufgelistet.

### Tabelle (II-26)

Tabelle (II-26) enthält die Verbindungen der allgemeinen Formel (II), in welcher
R¹⁴ = -SO₂CH₃ und
R¹⁵ = wie in Tabelle (II-1) aufgelistet.

### Tabelle (II-27)

Tabelle (II-27) enthält die Verbindungen der allgemeinen Formel (II), in welcher
R¹⁴ =-SO₂C₂H₅ und
R¹⁵ = wie in Tabelle (II-1) aufgelistet.

### Tabelle (II-28)

Tabelle (II-28) enthält die Verbindungen der allgemeinen Formel (II), in welcher
R¹⁴ = -SC₂H₅ und
R¹⁵ = wie in Tabelle (II-1) aufgelistet.

### Tabelle (II-29)

Tabelle (II-29) enthält die Verbindungen der allgemeinen Formel (II), in welcher
R¹⁴ = -SC₃H₇-i und
R¹⁵ = wie in Tabelle (II-1) aufgelistet.

### Tabelle (II-30)

Tabelle (II-30) enthält die Verbindungen der allgemeinen Formel (II), in welcher
R¹⁴ = -SCHF₂ und
R¹⁵ = wie in Tabelle (II-1) aufgelistet.

### Tabelle (II-31)

Tabelle (II-31) enthält die Verbindungen der allgemeinen Formel (II), in welcher
R¹⁴ = -SCH₂CH₂CH₂F und
R¹⁵ = wie in Tabelle (II-1) aufgelistet.

### Tabelle (II-32)

Tabelle (II-32) enthält die Verbindungen der allgemeinen Formel (II), in welcher
R¹⁴ = -OCH₂CH₂CH₂F und
R¹⁵ = wie in Tabelle (II-1) aufgelistet.

### Tabelle (II-33)

Tabelle (II-33) enthält die Verbindungen der allgemeinen Formel (II), in welcher
R¹⁴ = 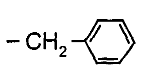 und
R¹⁵ = wie in Tabelle (II-1) aufgelistet.

### Tabelle (II-34)

Tabelle (II-34) enthält die Verbindungen der allgemeinen Formel (II), in welcher
R¹⁴ = CH₂CF₃ und
R¹⁵ = wie in Tabelle (II-1) aufgelistet.

### Tabelle (II-35)

Tabelle (II-35) enthält die Verbindungen der allgemeinen Formel (II), in welcher
R¹⁴ = 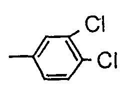 und
R¹⁵ = wie in Tabelle (II-1) aufgelistet.

### Tabelle (II-36)

Tabelle (II-36) enthält die Verbindungen der allgemeinen Formel (II), in welcher
R¹⁴ = 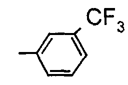 und
R¹⁵ = wie in Tabelle (II-1) aufgelistet.

### Tabelle (II-37)

Tabelle (II-37) enthält die Verbindungen der allgemeinen Formel (II), in welcher
R¹⁴ = -OCH₂CCl₃ und
R¹⁵ = wie in Tabelle (II-1) aufgelistet.

### Tabelle (II-38)

Tabelle (II-38) enthält die Verbindungen der allgemeinen Formel (II), in welcher
R¹⁴ = -SCH₂Cl und
R¹⁵ = wie in Tabelle (II-1) aufgelistet.

### Tabelle (II-39)

Tabelle (II-39) enthält die Verbindungen der allgemeinen Formel (II), in welcher
R¹⁴ = -SCH₂F und
R¹⁵ = wie in Tabelle (II-1) aufgelistet.

### Tabelle (II-40)

Tabelle (II-40) enthält die Verbindungen der allgemeinen Formel (II), in welcher
R¹⁴ = -SCH₂CH₂F und
R¹⁵ = wie in Tabelle (II-1) aufgelistet.

### Tabelle (II-41)

Tabelle (II-41) enthält die Verbindungen der allgemeinen Formel (II), in welcher
R¹⁴ = -SCH₂CHF₂ und
R¹⁵ = wie in Tabelle (II-1) aufgelistet.

### Tabelle (II-42)

Tabelle (II-42) enthält die Verbindungen der allgemeinen Formel (II), in welcher
R¹⁴ = -SCH₂CF₃ und
R¹⁵ = wie in Tabelle (II-1) aufgelistet.

### Tabelle (II-43)

Tabelle (II-43) enthält die Verbindungen der allgemeinen Formel (II), in welcher
R¹⁴ = -SCH₂CH₂CF₃ und
R¹⁵ = wie in Tabelle (II-1) aufgelistet.

### Tabelle (II-44)

Tabelle (II-44) enthält die Verbindungen der allgemeinen Formel (II), in welcher
R¹⁴ = -SCH₂CH=CH₂ und
R¹⁵ = wie in Tabelle (II-1) aufgelistet.

### Tabelle (II-45)

Tabelle (II-45) enthält die Verbindungen der allgemeinen Formel (II), in welcher
R¹⁴ = -SCH₂C≡CH und
R¹⁵ = wie in Tabelle (II-1) aufgelistet.

### Tabelle (II-46)

Tabelle (II-46) enthält die Verbindungen der allgemeinen Formel (II), in welcher
R¹⁴ = -SCH=C=CH₂ und
R¹⁵ = wie in Tabelle (II-1) aufgelistet.

### Tabelle (II-47)

Tabelle (II-47) enthält die Verbindungen der allgemeinen Formel (II), in welcher
R¹⁴ = -OCH₂CF₂CHF₂ und
R¹⁵ = wie in Tabelle (II-1) aufgelistet.

### Tabelle (II-48)

Tabelle (II-48) enthält die Verbindungen der allgemeinen Formel (II), in welcher
R¹⁴ = 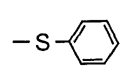 und
R¹⁵ = wie in Tabelle (II-1) aufgelistet.

### Tabelle (II-49)

Tabelle (II-49) enthält die Verbindungen der allgemeinen Formel (II), in welcher
R¹⁴ = 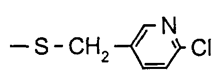 und
R¹⁵ = wie in Tabelle (II-1) aufgelistet.

Die weiterhin zur Durchführung des erfindungsgemäßen Verfahrens (a) als Ausgangsstoffe benötigten Halogen-Derivate sind durch die Formel (III) allgemein definiert. In dieser Formel (III) stehen R¹¹, R¹², R¹³ und A¹ vorzugsweise und besonders bevorzugt für diejenigen Reste, die bereits im Zusammenhang mit der Beschreibung der erfindungsgemäßen Verbindungen der Formel (Ia) als bevorzugt und besonders bevorzugt für diese Substituenten genannt wurden. Hal steht vorzugsweise für Fluor, Chlor oder Brom, insbesondere für Fluor oder Chlor.

Die Halogen-Derivate der Formel (III) sind allgemein bekannte Verbindungen der organischen Chemie bzw. in allgemein bekannter Art und Weise erhältlich.

Die zur Durchführung des erfindungsgemäßen Verfahrens (b) als Ausgangsstoffe benötigten Hydrazin-Derivate sind durch die Formel (IV) allgemein definiert. In dieser Formel (IV) stehen R¹¹, R¹², R¹³ und A¹ vorzugsweise und besonders bevorzugt für diejenigen Reste, die bereits im Zusammenhang mit der Beschreibung der erfindungsgemäßen Verbindungen der Formel (Ia) als bevorzugt und besonders bevorzugt für diese Substituenten genannt wurden.

Die Hydrazin-Derivate der Formel (IV) sind bekannt (vgl. z.B. US-PS 41 27 575; US-PS 36 09 158; DE-OS 25 58 399; J. Chem. Soc. C; 1971, 167-174) oder können nach bekannten Verfahren in einfacher Weise erhalten werden (vgl. z.B. Houben-Weyl "Methoden der Organischen Chemie" Band X/2, S. 203, Thieme Verlag Stuttgart, 1967).

Die weiterhin zur Durchführung des erfindungsgemäßen Verfahrens (b) als Ausgangsstoffe benötigten Iminocarbonester sind durch die Formel (V) allgemein definiert. In dieser Formel (V) stehen R²¹ und R²² unabhängig voneinander vorzugsweise für C₁-C₄-Alkyl, insbesondere für Methyl oder Ethyl. R¹⁴⁻¹ steht bevorzugt bzw. besonders bevorzugt für die oben im Zusammenhang mit der Beschreibung der erfindungsgemäßen Verbindungen der Formel (Ia) bei R¹⁴ für die entsprechenden Substituenten als bevorzugt bzw, besonders bevorzugt genannten Bedeutungen.

Die Iminocarbonester der Formel (V) sind bekannt oder nach bekannten Verfahren erhältlich (vgl. z.B. Ber. 119, 2444-2457 [1986]; Bull. chem. Soc. Jpn. 55, 3943-3944 [1982]; Chem. Lett. 1982, 1015-1016; Chem. Lett. 1987, 1403-1404; J. Amer. chem. Soc. 95, 3957-3963 [1973]; J. org. Chem. 36, 3251-3252 [1971]).

Die zur Durchführung des erfindungsgemäßen Verfahrens (c) als Ausgangsstoffe benötigten N-Aryl-1,2,4-triazolin-5-one der Formel (Id) sind erfindungsgemäße Verbindungen.

Die weiterhin zur Durchführung des erfindungsgemäßen Verfahrens (c) als Ausgangsstoffe benötigten Alkylierungsmittel sind durch die Formel (VI) allgemein definiert. In dieser Formel (VI) steht R¹⁵⁻¹ vorzugsweise bzw. besonders bevorzugt für die oben im Zusammenhang mit der Beschreibung der erfindungsgemäßen Verbindungen der Formel (Ia) bei R¹⁵ für die entsprechenden Substituenten als bevorzugt bzw. besonders bevorzugt genannten Bedeutungen. E steht für einen üblichen elektronenanziehenden Abgangsrest, beispielsweise für Halogen, insbesondere für Chlor, Brom oder Iod; oder für jeweils gegebenenfalls substituiertes Alkylsulfonyloxy, Alkoxysulfonyloxy oder Arylsulfonyloxy, insbesondere für Methansulfonyloxy, Trifluormethansulfonyloxy, Methoxysulfonyloxy, Ethoxysulfonyloxy oder p-Toluolsulfonyloxy.

Die Alkylierungsmittel der Formel (VI) sind allgemein bekannte Verbindungen der Organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl-oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Ester, wie Essigsäuremethylester oder Essigsäureethylester.

Das erfindungsgemäße Verfahren (a) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydroxide, wie Natriumhydroxid, Calciumhydroxid oder Kaliumhydroxid, Ammoniumhydroxid, Alkalimetallcarbonate, wie Natriumcarbonat, Kaliumcarbonat, Kaliumhydrogencarbonat oder Natriumhydrogencarbonat, Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU), ferner auch Piperidin.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und +180°C, vorzugsweise bei Temperaturen zwischen +20°C und +120°C.

Das erfindungsgemäße Verfahren (a) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (a) setzt man pro Mol 1H-Triazolinon der Formel (II) im allgemeinen 1,0 bis 3,0 Mol, vorzugsweise 1,0 bis 1,5 Mol Halogen-Derivat der Formel (III) und gegebenenfalls 1,0 bis 3,0 Mol, vorzugsweise 1,0 bis 1,5 Mol Base als Reaktionshilfsmittel ein.

Die Reaktionsdurchführung, Aufarbeitung und Isolierung der Reaktionsprodukte erfolgt nach allgemein üblichen, bekannten Verfahren (vergleiche auch die Herstellungsbeispiele).

Als Verdünnungsmittel zur Durchführung des erfindungsgemäßen Verfahrens (b) kommen inerte organische Lösungsmittel infrage. Vorzugsweise verwendet man die bei der Beschreibung der Durchführung des erfindungsgemäßen Verfahrens (a) aufgezählten Lösungsmittel.

Die Reaktionstemperaturen können bei der Durchführung des erfindungsgemäßen Verfahrens (b) in einem größeren Bereich variiert werden. Im allgemeinen arbeitet man bei Temperaturen zwischen -20°C und +150°C, vorzugsweise bei Temperaturen zwischen 0°C und +120°C.

Das erfindungsgemäße Verfahren (b) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (b) setzt man pro Mol Hydrazin-Derivat der Formel (IV) im allgemeinen 1,0 bis 3,0 Mol, vorzugsweise 1,0 bis 1,5 Mol Iminocarbonester der Formel (V) ein.

Die Reaktionsdurchführung, Aufarbeitung und Isolierung der Reaktionsprodukte erfolgt nach allgemein üblichen, bekannten Verfahren.

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid.

Das erfindungsgemäße Verfahren (c) kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-C₁₃/C₁₅-alkylammoniumchlorid, Trimethyl-C₁₃/C₁₅-alkylammoniumbromid, Dibenzyl-dimethyl-ammoniummethylsulfat, Dimethyl-C₁₂/C₁₄-alkyl-benzylammoniumchlorid, Dimethyl-C₁₂/C₁₄-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Das erfindungsgemäße Verfahren (c) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat, Ammoniumhydroxid, Ammoniumacetat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und +150°C, vorzugsweise bei Temperaturen zwischen 0°C und +120°C.

Das erfindungsgemäße Verfahren (c) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (c) setzt man pro Mol N-Aryl-1,2,4-triazolin-5-on der Formel (Id) im allgemeinen 1,0 bis 3,0 Mol, vorzugsweise 1,0 bis 2,0 Mol Alkylierungsmittel der Formel (VI) und gegebenenfalls 1,0 bis 3,0 Mol, vorzugsweise 1,0 bis 2,0 Mol Base als Reaktionshilfsmittel ein.

Die Reaktionsdurchführung, Aufarbeitung und Isolierung der Reaktionsprodukte erfolgt nach allgemein üblichen, bekannten Verfahren.

Die erfindungsgemäß verwendbaren Wirkstoffe der Formel (Ia) eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.
Aus der Ordnung der Chilopoda z.B. Geophilus carpophagus, Scutigera spec..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria, Supella spp..

Aus der Ordnung der Dermaptera z.B. Forficula auricularia.
Aus der Ordnung der Isoptera z.B. Reticulitermes spp..

Aus der Ordnung der Anoplura z.B. Phylloxera vastatrix, Pemphigus spp., Phthirus spp., Pediculus spp., Haematopinus spp., Linognathus spp., Solenopotes spp..
Aus der Ordnung der Mallophaga z.B. Trichodectes spp., Damalinea spp., Trimenopon spp., Monopon spp., Trinoton spp., Bovicola spp., Werneckiella spp., Lepikentron spp., Felicola spp..

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex spp., Rhodnius spp., Triatoma spp., Panstrongylus spp..

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp..

Aus der Ordnung der Diptera z.B. Aedes spp., Anopheles spp., Culex spp., Drosophila melanogaster, Musca spp., Lucilia spp., Chrysomyia spp., Cuterebra spp., Tannia spp., Bibio hortulanus, Oscinella frit, Phorbia spp., Pegomyia hyoscyami, Ceratitis capitata, Dacus oleae, Tipula paludosa, Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Hydrotaea spp., Stomoxys spp., Haematobia spp., Morellia spp., Fannia spp., Glossina spp., Calliphora spp., Wohlfahrtia spp., Sarcophaga spp., Oestrus spp., Hypoderma spp., Gasterophilus spp., Hippobosca spp., Lipoptena spp., Melophagus spp., Muscina spp..

Aus der Ordnung der Siphonapterida z.B. Xenopsylla spp., Ceratophyllus spp., Pulex spp., Ctenocephalides spp..
Aus der Ordnung der Arachnida z.B. Scorpio maurus, Latrodectus mactans.

Aus der Ordnung der Acarina z.B. Myocoptes spp., Acarus siro, Argas spp., Ornithodoros spp., Omithonyssus spp., Dermanyssus spp., Eriophyes ribis, Phyllocoptruta oleivora, Boophilus spp., Rhipicephalus spp., Amblyomma spp., Hyalomma spp., Ixodes spp., Tarsonemus spp., Bryobia praetiosa, Panonychus spp., Tetranychus spp., Dermacentor spp., Haemaphysalis spp., Raillietia spp., Pneumonyssus spp., Sternostorma spp., Varroa spp., Otobius spp..

Aus der Ordnung der Actinedida (Prostigmata) und Acaridida (Astigmata) z.B.: Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp..

Die erfindungsgemäß verwendbaren Wirkstoffe zeichnen sich insbesondere durch eine hohe insektizide Wirksamkeit aus.

Sie lassen sich mit besonders gutem Erfolg einsetzen zur Bekämpfung von pflanzenschädigenden Blatt- und Bodeninsekten, beispielsweise gegen die Larven des Meerettichblattkäfers (Phaedon cochleariae) oder gegen die Larven der grünen Reiszikade (Nephotettix cincticeps) gegen die Raupen der Kohlschabe (Plutella maculipennis), ferner gegen Raupen des Eulenfalters (Spodoptera frugiperda) und gegen die Tabakknospenraupe (Heliothis virescens).

Weiterhin weisen die erfindungsgemäß verwendbaren Wirkstoffe der Formel (Ia) auch eine akarizide und eine fungizide Wirkung auf, beispielsweise gegen Pyricularia oryzae am Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylhamstoffe, durch Mikroorganismen hergestellte Stoffe u.a.

### Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox, Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung, Mancopper, Mancozeb, Maneb, Mepanipyrim, Mepronil, Metalaxyl, Metconazol, Methasulfocarb, Methfüroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol, Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxyphenoxy-alkansäureester, wie z.B. Diclofop-methyl, Fenoxaprop-ethyl, Fluazifopbutyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuronmethyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufinilben, Fliegen (stechend und leckend), parasitierende Fliegenlarven, Läuse, Haarlinge, Federlinge und Flöhe. Beispielsweise zeigen sie eine hervorragende Wirksamkeit gegen Zecken, wie beispielsweise Boophilus microplus.

Die erfindungsgemäßen Wirkstoffe der Formel (Ia) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektion (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Haltern, Markierungsvorrichtungen usw.

Bei der Anwendung für Vieh, Geflügel, Haustiere etc. kann man die Wirkstoffe der Formel (Ia) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (Ia) eine hohe insektizide Wirkung gegen Insekten zeigen, die technische Materialien zerstören.

### Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis; Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze, wie Lepisma saccarina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und- türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der Wo 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, genannt.

Die Herstellung und die Verwendung der erfindungsgemäßen Wirkstoffe geht aus den nachfolgenden Beispielen hervor.

### Herstellungsbeispiele

### Beispiel 1

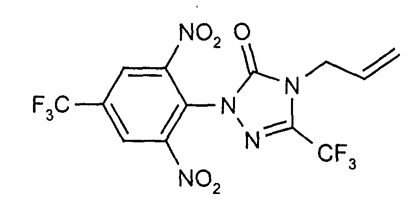

### (Verfahren a)

3,86 g (0,02 mol) 3-Trifluormethyl-4-propenyl-lH-1,2,4-triazolin-5-on und 2,76 g (0,02 mol) Kaliumcarbonat in 100 ml Dimethylsulfoxid werden bei Raumtemperatur mit 5,41 g (0,02 mol) 2,6-Dinitro-4-trifluormethyl-chlorbenzol versetzt und 4 Stunden bei 80°C und 1 Stunde bei 120°C gerührt. Zur Aufarbeitung wird die Reaktionsmischung mit Eiswasser verrührt, das ausgefallene Produkt abgesaugt und dieses aus Isopropanol umkristallisiert.

Man erhält 4,4 g (52 % der Theorie) 1-(2,6-Dinitro-4-trifluormethyl-phenyl)-3-trifluormethyl-4-propenyl-1,2,4-triazolin-5-on vom Schmelzpunkt 136°C.

Analog zu Beispiel 1 sowie entsprechend der allgemeinen Beschreibung der erfindungsgemäßen Herstellungsverfahren können die in der nachstehenden Tabelle 1 aufgefürten Verbindungen der Formel (Ia) hergestellt werden.

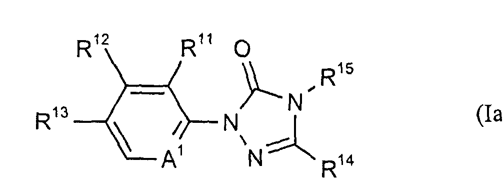

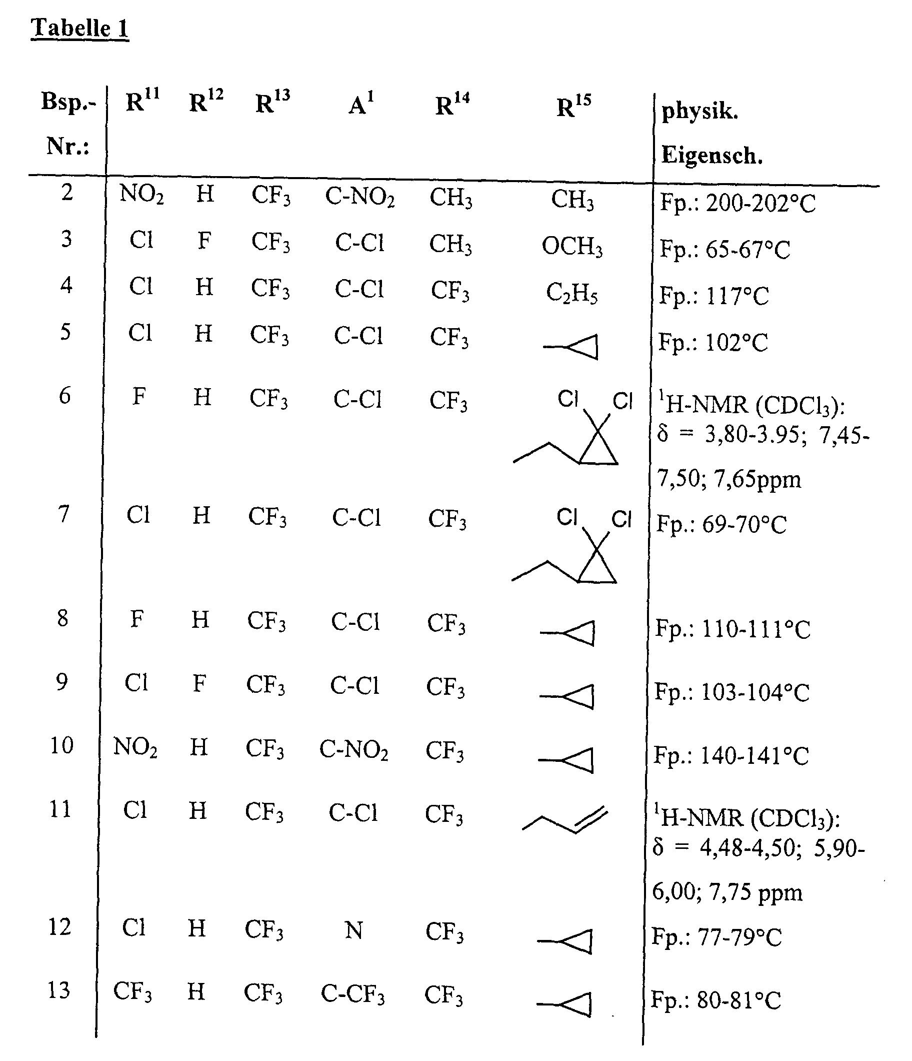

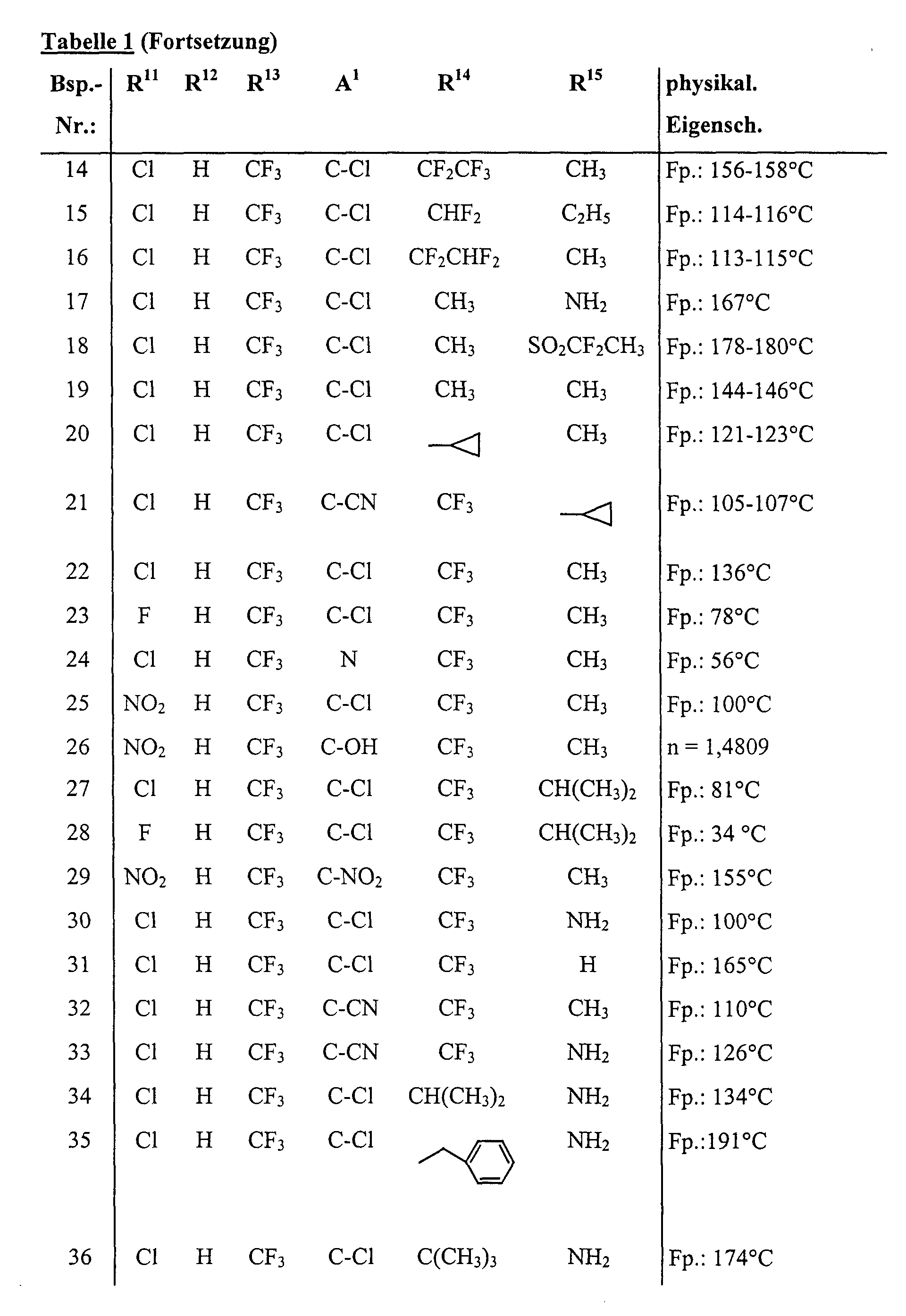

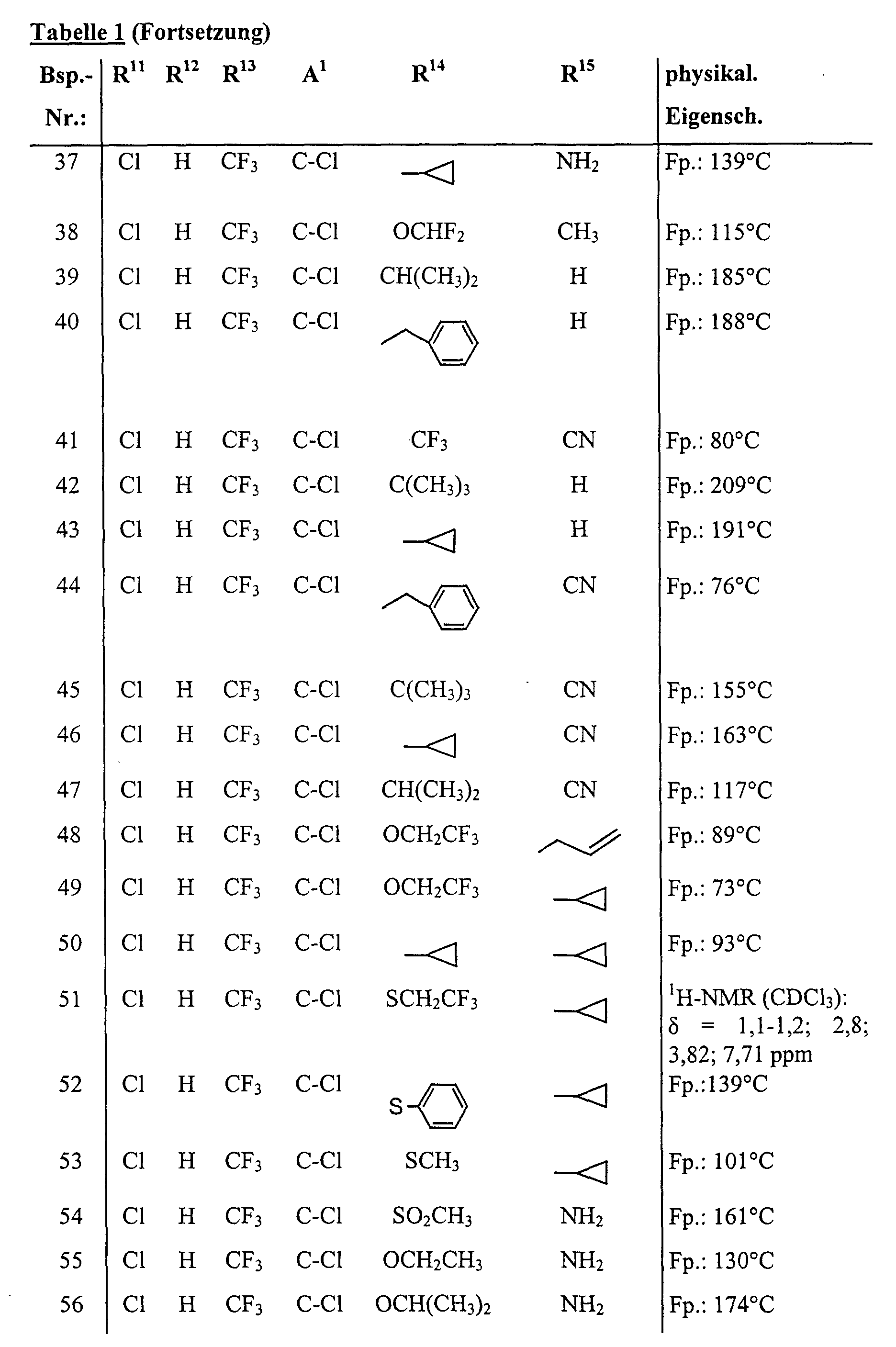

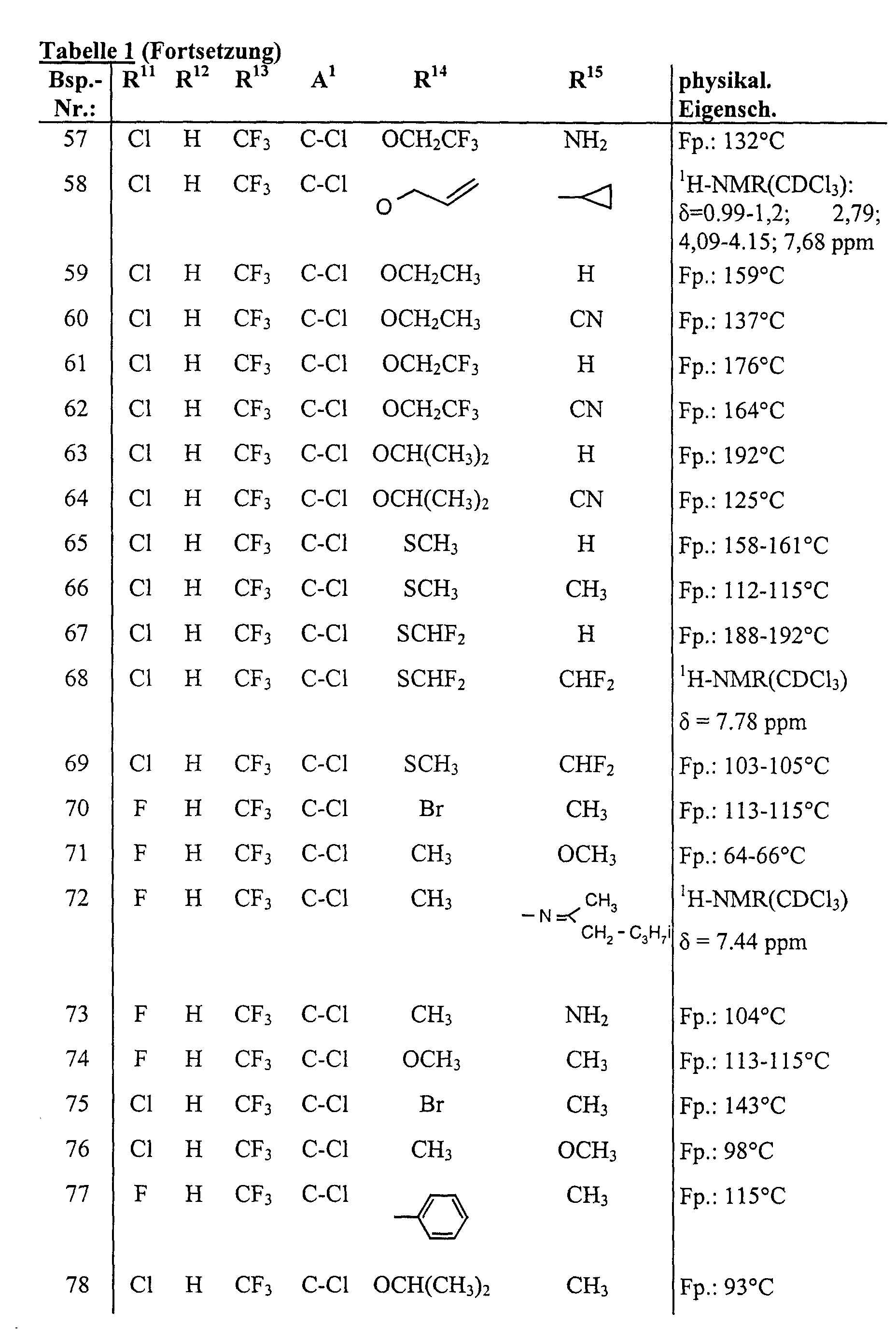

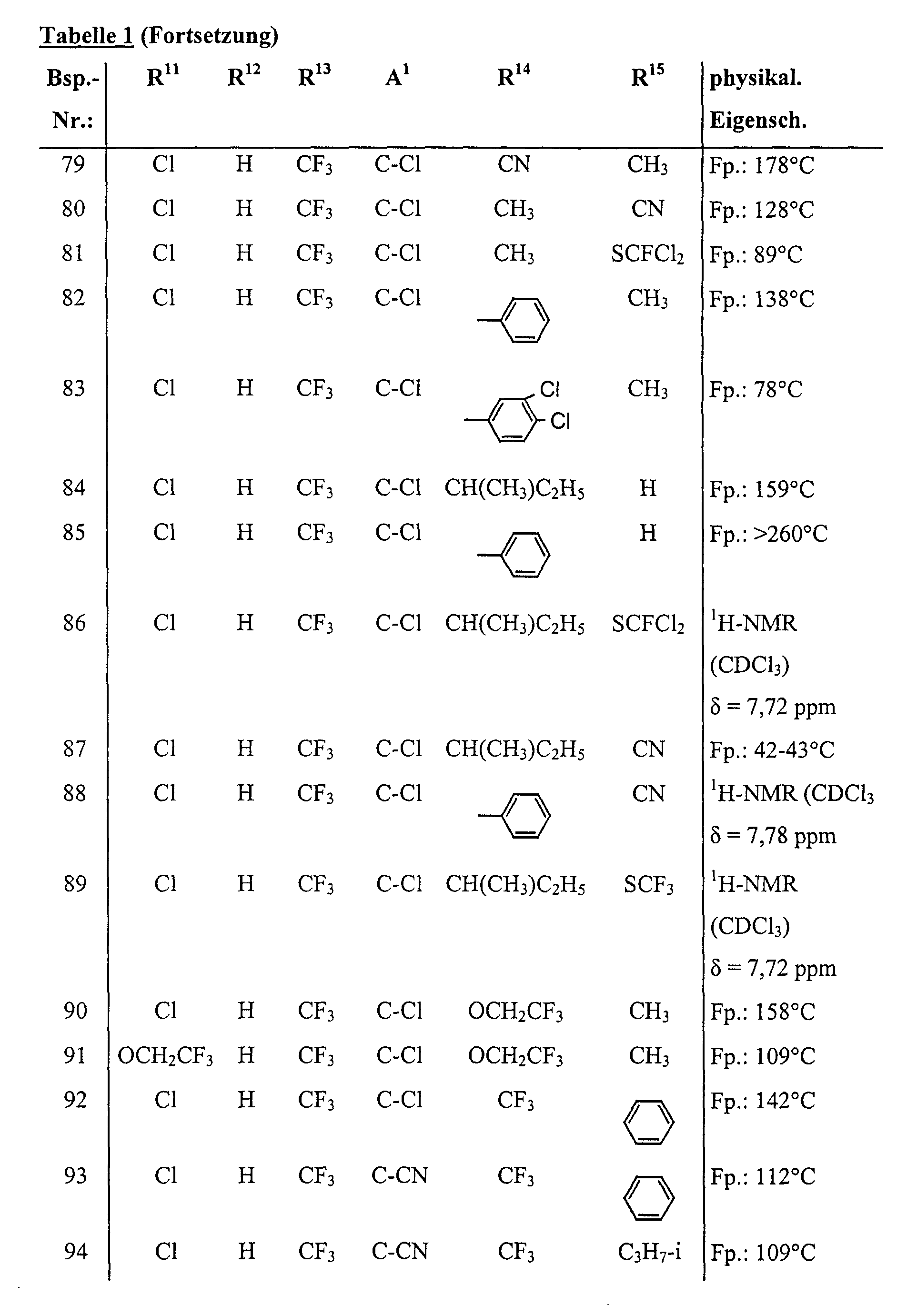

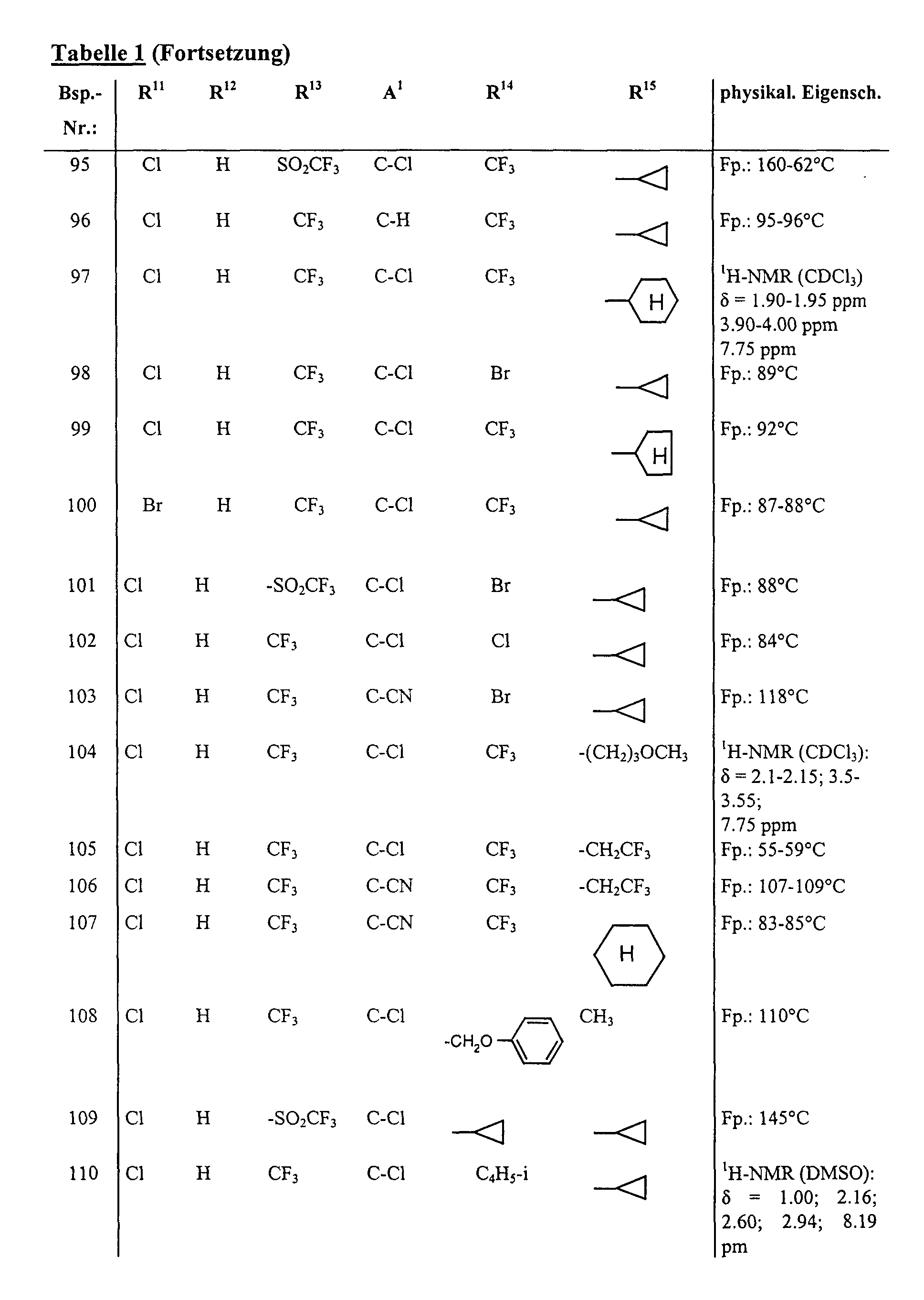

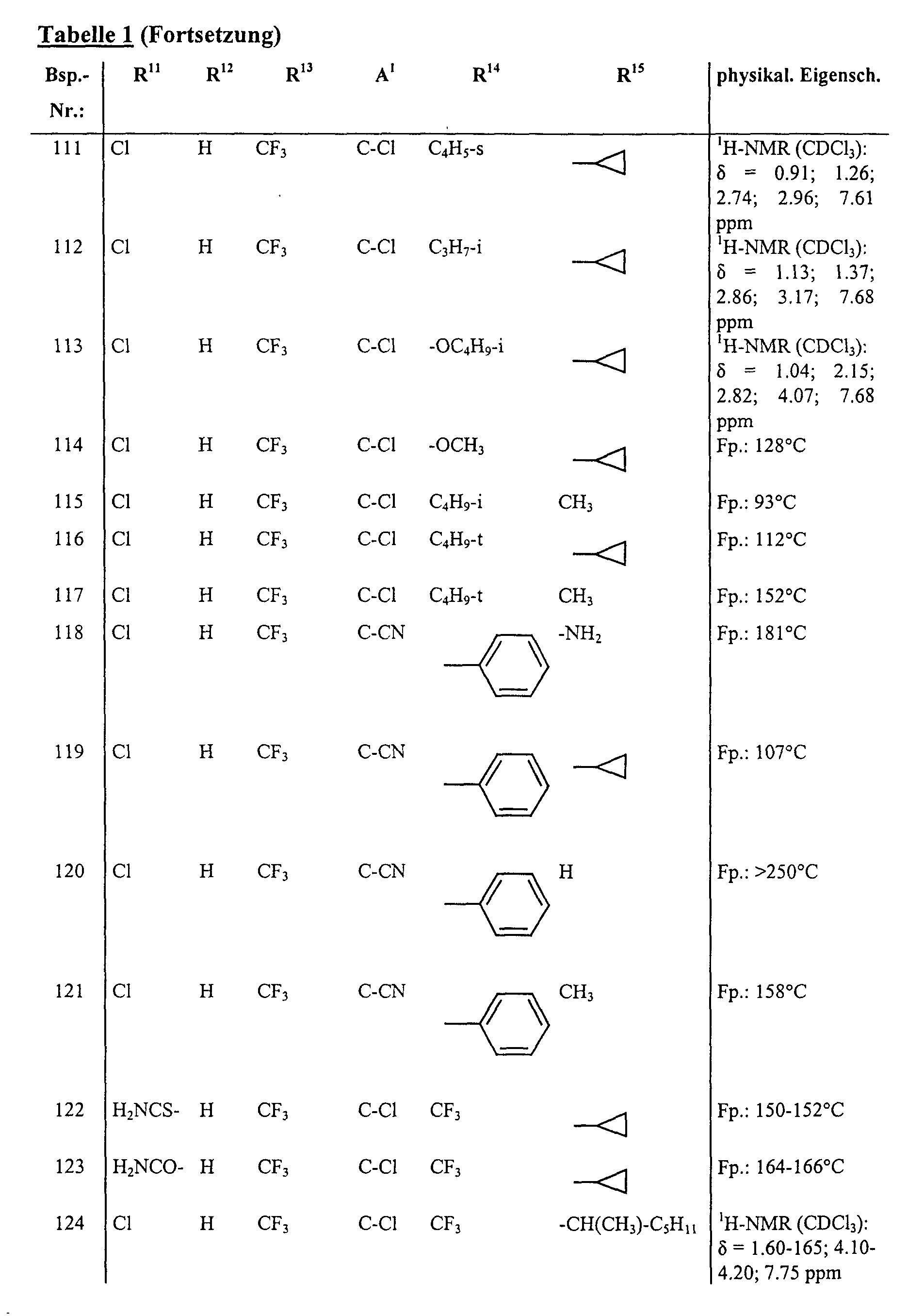

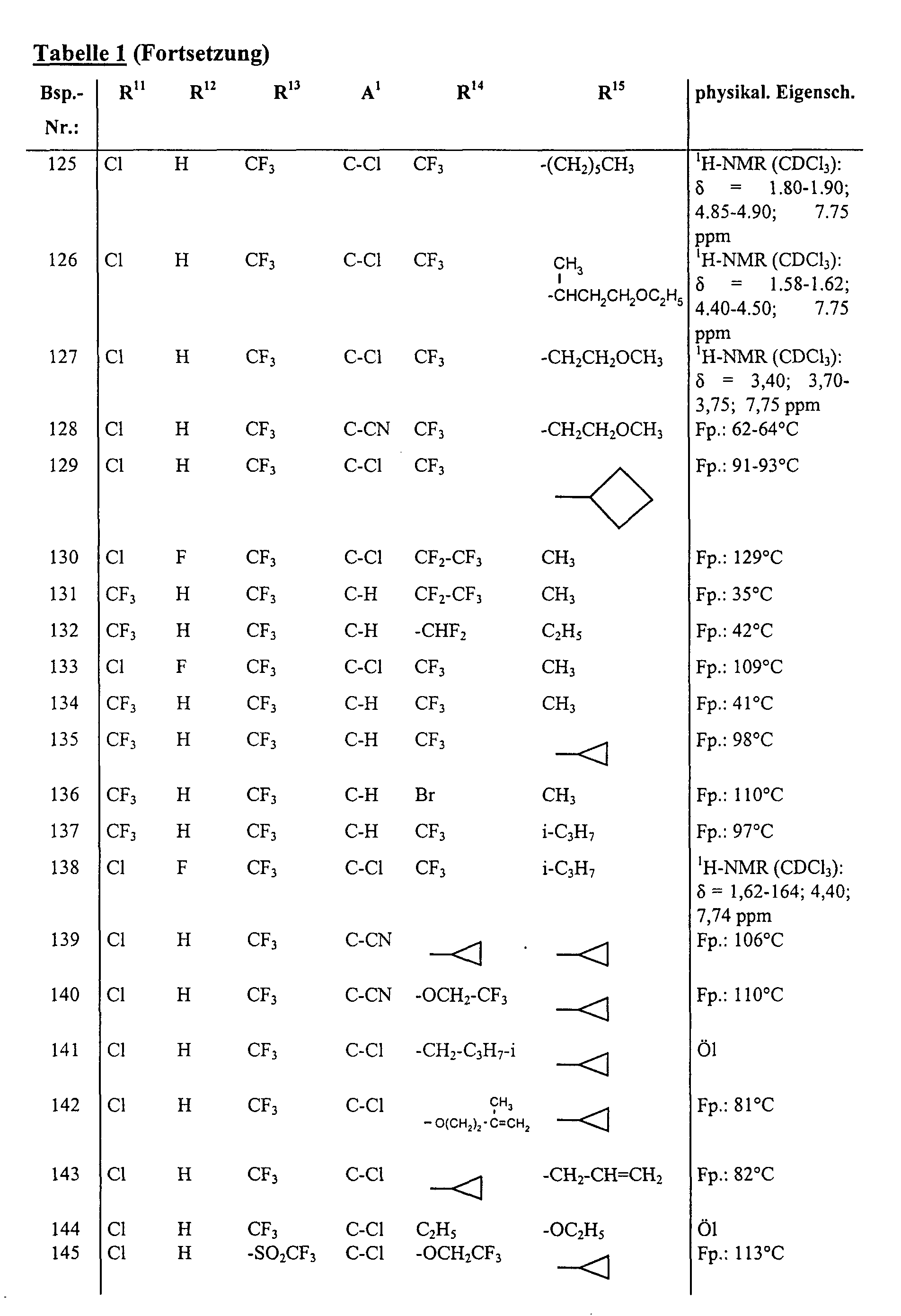

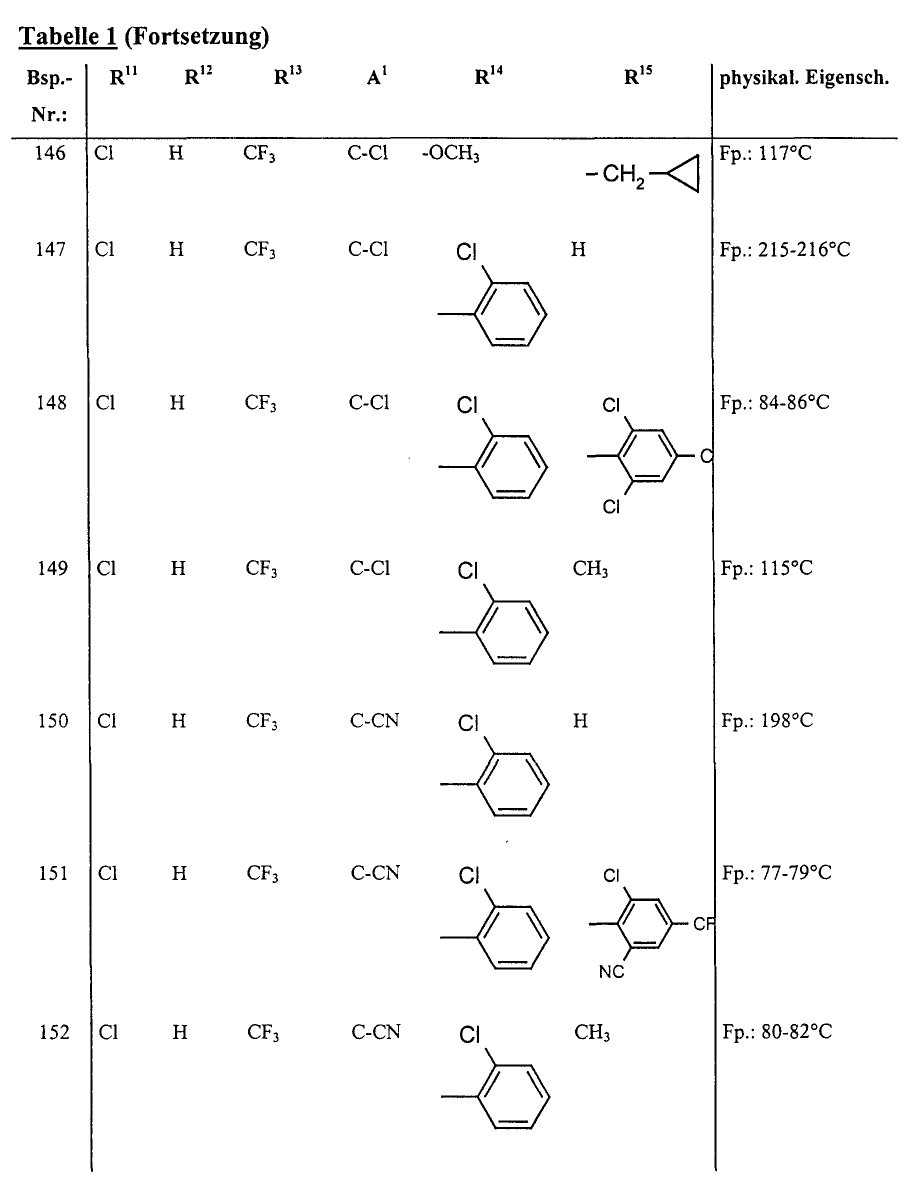

### Anwendungsbeispiele

In den folgenden Anwendungsbeispielen werden die nachstehend aufgeführten Verbindungen als Vergleichssubstanzen eingesetzt: 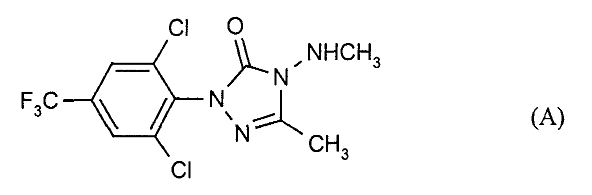 1-(2,6-Dichlor-4-trifluormethyl-phenyl)-3-methyl-4-methylamino-1,2,4-triazolin-5-on 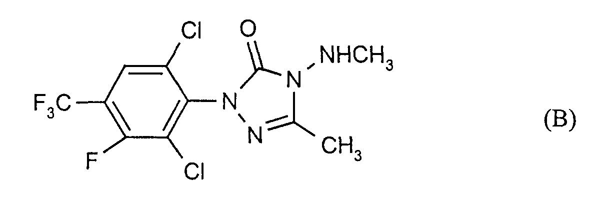 1-(2,6-Dichlor-3-fluor-4-trifluormethyl-phenyl)-3-methyl-4-methylamino-1,2,4-triazolin-5-on 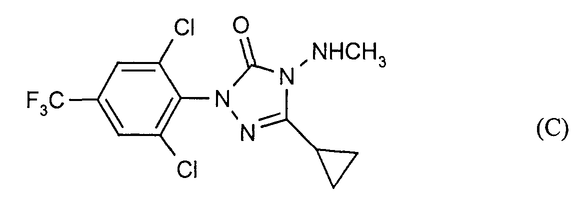 1-(2,6-Dichlor-4-trifluormethyl-phenyl)-3-cyclopropyl-4-methylamino-1,2,4-triazolin-5-on
(alle bekannt aus EP-A 0 617 026, (A) = Bsp. 44, (B) = Bsp. 45, (C) = Bsp. 72).

### Beispiel A

### Phaedon-Larven-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (*Brassica oleracea*) werden durch Tauchen in die Wirkstoffzubereitung der gewünschten Konzentration behandelt und mit Meerrettichblattkäfer-Larven (*Phaedon cochleariae*) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die Verbindungen der Herstellungsbeispiele 5, 8, 11, 12, 13, 15, 21, 27, 36, 42, 45, 50, 57 und 83 bei einer beispielhaften Wirkstoffkonzentration von 0,1 % nach 7 Tagen eine Abtötung von 100 %, während die bekannten Verbindungen (B) bzw. (C) keine bzw. eine 10 %-ige Abtötung zeigen.

### Beispiel B

### Plutella-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (*Brassica oleracea*) werden durch Tauchen in die Wirkstoffzubereitung der gewünschten Konzentration behandelt und mit Raupen der Kohlschabe *Plutella maculipennis* besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die Verbindungen der Herstellungsbeispiele 4, 5, 12, 21, 22, 32, 49 und 81 bei einer beispielhaften Wirkstoffkonzentration von 0,1 % nach 7 Tagen eine Abtötung von 100 %, während die bekannte Verbindung (C) eine 5 %-ige Abtötung zeigt.

### Beispiel C

### Spodoptera-Frugiperda-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (*Brassica oleracea*) werden durch Tauchen in die Wirkstoffzubereitung der gewünschten Konzentration behandelt und mit Raupen des Eulenfalters *Spodoptera frugiperda* besetzt, solange die Blätter noch feucht sind.

Nach der gewünschten Zeit wird die Wirkung in % bestimmt. Dabei bedeutet 100 %, daß alle Raupen abgetötet wurden; 0 % bedeutet, daß keine Raupen abgetötet wurden.

Bei diesem Test zeigen z.B. die Verbindungen der Herstellungsbeispiele 10, 12, 21, 49 und 50 bei einer beispielhaften Wirkstoffkonzentration von 0,1 % nach 7 Tagen eine Abtötung von 100 %, während die bekannte Verbindung (C) keine Wirkung zeigt.

### Beispiel D

### Heliothis virescens - Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (*Glycine max*) werden durch Tauchen in die Wirkstoffzubereitung der gewünschten Konzentration behandelt und mit der Tabakknospenraupe *Heliothis virescens* besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die Verbindungen der Herstellungsbeispiele 4 und 5 bei einer beispielhaften Wirkstoffkonzentration von 0,1 % nach 7 Tagen eine Abtötung von 100%, während die bekannten Verbindungen (A) bzw. (B) keine Wirkung zeigen.

### Beispiel E

### Nephotettix-Test

| | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge *(Oryza sativa)* werden durch Tauchen in die Wirkstoffzubereitung der gewünschten Konzentration behandelt und mit Larven der Grünen Reiszikade *Nephotettix cincticeps* besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigen z.B. die Verbindungen der Herstellungsbeispiele 12, 31, 41, 45 und 58 bei einer beispielhaften Wirkstoffkonzentration von 0,1 % nach 6 Tagen eine Abtötung von 90 bis 100 %, während die bekannten Verbindungen (A), (B) und (C) keine Wirkung zeigen.

### Beispiel F

**Test mit Boophilus microplus resistent**/SP-resistenter Parkhurst-Stamm

| | |
|---|---|
| Testtiere | adulte gesogene Weibchen |
| Lösungsmittel | Dimethylsulfoxid |

20 mg Wirkstoff werden in 1 ml Dimethylsulfoxid gelöst, geringe Konzentrationen werden durch verdünnen in dem gleichen Lösungsmittel hergestellt.

Der Test wird in 5-fach-Bestimmung durchgeführt, 1 µl der Lösung wird in das Abdomen injiziert, die Tiere in Schalen überführt und in einem klimatisierten Raum aufbewahrt. Die Wirkung wird über die Hemmung der Eiablage bestimmt. Dabei bedeutet 100 %, daß keine Zecke gelegt hat.

Bei diesem Test zeigen z.B. die erfindungsgemäßen Verbindungen gemäß den Herstellungsbeispielen 5, 21, 32, 33, 34 und 36 bei einer beispielhaften Wirkstoffkonzentration von 20 µg eine 100%-ige Wirkung, während die bekannten Verbindungen (A), (B) und (C) keine Wirkung zeigen.

### Beispiel G

### Schabentest

| | |
|---|---|
| Testtiere | Periplaneta americana |
| Lösungsmittel | 35 Gewichtsteile Ethylenglykolmonomethylether |
| Emulgator | 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben (∅ 9,5 cm) pipettiert, die sich in Petrischalen entsprechender Größe befinden. Nach Trocknung der Filterscheiben werden 5 Testtiere P. americana überführt und abgedeckt.

Nach 3 Tagen wird die Wirksamkeit der Wirkstoffzubereitung bestimmt. Dabei bedeutet 100 %, daß alle Schaben abgetötet wurden; 0 % bedeutet, daß keine Schaben abgetötet wurden.

In diesem Test zeigen z.B. die erfindungsgemäßen Verbindungen gemäß den Herstellungsbeispielen 4, 5, 12, 21, 27, 28, 32, 36, 41 und 50 bei einer beispielhaften Wirkstoffkonzentration von 1000 ppm eine 100%-ige Wirkung, während die bekannten Verbindungen (A), (B) und (C) keine Wirkung zeigen.

### Beispiel H

### Test mit Fliegen (Musca domestica)

| | |
|---|---|
| Testtiere | adulte Musca domestica, Stamm Reichswald (OP, SP, Carbamat-resistent) |
| Lösungsmittel | 35 Gewichtsteile Ethylenglykolmonomethylether |
| Emulgator | 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierschalen (∅ 9,5 cm) pipettiert, die sich in Petrischalen entsprechender Größe befinden. Nach Trocknung der Filterscheiben werden 25 Testtiere in die Petrischale überführt und abgedeckt.

Nach 1, 3, 5 und 24 Stunden wird die Wirksamkeit der Wirkstoffzubereitung ermittelt. Dabei bedeutet 100 %, daß alle Fliegen abgetötet wurden; 0 % bedeutet, daß keine Fliegen abgetötet wurden.

Bei diesem Test zeigen z.B. die erfindungsgemäßen Verbindungen gemäß den Herstellungsbeispielen 4, 5, 8, 12, 21, 22, 23, 24, 28, 32, 33 und 36 bei einer beispielhaften Wirkstoffkonzentration von 100 ppm eine 100 %-ige Wirkung, während die bekannten Verbindungen (A), (B) und (C) keine Wirkung zeigen.

### Beispiel I

### Test mit Fliegenlarven / Entwicklungshemmende Wirkung

| | |
|---|---|
| Testtiere | Alle larvalen Stadien von *Lucilia cuprina* (OP-resistent) [Puppen und Adulte (ohne Kontakt zum Wirkstoff)] |
| Lösungsmittel | 35 Gewichtsteile Ethylenglykolmonomethylether |
| Emulgator | 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

30 - 50 Larven je Konzentration werden auf in Glasröhrchen befindliches Pferdefleisch (1 cm³) gebracht, auf welches 500 µl der zu testenden Verdünnung pipettiert werden. Die Glasröhrchen werden in Kunststoffbecher gestellt, deren Boden mit Seesand bedeckt ist, und im klimatisierten Raum (26°C ± 1,5°C, 70 % relative Feuchte ± 10 %) aufbewahrt. Die Wirkungskontrolle erfolgt nach 24 Stunden und 48 Stunden (lervizide Wirkung). Nach dem Auswandern der Larven (ca. 72 Stunden) werden die Glasröhrchen entfernt und gelochte Kunststoffdeckel auf die Becher gesetzt. Nach 1½-facher Entwicklungsdauer (Schlupf der Kontrollfliegen) werden die geschlüpften Fliegen und die Puppen/Puppenhüllen ausgezählt.

Als Kriterium für die Wirkung gilt der Eintritt des Todes bei den behandelten Larven nach 48 Stunden (larvizider Effekt), bzw. die Hemmung des Adultschlupfes aus den Puppen bzw. die Hemmung der Puppenbildung. Als Kriterium für die in-vitro-Wirkung einer Substanz gilt die Hemmung der Flohentwicklung, bzw. ein Entwicklungsstillstand vor dem Adulten-Stadium. Dabei bedeutet 100 % larvizide Wirkung, daß nach 48 Stunden alle Larven abgestorben sind. 100 % entwicklungsinhibitorische Wirkung bedeutet, daß keine adulten Fliegen geschlüpft sind.

Bei diesem Test zeigen z.B. die erfindungsgemäßen Verbindungen gemäß den Herstellungsbeispielen 4, 5, 8, 12, 21, 33, 42 und 50 bei einer Wirkstoffkonzentration von 1000 ppm eine 100%-ige Wirkung, während die bekannten Verbindungen (A), (B) und (C) keine Wirkung zeigen.

## Patentansprüche

1. Verbindungen der Formel (Ia) 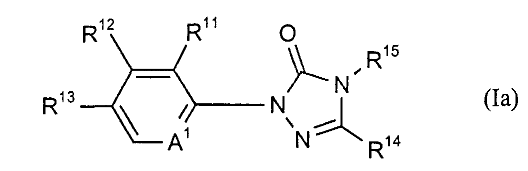 in welcher
A¹ für Stickstoff oder die Gruppe CR° steht,
R⁰ für Wasserstoff, Halogen, Nitro, Cyano, Hydroxy, gegebenenfalls durch Halogen, Cyano, Nitro, Alkoxy oder Amino substituiertes Alkyl, gegebenenfalls substituiertes Cycloalkyl oder den Rest -CX¹-NY¹¹Y¹² steht,
R¹¹ für Halogen, Nitro, Cyano, jeweils gegebenenfalls durch Halogen, Cyano, Nitro, Alkoxy oder Amino substituiertes Alkyl oder Alkoxy, gegebenenfalls substituiertes Cycloalkyl oder den Rest -CX¹-NY¹¹Y¹² steht,
R¹² für Wasserstoff, Halogen, gegebenenfalls durch Halogen, Cyano, Nitro, Alkoxy oder Amino substituiertes Alkyl oder gegebenenfalls substituiertes Cycloalkyl steht,
R¹³ für Halogenalkyl, Halogenalkoxy oder den Rest -S(O)ₙ¹ R¹⁶ steht,
R¹⁴ für Halogen, Cyano, jeweils gegebenenfalls durch Halogen, Cyano, Nitro, Alkoxy oder Amino substituiertes Alkyl oder Alkoxy, für Alkenyl, Alkenyloxy, Alkinyl, Hydroxy, Mercapto, jeweils gegebenenfalls substituiertes Cycloalkyl oder Cycloalkylalkyl, jeweils gegebenenfalls substituiertes Aryl, Aryloxyalkyl oder Aralkyl oder den Rest -S(O)ₙ¹R¹⁶ steht,
R¹⁵ für Wasserstoff, Cyano, Amino, jeweils gegebenenfalls durch Halogen, Cyano, Nitro, Alkoxy oder Amino substituiertes Alkyl oder Alkoxy, für Alkenyl, Alkenyloxy, Alkinyl, Alkinyloxy, jeweils gegebenenfalls substituiertes Cycloalkyl oder Cycloalkylalkyl, jeweils gegebenenfalls subsituiertes Aryl oder Aralkyl oder einen der Reste -S(O)ₙ¹R¹⁶, -NR¹⁷R¹⁸ oder -N=CR¹⁹R²⁰ steht,
R¹⁶ für gegebenenfalls durch Halogen, Cyano, Nitro, Alkoxy oder Amino substituiertes Alkyl, für Alkenyl, Alkandienyl, Alkinyl, gegebenenfalls substituiertes Cycloalkyl oder gegebenenfalls substituiertes Aryl steht,
R¹⁷ und R¹⁸ gemeinsam mit dem Stickstoffatom, an das sie gebunden sind, für einen gegebenenfalls substituierten Heterocyclus stehen, der gegebenenfalls ein oder mehrere weitere Heteroatome enthält,
R¹⁹ für Wasserstoff, gegebenenfalls durch Halogen, Cyano, Nitro, Alkoxy oder Amino substituiertes Alkyl, für Alkoxy, gegebenenfalls substituiertes Cycloalkyl oder jeweils gegebenenfalls subsituiertes Aryl oder Heterocyclyl steht,
R²⁰ für Wasserstoff, gegebenenfalls durch Halogen, Cyano, Nitro, Alkoxy oder Amino substituiertes Alkyl, für Alkoxy, gegebenenfalls substituiertes Cycloalkyl oder jeweils gegebenenfalls subsituiertes Aryl oder Heterocyclyl steht,
X¹ für Sauerstoff oder Schwefel steht,
Y¹¹ für Wasserstoff oder Alkyl steht,
Y¹² für Wasserstoff oder Alkyl steht und
n¹ für eine der Zahlen 0, 1 oder 2 steht,
mit Ausnahme der Verbindungen

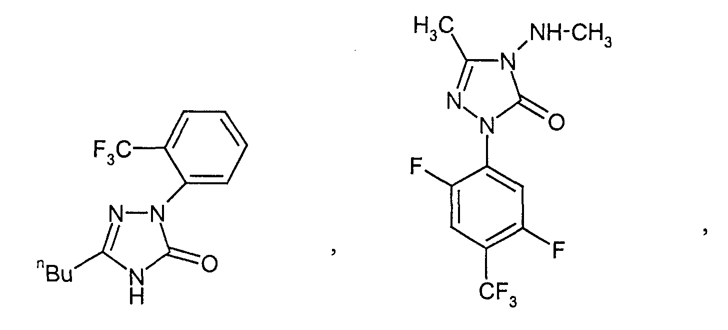

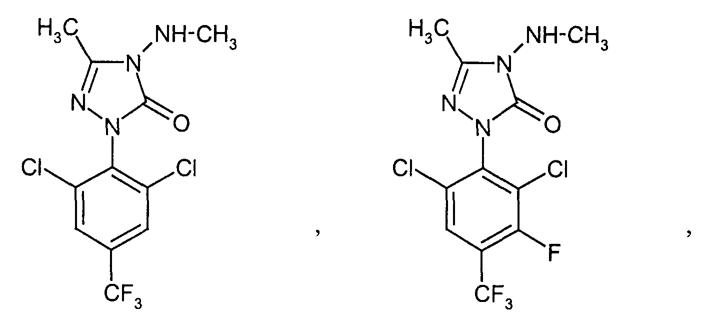

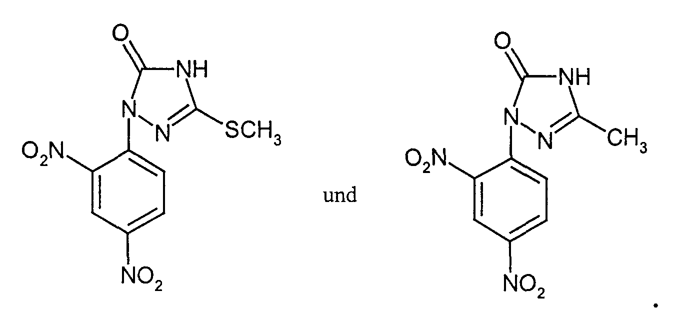

2. Verfahren zur Herstellung von Verbindungen der Formel (Ia) gemäß Anspruch 1, **dadurch gekennzeichnet**, daß man zum Erhalt von
a) Verbindungen der Formel (Ia) 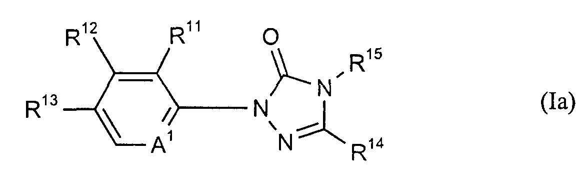 in welcher
R¹¹, R¹², R¹³, R¹⁴, R¹⁵ und A¹ die in Anspruch 1 angegebene Bedeutung haben,
1H-Triazolinone der Formel (II) 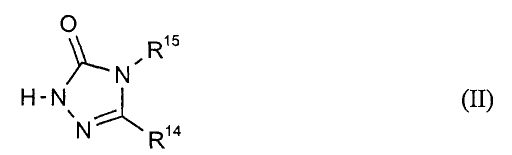 in welcher
R¹⁴ und R¹⁵ die in Anspruch 1 angegebene Bedeutung haben,
mit Halogen-Derivaten der Formel (III) 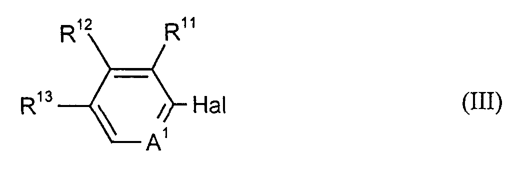 in welcher
R¹¹, R¹², R¹³ und A¹ die oben angegebene Bedeutung haben und
Hal für Halogen steht,
gegebenenfalls in Gegenwart eines Verdünnungsmittels und gegebenenfalls in Gegenwart eines Reaktionshilfsmittels umsetzt;
b) Verbindungen der Formel (Ib) 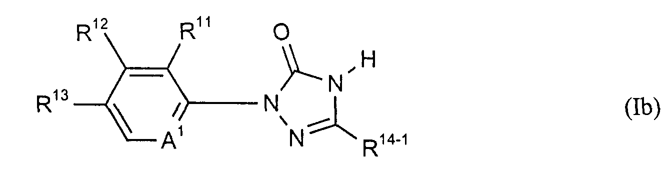 in welcher
R¹¹, R¹², R¹³ und A¹ die in Anspruch 1 angegebene angegebene Bedeutung haben und
R¹⁴⁻¹ für Cyano, jeweils gegebenenfalls durch Halogen, Cyano, Nitro, Alkoxy oder Amino substituiertes Alkyl oder Alkoxy, für Alkenyl, Alkenyloxy, Alkinyl, jeweils gegebenenfalls substituiertes Cycloalkyl oder Cycloalkylalkyl oder jeweils gegebenenfalls substituiertes Aryl, Aryloxyalkyl oder Aralkyl steht,
Hydrazin-Derivate der Formel (IV) 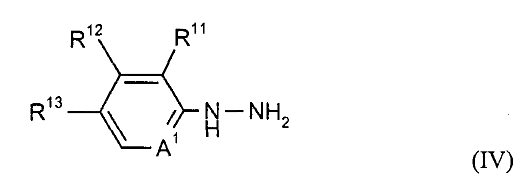 in welcher
R¹¹, R¹², R¹³ und A¹ die in Anspruch 1 angegebene angegebene Bedeutung haben,
mit Iminocarbonestern der Formel (V) 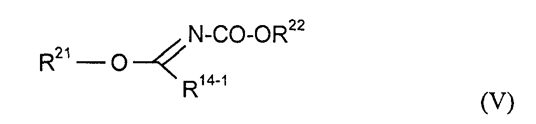 in welcher
R¹⁴⁻¹ die in Anspruch 1 angegebene angegebene Bedeutung hat und
R²¹ und R²² unabhängig voneinander für Alkyl (bevorzugt C₁-C₈-Alkyl) stehen,
in Gegenwart eines Verdünnungsmittels umsetzt;
c) Verbindungen der Formel (Ic) 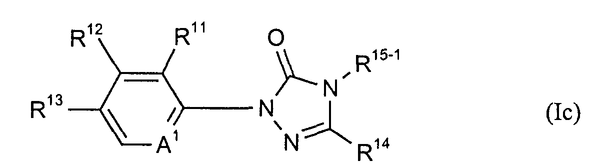 in welcher
R¹¹, R¹², R¹³, R¹⁴ und A¹ die in Anspruch 1 angegebene angegebene Bedeutung haben und
R¹⁵⁻¹ für Cyano, gegebenenfalls durch Halogen, Cyano, Nitro, Alkoxy oder Amino substituiertes Alkyl, für Alkenyl, Alkinyl, jeweils gegebenenfalls subsituiertes Cycloalkyl und Cycloalkylalkyl oder gegebenenfalls substituiertes Aryl steht,
N-Aryl-1,2,4-triazolin-5-one der Formel (Id) 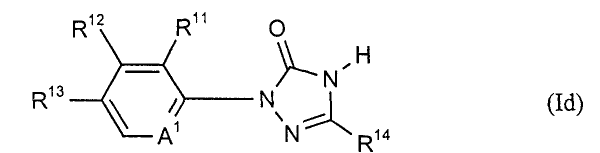 in welcher
R¹¹, R¹², R¹³, R¹⁴ und A¹ die oben angegebene Bedeutung haben,
mit Alkylierungsmitteln der Formel (VI)
E-R¹⁵⁻¹ (VI)
in welcher
R¹⁵⁻¹ die oben angegebene Bedeutung hat und
E für eine elektronenanziehende Abgangsgruppe steht,
gegebenenfalls in Gegenwart eines Verdünnungsmittels und gegebenenfalls in Gegenwart eines Reaktionshilfsmittels umsetzt.

3. Verbindungen der Formel (Ia) gemäß Anspruch 1, in welcher
A¹ für Stickstoff oder die Gruppe CR^{o} steht,
R^{O} für Wasserstoff, Fluor, Chlor, Brom, Nitro, Cyano, Hydroxy, gegebenenfalls durch Fluor, Chlor, Cyano, Nitro oder C₁-C₂-Alkoxy substituiertes C₁-C₄-Alkyl, gegebenenfalls durch Fluor, Chlor, Brom, C₁-C₄-Alkyl, C₁-C₄-Alkoxy, C₁-C₄-Halogenalkyl oder C₁-C₄-Halogenalkoxy substituiertes C₃-C₆-Cycloalkyl, oder für einen der Reste -CONH₂, -CSNH₂, -CO-NH-C₁-C₄-Alkyl, -CS-NH-C₁-C₄-Alkyl, -CO-N(C₁-C₄-Alkyl)₂ oder -CS-N(C₁-C₄-Alkyl)₂ steht,
R¹¹ für Fluor, Chlor, Brom, Nitro, Cyano, jeweils gegebenenfalls durch Fluor, Chlor, Cyano, Nitro oder C₁-C₂-Alkoxy substituiertes C₁-C₄-Alkyl oder C₁-C₄-Alkoxy, gegebenenfalls durch Fluor, Chlor, Brom, C₁-C₄-Alkyl, C₁-C₄-Alkoxy, C₁-C₄-Halogenalkyl oder C₁-C₄-Halogenalkoxy substituiertes C₃-C₆-Cycloalkyl, oder für einen der Reste -CONH₂, -CSNH₂, -CO-NH-C₁-C₄-Alkyl, -CS-NH-C₁-C₄-Alkyl, -CO-N(C₁-C₄-Alkyl)₂ oder -CS-N(C₁-C₄-Alkyl)₂ steht,
R¹² für Wasserstoff, Fluor, Chlor, C₁-C₄-Alkyl oder C₁-C₄-Halogenalkyl steht,
R¹³ für C₁-C₄-Halogenalkyl, C₁-C₄-Halogenalkoxy oder für einen der Reste -S-C₁-C₄-Halogenalkyl, -SO-C₁-C₄-Halogenalkyl oder -SO₂-C₁-C₄-Halogenalkyl steht,
R¹⁴ für Halogen, Cyano, jeweils gegebenenfalls durch Fluor, Chlor, Cyano, Nitro, C₁-C₄-Alkoxy oder Amino substituiertes C₁-C₄-Alkyl oder C₁-C₄-Alkoxy, für C₃-C₆-Alkenyl, C₃-C₆-Alkenyloxy, C₃-C₆-Alkinyl, Hydroxy, Mercapto, jeweils gegebenenfalls durch Fluor, Chlor, Brom, C₁-C₄-Alkyl, C₁-C₄-Alkoxy, C₁-C₄-Halogenalkyl oder C₁-C₄-Halogenalkoxy substituiertes C₃-C₆-Cycloalkyl oder C₃-C₆-Cycloalkyl-C₁-C₄-alkyl, jeweils gegebenenfalls durch Halogen, Cyano, Nitro, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio oder gegebenenfalls durch Halogen oder C₁-C₄-Alkyl substituiertes Phenyl substituiertes Phenyl, Phenyloxy-C₁-C₄-alkyl oder Phenyl-C₁-C₄-alkyl oder für den Rest -S(O)ₙ¹R¹⁶ steht,
R¹⁵ für Wasserstoff, Cyano, Amino, jeweils gegebenenfalls durch Fluor, Chlor, Cyano, Nitro, C₁-C₄-Alkoxy oder Amino substituiertes C₁-C₄-Alkyl oder C₁-C₄-Alkoxy, für C₃-C₆-Alkenyl, C₃-C₆-Alkenyloxy, C₃-C₆-Alkinyl, C₃-C₆-Alkinyloxy, jeweils gegebenenfalls durch Fluor, Chlor, Brom, C₁-C₄-Alkyl, C₁-C₄-Alkoxy, C₁-C₄-Halogenalkyl oder C₁-C₄-Halogenalkoxy substituiertes C₃-C₆-Cycloalkyl oder C₃-C₆-Cycloalkyl-C₁-C₄-alkyl, gegebenenfalls durch Halogen, Cyano, Nitro, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio oder gegebenenfalls durch Halogen oder C₁-C₄-Alkyl substituiertes Phenyl substituiertes Phenyl, gegebenenfalls durch C₁-C₄-Alkyl substituiertes Benzyl oder für einen der Reste -S(O)ₙ¹R¹⁶, -NR¹⁷R¹⁸ oder -N=CR¹⁹R²⁰ steht,
R¹⁶ für gegebenenfalls durch Fluor, Chlor, Cyano oder Nitro substituiertes C₁-C₄-Alkyl, für C₃-C₆-Alkenyl, C₃-C₆-Alkandienyl, C₃-C₆-Alkinyl, gegebenenfalls durch Fluor, Chlor, Brom, C₁-C₄-Alkyl, C₁-C₄-Alkoxy, C₁-C₄-Halogenalkyl oder C₁-C₄-Halogenalkoxy substituiertes C₃-C₆-Cycloalkyl, oder gegebenenfalls durch Halogen, Cyano, Nitro, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio oder gegebenenfalls durch Halogen oder C₁-C₄-Alkyl substituiertes Phenyl substituiertes Phenyl steht,
n¹ für eine der Zahlen 0, 1 oder 2 steht,
R¹⁷ und R¹⁸ gemeinsam mit dem Stickstoffatom, an das sie gebunden sind, für einen gegebenenfalls durch Halogen, C₁-C₄-Alkyl, C₁-C₄-Alkoxy oder C₁-C₄-Alkylthio substituierten, gesättigten fünf- bis siebengliedrigen Heterocyclus mit zusätzlich zum Stickstoffatom 2 oder 3 gleichen oder verschiedenen Heteroatomen aus der Reihe Stickstoff, Sauerstoff und Schwefel stehen,
R¹⁹ für Wasserstoff, gegebenenfalls durch Fluor, Chlor, Cyano, Nitro, Amino oder C₁-C₄-Alkoxy substituiertes C₁-C₄-Alkyl oder für C₁-C₄-Alkoxy steht und
R²⁰ für Wasserstoff, gegebenenfalls durch Fluor, Chlor, Cyano, Nitro, Amino oder C₁-C₄-Alkoxy substituiertes C₁-C₄-Alkyl oder für C₁-C₄-Alkoxy steht
mit Ausnahmen der Verbindungen

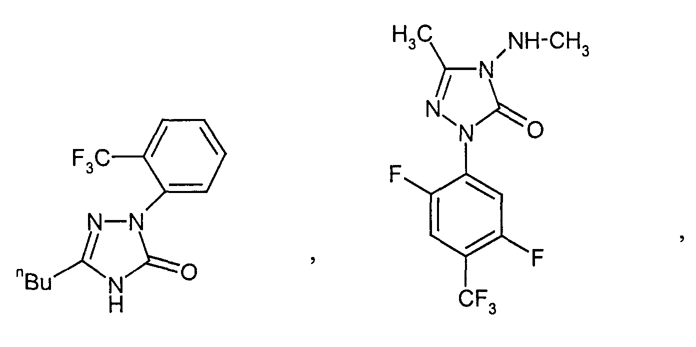

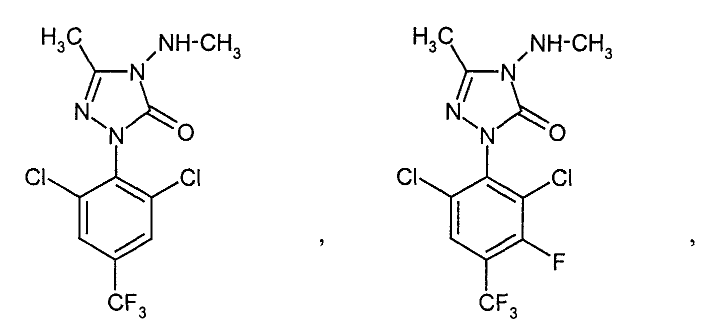

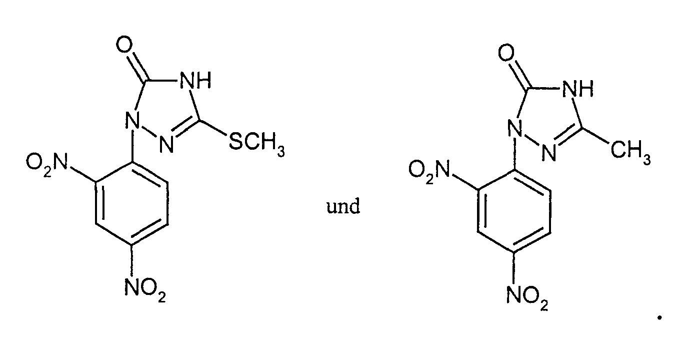

4. Verbindungen der Formel (Ia) gemäß Anspruch 1, in welcher
A¹ für Stickstoff oder die Gruppe CR° steht,
R^{O} für Wasserstoff, Fluor, Chlor, Brom, Nitro, Cyano, Hydroxy, Methyl, Ethyl, n- oder i-Propyl, Methoxymethyl, Trifluormethyl, Difluormethyl, Chlordifluormethyl, Dichlorfluormethyl oder für einen der Reste -CONH₂, -CSNH₂, -CONHCH₃, -CSNHCH₃, -CON(CH₃)₂ oder -CSN(CH₃)₂ steht,
R¹¹ für Fluor, Chlor, Brom, Nitro, Cyano, Methyl, Ethyl, n- oder i-Propyl, Methoxymethyl, Trifluormethyl, Difluormethyl, Chlordifluormethyl, Dichlorfluormethyl, Trifluormethoxy, Difluormethoxy, Trifluorethoxy oder für einen der Reste -CONH₂, -CSNH₂, -CONHCH₃, -CSNHCH₃, -CON(CH₃)₂ und -CSN(CH₃)₂ steht,
R¹² für Wasserstoff, Fluor, Chlor, Methyl oder Trifluormethyl steht,
R¹³ für Trifluormethyl, Difluormethyl, Chlordifluormethyl, Dichlorfluormethyl, Trifluormethoxy, Difluormethoxy, Chlordifluormethoxy, Trifluormethylsulfenyl, Trifluormethylsulfinyl oder Trifluormethylsulfonyl steht,
R¹⁴ für Chlor, Brom, Cyano, Methyl, Ethyl, n- oder i-Propyl, n-, i-, s- oder t-Butyl, Methoxy, Ethoxy, n- oder i-Propoxy, n-, i-, s- oder t-Butoxy, Methoxymethyl, Methoxyethyl, Ethoxymethyl, Ethoxyethyl, Trifluormethyl, Difluormethyl, Chlordifluormethyl, Dichlorfluormethyl, Trifluorethyl, Tetrafluorethyl, Pentafluorethyl, Fluorpropyl, Trifluormethoxy, Difluormethoxy, Chlordifluormethoxy, Dichlorfluormethoxy, Trifluorethoxy, Tetrafluorethoxy, Pentafluorethoxy, Cyanmethyl, Allyl, Allyloxy, Propargyl, Hydroxy, Mercapto, jeweils gegebenenfalls durch Fluor, Chlor, Brom, Methyl, Ethyl, Methoxy, Trifluormethyl oder Trifluormethoxy substituiertes Cyclopropyl, Cyclopropylmethyl, Cyclopentyl, Cyclopentylmethyl, Cyclohexyl oder Cyclohexylmethyl, jeweils gegebenenfalls durch Fluor, Chlor, Methyl, Methoxy, Trifluormethyl, Trifluormethoxy, Methylthio, Trifluormethylthio, Nitro oder gegebenenfalls durch Fluor, Chlor oder Methyl substituiertes Phenyl substituiertes Phenyl, Phenoxymethyl oder Benzyl oder für den Rest -S(O)ₙ¹R¹⁶ steht,
R¹⁵ für Wasserstoff, Cyano, Amino, Methyl, Ethyl, n- oder i-Propyl, n-, i-, s- oder t-Butyl, Methoxy, Ethoxy, n- oder i-Propoxy, n-, i-, s- oder t-Butoxy, Methoxymethyl, Methoxyethyl, Ethoxymethyl, Ethoxyethyl, Difluormethyl, Chlordifluormethyl, Dichlorfluormethyl, Trifluorethyl, Fluorpropyl, Difluormethoxy, Chlordifluormethoxy, Dichlorfluormethoxy, Cyanmethyl, Allyl, Allyloxy, Propargyl, Propargyloxy, Butinyloxy, jeweils gegebenenfalls durch Fluor, Chlor, Brom, Methyl, Ethyl, Methoxy, Trifluormethyl oder Trifluormethoxy substituiertes Cyclopropyl, Cyclopropylmethyl, Cyclopentyl, Cyclopentylmethyl, Cyclohexyl oder Cyclohexylmethyl, gegebenenfalls durch Fluor, Chlor, Methyl, Methoxy, Trifluormethyl, Trifluormethoxy, Methylthio, Trifluormethylthio, Cyano, Nitro sowie gegebenenfalls durch Fluor, Chlor oder Methyl substituiertes Phenyl substituiertes Phenyl, für gegebenenfalls durch Methyl, Ethyl, n- oder i-Propyl, n-, i-, s- oder t-Butyl substituiertes Benzyl oder für einen der Reste -S(O)ₙ¹R¹⁶, -NR¹⁷R¹⁸ oder -N=CR¹⁹R²⁰ steht,
R¹⁶ für Methyl, Ethyl, n- oder i-Propyl, n-, i-, s- oder t-Butyl, Trifluormethyl, Difluormethyl, Chlordifluormethyl, Dichlorfluormethyl, Trifluorethyl, Tetrafluorethyl, Pentafluorethyl, Fluorpropyl, Allyl, Allenyl, Propargyl, jeweils gegebenenfalls durch Fluor, Chlor, Brom, Methyl, Ethyl, Methoxy, Trifluormethyl oder Trifluormethoxy substituiertes Cyclopropyl, Cyclopentyl oder Cyclohexyl, oder gegebenenfalls durch Fluor, Chlor, Methyl, Methoxy, Trifluormethyl, Trifluormethoxy, Methylthio, Trifluormethylthio, Cyano, Nitro oder gegebenenfalls durch Fluor, Chlor oder Methyl substituiertes Phenyl substituiertes Phenyl steht,
n¹ für eine der Zahlen 0, 1 oder 2 steht,
R¹⁷ und R¹⁸ mit dem Stickstoffatom, an das sie gebunden sind, für jeweils gegebenenfalls durch Methyl, Ethyl, Methoxy oder Ethoxy substituiertes Piperidinyl, Piperazinyl oder Morpholinyl stehen,
R¹⁹ für Wasserstoff, Methyl, Ethyl, n- oder i-Propyl, n-, i-, s- oder t-Butyl, Methoxy oder Ethoxy steht und
R²⁰ für Wasserstoff, Methyl, Ethyl, n- oder i-Propyl, n-, i-, s- oder t-Butyl, Methoxy oder Ethoxy steht,
mit Ausnahme der Verbindungen

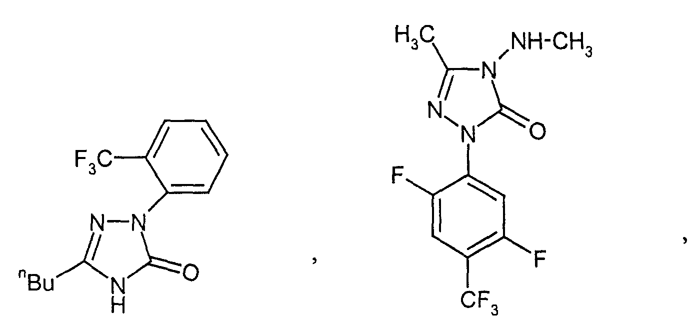

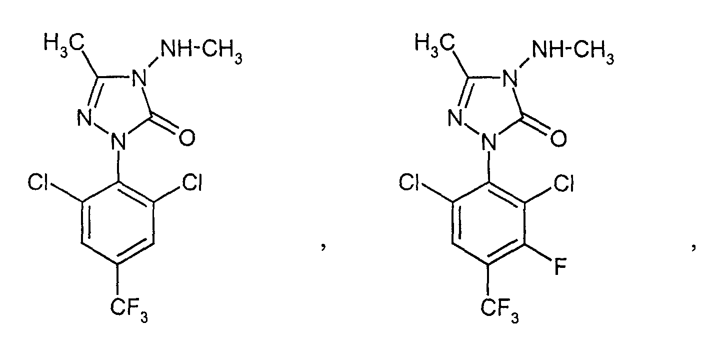

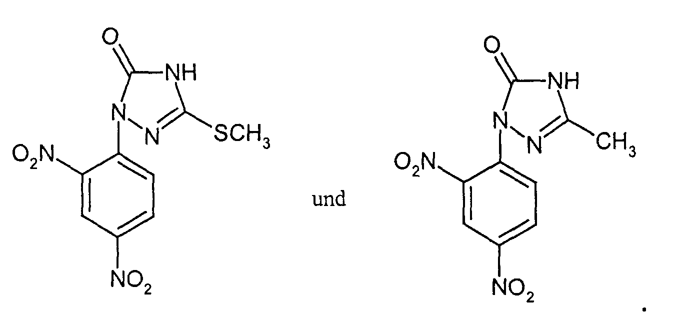

5. Mittel zur Bekämpfung tierischer Schädlinge, gekennzeichnet durch einen Gehalt an mindestens einer Verbindung der Formel (Ia) gemäß Anspruch 1.

6. Verfahren zur Bekämpfung von tierischen Schädlingen, **dadurch gekennzeichnet**, daß man Verbindungen der Formel (Ia) gemäß Anspruch 1 auf Schädlinge und/oder ihren Lebensraum einwirken läßt.

7. Verwendung von Verbindungen der Formel (Ia) gemäß Anspruch 1 zur Bekämpfung von tierischen Schädlingen.

8. Verfahren zur Herstellung von Schädlingsbekämpfungsmitteln, **dadurch gekennzeichnet**, daß man Verbindungen der Formel (Ia) gemäß Anspruch 1 mit Streckmitteln und/oder oberflächenaktiven Stoffen vermischt.

9. Verwendung von Verbindungen der Formel (Ia) gemäß Anspruch 1 zur Herstellung von Schädlingsbekämpfungsmitteln.

## Claims

1. Compounds of the formula (Ia) 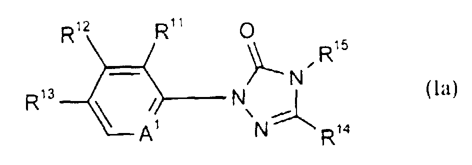 in which
A¹ represents nitrogen or the group CR°,
R^{o} represents hydrogen, halogen, nitro, cyano, hydroxyl, optionally halogen-, cyano-, nitro-, alkoxy- or amino-substituted alkyl, optionally substituted cycloalkyl or the radical -CX¹-NY¹¹Y¹²,
R¹¹ represents halogen, nitro, cyano, respectively optionally halogen-, cyano-, nitro-, alkoxy- or amino-substituted alkyl or alkoxy, optionally substituted cycloalkyl or the radical -CX¹-NY¹¹Y¹²,
R¹² represents hydrogen, halogen, optionally halogen-, cyano-, nitro-, alkoxy- or amino-substituted alkyl or optionally substituted cycloalkyl,
R¹³ represents, halogenoalkyl, halogenoalkoxy or the radical -S(O)ₙ¹R¹⁶,
R¹⁴ represents halogen, cyano, respectively optionally halogen-, cyano-, nitro-, alkoxy- or amino-substituted alkyl or alkoxy, represents alkenyl, alkenyloxy, alkinyl, hydroxyl, mercapto, respectively optionally substituted cycloalkyl or cycloalkylalkyl, respectively optionally substituted aryl, aryloxyalkyl or aralkyl or the radical -S(O)ₙ¹R¹⁶,
R¹⁵ represents hydrogen, cyano, amino, respectively optionally halogen-, cyano-, nitro-, alkoxy- or amino-substituted alkyl or alkoxy, represents alkenyl, alkenyloxy, alkinyl, alkinyloxy, respectively optionally substituted cycloalkyl or cycloalkylalkyl, respectively optionally substituted aryl or aralkyl or one of the radicals -S(O)ₙ¹R¹⁶, NR¹⁷R¹⁸ or -N=CR¹⁹R²⁰,
R¹⁶ represents optionally halogen-, cyano-, nitro-, alkoxy- or amino-substituted alkyl, represents alkenyl, alkanedienyl, alkinyl, optionally substituted cycloalkyl or optionally substituted aryl,
R¹⁷ and R¹⁸ join with the linking nitrogen atom to represent an optionally substituted heterocycle which may optionally contain one or more additional hetero atoms,
R¹⁹ represents hydrogen, optionally halogen-, cyano-, nitro-, alkoxy- or amino-substituted alkyl, represents alkoxy, optionally substituted cycloalkyl or respectively optionally substituted aryl or heterocyclyl,
R²⁰ represents hydrogen, optionally halogen-, cyano-, nitro-, alkoxy- or amino-substituted alkyl, represents alkoxy, optionally substituted cycloalkyl or respectively optionally substituted aryl or heterocyclyl,
X¹ represents oxygen or sulphur,
Y¹¹ represents hydrogen or alkyl,
Y¹² represents hydrogen or alkyl, and
n¹ represents one of the numbers 0, 1 or 2,
excluding the compounds

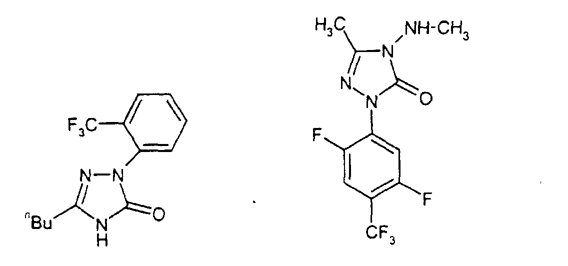

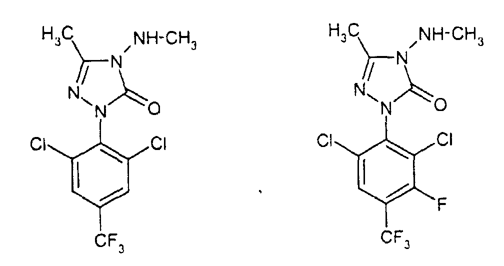

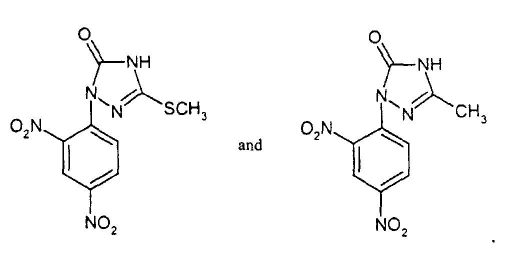

2. Process for preparing compounds of the formula (Ia) according to Claim 1, **characterized in that**
a) compounds of the formula (Ia) 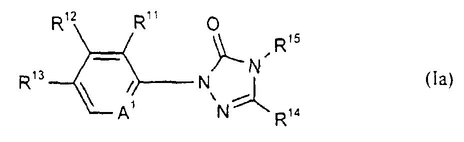 in which
R¹¹, R¹², R¹³, R¹⁴, R¹⁵ and A¹ are each as defined in Claim 1
are obtained when 1H-triazolinones of the formula (II) 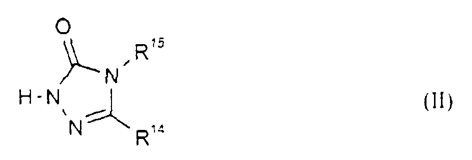 in which
R¹⁴ and R¹⁵ are each as defined in Claim 1
are reacted with halogen derivatives of the formula (III) 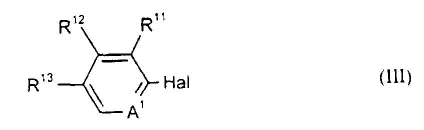 in which
R¹¹, R¹², R¹³ and A¹ are each as defined above and
Hal represents halogen,
if appropriate in the presence of a diluent and if appropriate in the presence of a reaction auxiliary;
b) compounds of the formula (Ib) 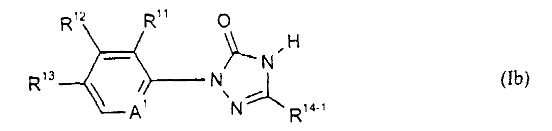 in which
R¹¹, R¹², R¹³, and A¹ are each as defined in Claim 1 and
R¹⁴⁻¹ represents cyano, respectively optionally halogen-, cyano-, nitro-, alkoxy- or amino-substituted alkyl or alkoxy, represents alkenyl, alkenyloxy, alkinyl, respectively optionally substituted cycloalkyl or cycloalkylalkyl or respectively optionally substituted aryl, aryloxyalkyl or aralkyl,
are obtained when hydrazine derivatives of the formula (IV) 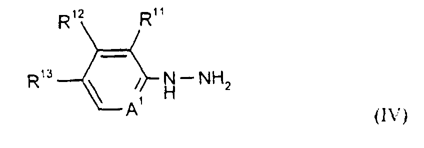 in which
R¹¹, R¹², R¹³, and A¹ are each as defined in Claim 1
are reacted with iminocarboxylic esters of the formula (V) 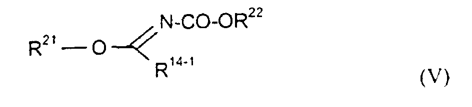 in which
R¹⁴⁻¹ is as defined above and
R²¹ and R²² independently of one another each represent alkyl (preferably C₁-C₈-alkyl),
in the presence of a diluent;
c) compounds of the formula (Ic) 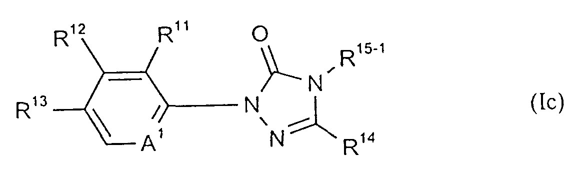 in which
R¹¹, R¹², R¹³, R¹⁴, and A¹ are each as defined in Claim 1 and
R¹⁵⁻¹ represents cyano, optionally halogen-, cyano-, nitro-, alkoxy- or amino-substituted alkyl, represents alkenyl, alkinyl, respectively optionally substituted cycloalkyl and cycloalkylalkyl or optionally substituted aryl,
are obtained when N-aryl-1,2,4-triazolin-5-ones of the formula (Id) 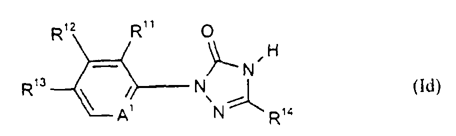 in which
R¹¹, R¹², R¹³, R¹⁴, and A¹ are each as defined above
are reacted with alkylating agents of the formula (VI)
E-R¹⁵⁻¹ (VI)
in which
R¹⁵⁻¹ is as defined above and
E represents an electron-withdrawing leaving group,
if appropriate in the presence of a diluent and if appropriate in the presence of a reaction auxiliary.

3. Compounds of the formula (Ia) according to Claim 1 in which
A¹ represents nitrogen or the group CR°,
R^{o} represents hydrogen, fluorine, chlorine, bromine, nitro, cyano, hydroxyl, optionally fluorine-, chlorine-, cyano-, nitro- or C₁-C₂-alkoxysubstituted C₁-C₄-alkyl, optionally fluorine-, chlorine-, bromine-, C₁-C₄-alkyl-, C₁-C₄-alkoxy-, C₁-C₄-halogenoalkyl- or C₁-C₄-halogenoalkoxysubstituted C₃-C₆-cycloalkyl, or represents one of the radicals -CONH₂, -CSNH₂, -CO-NH-C₁-C₄-alkyl, -CS-NH-C₁-C₄-alkyl, -CO-N(C₁-C₄-alkyl)₂, or -CS-N(C₁-C₄-alkyl)₂,
R¹¹ represents fluorine, chlorine, bromine, nitro, cyano, respectively optionally fluorine-, chlorine-, cyano-, nitro- or C₁-C₂-alkoxysubstituted C₁-C₄-alkyl or C₁-C₄-alkoxy, optionally fluorine-, chlorine-, bromine-, C₁-C₄-alkyl-, C₁-C₄-alkoxy-, C₁-C₄-halogenoalkyl- or C₁-C₄-halogenoalkoxy-substituted C₃-C₆-cycloalkyl, or represents one of the radicals -CONH₂, -CSNH₂, -CO-NH-C₁-C₄-alkyl, -CS-NH-C₁-C₄-alkyl, -CO-N(C₁-C₄-alkyl)₂ or -CS-N(C₁-C₄-alkyl)₂,
R¹² represents hydrogen, fluorine, chlorine, C₁-C₄-alkyl or C₁-C₄-halogenoalkyl,
R¹³ represents C₁-C₄-halogenoalkyl, C₁-C₄-halogenoalkoxy or one of the radicals -S-C₁-C₄-halogenoalkyl, -SO-C₁-C₄-halogenoalkyl, or -SO₂-C₁-C₄-halogenoalkyl,
R¹⁴ represents halogen, cyano, respectively optionally fluorine-, chlorine-, cyano-, nitro-, C₁-C₄-alkoxy- or amino-substituted C₁-C₄-alkyl or C₁-C₄-alkoxy, represents C₃-C₆-alkenyl, C₃-C₆-alkenyloxy, C₃-C₆-alkinyl, hydroxyl, mercapto, respectively optionally fluorine-, chlorine-, bromine-, C₁-C₄-alkyl-, C₁-C₄-alkoxy-, C₁-C₄-halogenoalkyl- or C₁-C₄-halogenoalkoxysubstituted C₃-C₆-cycloalkyl, or C₃-C₆-cycloalkyl-C₁-C₄-alkyl, represents phenyl, phenyloxy-C₁-C₄-alkyl or phenyl-C₁-C₄-alkyl, each of which is optionally substituted by halogen, cyano, nitro, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio or optionally halogen- or C₁-C₄-alkyl-substituted phenyl, or represents the radical -S(O)ₙ¹R¹⁶,
R¹⁵ represents hydrogen, cyano, amino, respectively optionally fluorine-, chlorine-, cyano-, nitro-, C₁-C₄-alkoxy- or amino-substituted C₁-C₄-alkyl or C₁-C₄-alkoxy, represents C₃-C₆-alkenyl, C₃-C₆-alkenyloxy, C₃-C₆-alkinyl, C₃-C₆-alkinyloxy, respectively optionally fluorine-, chlorine-, bromine-, C₁-C₄-alkyl-, C₁-C₄-alkoxy-, C₁-C₄-halogenoalkyl- or C₁-C₄-halogenoalkoxysubstituted C₃-C₆-cycloalkyl or C₃-C₆-cycloalkyl-C₁-C₄-alkyl, represents phenyl which is optionally substituted by halogen, cyano, nitro, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio or optionally halogen- or C₁-C₄-alkyl-substituted phenyl, represents optionally C₁-C₄-alkylsubstituted benzyl or one of the radicals -S(O)ₙ¹R¹⁶, -NR¹⁷R¹⁸ or -N=CR¹⁹R²⁰,
R¹⁶ represents optionally fluorine-, chlorine-, cyano- or nitro-substituted C₁-C₄-alkyl, represents C₃-C₆-alkenyl, C₃-C₆-alkanedienyl, C₃-C₆-alkinyl, optionally fluorine-, chlorine-, bromine-, C₁-C₄-alkyl-, C₁-C₄-alkoxy-, C₁-C₄-halogenoalkyl- or C₁-C₄-halogenoalkoxysubstituted C₃-C₆-cycloalkyl, or phenyl which is optionally substituted by halogen, cyano, nitro, C₁-C₄-alkyl, C₁-C₄-alkoxy, C₁-C₄-alkylthio, C₁-C₄-halogenoalkyl, C₁-C₄-halogenoalkoxy, C₁-C₄-halogenoalkylthio or optionally halogen- or C₁-C₄-alkyl-substituted phenyl,
n¹ represents one of the numbers 0, 1 or 2,
R¹⁷ and R¹⁸ join with the linking nitrogen atom to represent an optionally halogen-, C₁-C₄-alkyl-, C₁-C₄-alkoxy- or C₁-C₄-alkylthio-substituted saturated 5- to 7-membered heterocycle having, in addition to the nitrogen atom, 2 or 3 identical or different hetero atoms from the group consisting of nitrogen, oxygen and sulphur,
R¹⁹ represents hydrogen, optionally fluorine-, chlorine-, cyano-, nitro-, amino- or C₁-C₄-alkoxy-substituted C₁-C₄-alkyl or represents C₁-C₄-alkoxy and
R²⁰ represents hydrogen, optionally fluorine-, chlorine-, cyano-, nitro-, amino- or C₁-C₄-alkoxy-substituted C₁-C₄-alkyl or represents C₁-C₄-alkoxy,
excluding the compounds

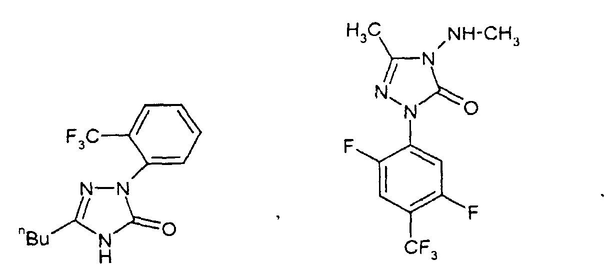

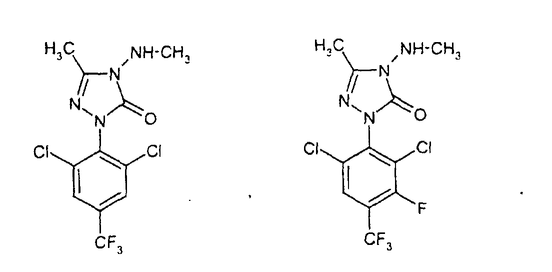

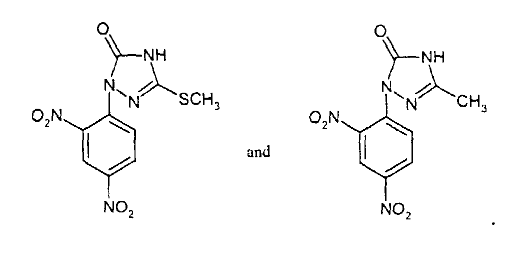

4. Compounds of the formula (Ia) according to Claim 1 in which
A¹ represents nitrogen or the group CR°,
R^{O} represents hydrogen, fluorine, chlorine, bromine, nitro, cyano, hydroxyl, methyl, ethyl, n- or i-propyl, methoxymethyl, trifluoromethyl, difluoromethyl, chlorodifluoromethyl, dichlorofluoromethyl or represents one of the radicals -CONH₂, -CSNH₂, -CONHCH₃, -CSNHCH₃, -CON(CH₃)₂ or -CSN(CH₃)₂,
R¹¹ represents fluorine, chlorine, bromine, nitro, cyano, methyl, ethyl, n- or i-propyl, methoxymethyl, trifluoromethyl, difluoromethyl, chlorodifluoromethyl, dichlorofluoromethyl, trifluoromethoxy, difluoromethoxy, trifluoroethoxy or one of the radicals -CONH₂, -CSNH₂, -CONHCH₃, -CSNHCH₃, -CON(CH₃)₂ and -CSN(CH₃)₂,
R¹² represents hydrogen, fluorine, chlorine, methyl or trifluoromethyl,
R¹³ represents trifluoromethyl, difluoromethyl, chlorodifluoromethyl, dichlorofluoromethyl, trifluoromethoxy, difluoromethoxy, chlorodifluoromethoxy, trifluoromethylsulphenyl, trifluoromethyl-sulphinyl or trifluoromethylsulphonyl,
R¹⁴ represents chlorine, bromine, cyano, methyl, ethyl, n- or i-propyl, n-, i-, s- or t-butyl, methoxy, ethoxy, n- or i-propoxy, n-, i-, s- or t-butoxy, methoxymethyl, methoxyethyl, ethoxymethyl, ethoxyethyl, trifluoromethyl, difluoromethyl, chlorodifluoromethyl, dichlorofluoromethyl, trifluoroethyl, tetrafluoroethyl, pentafluoroethyl, fluoropropyl, trifluoromethoxy, difluoromethoxy, chlorodifluoromethoxy, dichlorofluoromethoxy, trifluoroethoxy, tetrafluoroethoxy, pentafluoroethoxy, cyanomethyl, allyl, allyloxy, propargyl, hydroxyl, mercapto, respectively optionally fluorine-, chlorine-, bromine-, methyl-, ethyl-, methoxy-, trifluoromethyl- or trifluoromethoxy-substituted cyclopropyl, cyclopropylmethyl, cyclopentyl, cyclopentylmethyl, cyclohexyl or cyclohexylmethyl, represents phenyl, phenoxymethyl or benzyl, each of which is optionally substituted by fluorine, chlorine, methyl, methoxy, trifluoromethyl, trifluoromethoxy, methylthio, trifluoromethylthio, nitro, or optionally fluorine-, chlorine- or methyl-substituted phenyl, or represents the radical -S(O)ₙ¹R¹⁶,
R¹⁵ represents hydrogen, cyano, amino, methyl, ethyl, n- or i-propyl, n-, i-, s- or t-butyl, methoxy, ethoxy, n- or i-propoxy, n-, i-, s- or t-butoxy, methoxymethyl, methoxyethyl, ethoxymethyl, ethoxyethyl, difluoromethyl, chlorodifluoromethyl, dichlorofluoromethyl, trifluoroethyl, fluoropropyl, difluoromethoxy, chlorodifluoromethoxy, dichlorofluoromethoxy, cyanomethyl, allyl, allyloxy, propargyl, propargyloxy, butinyloxy, respectively optionally fluorine-, chlorine-, bromine-, methyl-, ethyl-, methoxy-, trifluoromethyl- or trifluoromethoxy-substituted cyclopropyl, cyclopropylmethyl, cyclopentyl, cyclopentylmethyl, cyclohexyl or cyclohexylmethyl, represents phenyl which is optionally substituted by fluorine, chlorine, methyl, methoxy, trifluoromethyl, trifluoromethoxy, methylthio, trifluoromethylthio, cyano, nitro, or optionally fluorine-, chlorine- or methyl-substituted phenyl, represents optionally methyl-, ethyl-, n- or i-propyl-, n-, i-, s- or t-butyl-substituted benzyl or represents one of the radicals -S(O)ₙ¹R¹⁶, NR¹⁷R¹⁸ or -N=CR¹⁹R²⁰,
R¹⁶ represents methyl, ethyl, n- or i-propyl, n-, i-, s- or t-butyl, trifluoromethyl, difluoromethyl, chlorodifluoromethyl, dichlorofluoromethyl, trifluoroethyl, tetrafluoroethyl, pentafluoroethyl, fluoropropyl, allyl, allenyl, propargyl, respectively optionally fluorine-, chlorine-, bromine-, methyl-, ethyl-, methoxy-, trifluoromethyl- or trifluoromethoxy-substituted cyclopropyl, cyclopentyl or cyclohexyl, or phenyl which is optionally substituted by fluorine, chlorine, methyl, methoxy, trifluoromethyl, trifluoromethoxy, methylthio, trifluoromethylthio, cyano, nitro, or optionally fluorine-, chlorine- or methyl-substituted phenyl,
n¹ represents one of the numbers 0, 1 or 2,
R¹⁷ and R¹⁸ join with the linking nitrogen atom to represent respectively optionally methyl-, ethyl-, methoxy- or ethoxy-substituted piperidinyl, piperazinyl or morpholinyl,
R¹⁹ represents hydrogen, methyl, ethyl, n- or i-propyl, n-, i-, s- or t-butyl, methoxy or ethoxy and
R²⁰ represents hydrogen, methyl, ethyl, n- or i-propyl, n-, i-, s- or t-butyl, methoxy or ethoxy,
excluding the compounds

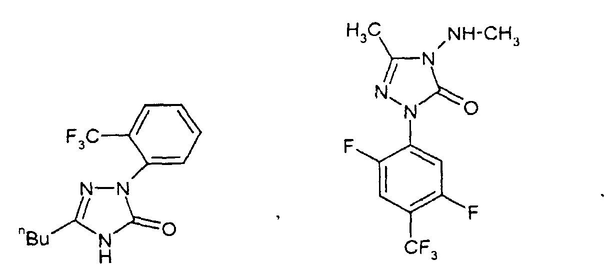

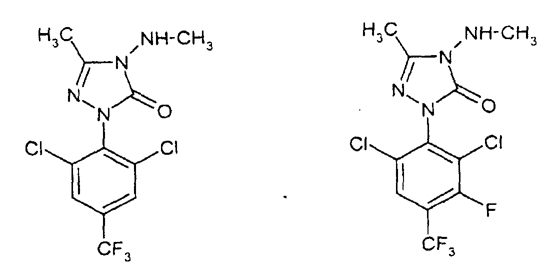

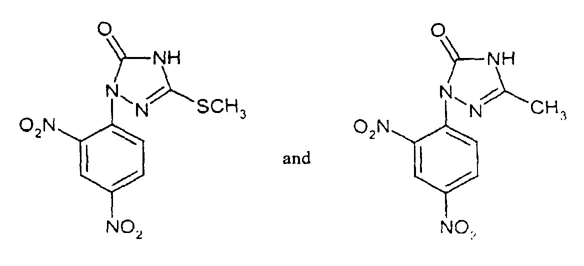

5. Composition for controlling animal pests, **characterized in that** it comprises at least one compound of the formula (Ia) according to Claim 1.

6. Method for controlling animals pests, **characterized in that** compounds of the formula (Ia) according to Claim 1 are allowed to act on pests and/or their habitat.

7. Use of compounds of the formula (Ia) according to Claim 1 for controlling animal pests.

8. Process for preparing pesticides, **characterized in that** compounds of the formula (Ia) according to Claim 1 are mixed with extenders and/or surfactants.

9. Use of compounds of the formula (Ia) according to Claim 1 for preparing pesticides.

## Revendications

1. Composés de formule (Ia) 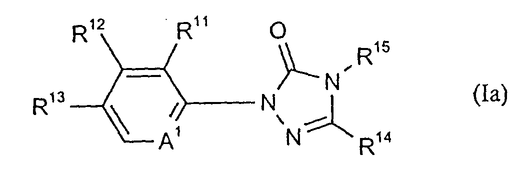 dans laquelle
A¹ représente l'azote ou le groupe CR⁰,
R⁰ est l'hydrogène, un halogène, un groupe nitro, cyano, hydroxy, un groupe alkyle portant éventuellement un substituant halogéno, cyano, nitro, alkoxy ou amino, un groupe cycloalkyle éventuellement substitué ou le reste -CX¹-NY¹¹Y¹²,
R¹¹ représente un halogène, un groupe nitro, cyano, un groupe alkyle ou alkoxy portant chacun éventuellement un substituant halogéno, cyano, nitro, alkoxy ou amino, un groupe cycloalkyle éventuellement substitué ou le reste -CX¹-NY¹¹Y¹²,
R¹² représente l'hydrogène, un halogène, un groupe alkyle portant éventuellement un substituant halogéno, cyano, nitro, alkoxy ou amino, ou un groupe cycloalkyle éventuellement substitué,
R¹³ est un groupe halogénalkyle, halogénalkoxy ou le reste -S(O)ₙ¹R¹⁶,
R¹⁴ représente un halogène, un groupe cyano, un groupe alkyle ou alkoxy portant chacun le cas échéant un substituant halogéno, cyano, nitro, alkoxy ou amino, un groupe alcényle, alcényloxy, alcynyle, hydroxy, mercapto, un groupe cycloalkyle ou cycloalkylalkyle dont chacun est éventuellement substitué, un groupe aryle, aryloxyalkyle ou aralkyle dont chacun est éventuellement substitué ou le reste -S(O)ₙ¹R¹⁶,
R¹⁵ représente l'hydrogène, un groupe cyano, amino, un groupe alkyle ou alkoxy dont chacun porte éventuellement un substituant halogéno, cyano, nitro, alkoxy ou amino, un groupe alcényle, alcényloxy, alcynyle, alcynyloxy, un groupe cycloalkyle ou cycloalkylalkyle dont chacun est éventuellement substitué, un groupe aryle ou aralkyle dont chacun est éventuellement substitué ou l'un des restes -S(O)ₙ¹R¹⁶, -NR¹⁷R¹⁸ ou -N=CR¹⁹R²⁰,
R¹⁶ est un groupe alkyle portant éventuellement un substituant halogéno, cyano, nitro, alkoxy ou amino, un groupe alcényle, alcanediényle, alcynyle, un groupe cycloalkyle éventuellement substitué ou un groupe aryle éventuellement substitué,
R¹⁷ et R¹⁸ forment conjointement avec l'atome d'azote auquel ils sont liés un hétérocycle éventuellement substitué qui contient le cas échéant un ou plusieurs autres hétéroatomes,
R¹⁹ représente l'hydrogène, un groupe alkyle portant éventuellement un substituant halogéno, cyano, nitro, alkoxy ou amino, un groupe alkoxy, un groupe cycloalkyle éventuellement substitué ou un groupe aryle ou hétérocyclyle dont chacun est éventuellement substitué,
R²⁰ représente l'hydrogène, un groupe alkyle portant éventuellement un substituant halogéno, cyano, nitro, alkoxy ou amino, un groupe alkoxy, un groupe cycloalkyle éventuellement substitué ou un groupe aryle ou hétérocyclyle dont chacun est éventuellement substitué,
X¹ représente l'oxygène ou le soufre,
Y¹¹ représente l'hydrogène ou un groupe alkyle,
Y¹² représente l'hydrogène ou un groupe alkyle, et
n¹ représente le nombre 0, 1 ou 2,
à l'exception des composés

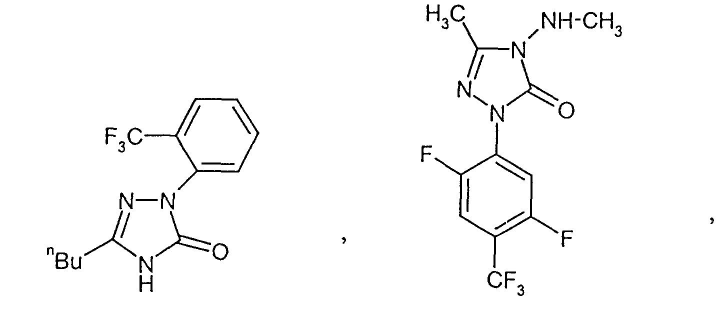

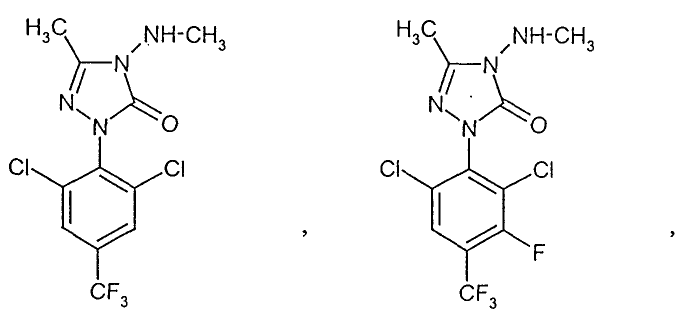

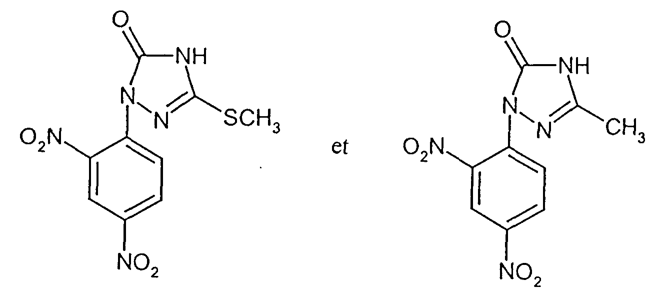

2. Procédé de production de composés de formule (Ia) suivant la revendication 1, **caractérisé en ce qu**'on obtient
a) des composés de formule (Ia) 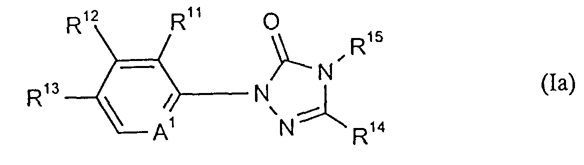 dans laquelle
R¹¹, R¹², R¹³, R¹⁴, R¹⁵ et A¹ ont la définition indiquée dans la revendication 1,
en faisant réagir des 1H-triazolinones de formule (II) 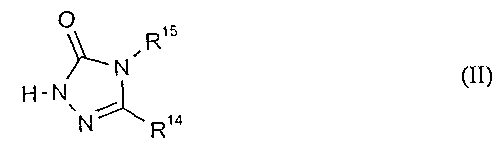 dans laquelle
R¹⁴ et R¹⁵ ont la définition indiquée dans la revendication 1,
avec des dérivés halogénés de formule (III) 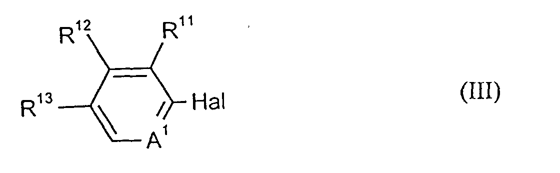 dans laquelle
R¹¹, R¹², R¹³ et A¹ ont la définition indiquée
ci-dessus et
Hal représente un halogène,
le cas échéant en présence d'un diluant et en présence éventuelle d'un auxiliaire de réaction ;
b) des composés de formule (Ib) 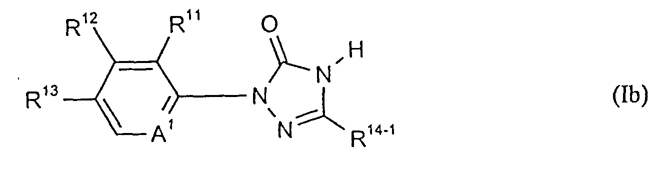 dans laquelle
R¹¹, R¹², R¹³ et A¹ ont la définition indiquée dans la revendication 1 et
R¹⁴⁻¹ représente un groupe cyano, un groupe alkyle ou alkoxy dont chacun est éventuellement substitué par un radical halogéno, cyano, nitro, alkoxy ou amino,
un groupe alcényle, alcényloxy, alcynyle, un groupe cycloalkyle ou cycloalkylalkyle dont chacun est éventuellement substitué ou un groupe aryle, aryloxyalkyle ou aralkyle dont chacun est éventuellement substitué,
en faisant réagir des dérivés d'hydrazine de formule (IV) 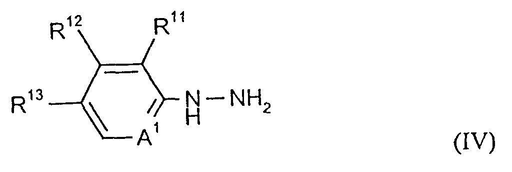 dans laquelle
R¹¹, R¹², R¹³ et A¹ ont la définition indiquée dans la revendication 1,
avec des esters d'acides iminocarboxyliques de formule (V) 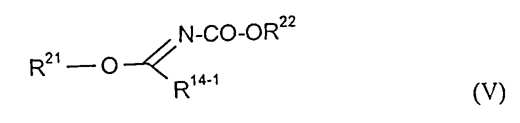 dans laquelle
R¹⁴⁻¹ a la définition indiquée dans la revendication 1 et
R²¹ et R²² représentent indépendamment l'un de l'autre un groupe alkyle (de préférence alkyle en C₁ à C₈,
en présence d'un diluant ;
c) des composés de formule (Ic) 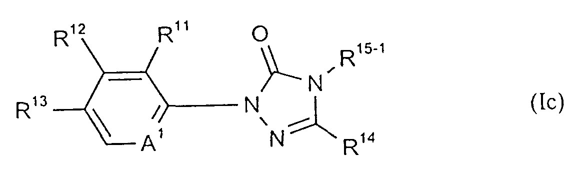 dans laquelle
R¹¹, R¹², R¹³, R¹⁴ et A¹ ont la définition indiquée dans la revendication 1 et
R¹⁵⁻¹ représente un groupe cyano, un groupe alkyle éventuellement substitué par un radical halogéno, cyano, nitro, alkoxy ou amino, un groupe alcényle, alcynyle, des groupes cycloalkyle et cycloalkylalkyle dont chacun est éventuellement substitué ou un groupe aryle éventuellement substitué,
en faisant réagir des N-aryl-1,2,4-triazoline-5-ones de formule (Id) 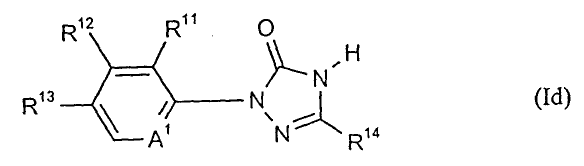 dans laquelle
R¹¹, R¹², R¹³, R¹⁴ et A¹ ont la définition indiquée ci-dessus,
avec des agents d'alkylation de formule (VI)
E-R¹⁵⁻¹ (VI)
dans laquelle
R¹⁵⁻¹ a la définition indiquée ci-dessus et
E représente un groupe partant attirant les électrons,
éventuellement en présence d'un diluant et en présence éventuelle d'un auxiliaire de réaction.

3. Composés de formule (Ia) suivant la revendication 1, dans laquelle
A¹ représente l'azote ou le groupe CR⁰,
R⁰ est l'hydrogène, le fluor, le chlore, le brome, un groupe nitro, cyano, hydroxy, un groupe alkyle en C₁ à C₄ portant éventuellement un substituant fluoro, chloro, cyano, nitro ou alkoxy en C₁ ou C₂, un groupe cycloalkyle en C₃ à C₆ portant éventuellement un substituant fluoro, chloro, bromo, alkyle en C₁ à C₄, alkoxy en C₁ à C₄, halogénalkyle en C₁ à C₄ ou halogénalkoxy en C₁ à C₄, ou l'un des restes -CONH₂, -CSNH₂, -CO-NH- (alkyle en C₁ à C₄), -CS-NH-(alkyle en C₁ à C₄), -CO-N-(alkyle en C₁ à C₄)₂ ou -CS-N-(alkyle en C₁ à C₄)₂,
R¹¹ est le fluor, le chlore, le brome, un groupe nitro, cyano, un groupe alkyle en C₁ à C₄ ou alkoxy en C₁ à C₄ dont chacun porte éventuellement un substituant fluoro, chloro, cyano, nitro ou alkoxy en C₁ ou C₂, un groupe cycloalkyle en C₃ à C₆ portant éventuellement un substituant fluoro, chloro, bromo, alkyle en C₁ à C₄, alkoxy en C₁ à C₄, halogénalkyle en C₁ à C₄ ou halogénalkoxy en C₁ à C₄, ou l'un des restes -CONH₂, -CSNH₂, -CO-NH-(alkyle en C₁ à C₄), -CS-NH-(alkyle en C₁ à C₄), -CO-N-(alkyle en C₁ à C₄)₂ ou -CS-N-(alkyle en C₁ à C₄)₂,
R¹² représente l'hydrogène, le fluor, le chlore, un groupe alkyle en C₁ à C₄ ou halogénalkyle en C₁ à C₄,
R¹³ est un groupe halogénalkyle en C₁ à C₄, halogénalkoxy en C₁ à C₄ ou l'un des restes -S-(halogénalkyle en C₁ à C₄), -SO-(halogénalkyle en C₁ à C₄) ou -SO₂-(halogénalkyle en C₁ à C₄),
R¹⁴ représente un halogène, un groupe cyano, un groupe alkyle en C₁ à C₄ ou alkoxy en C₁ à C₄ dont chacun porte éventuellement un substituant fluoro, chloro, cyano, nitro, alkoxy en C₁ à C₄ ou amino, un groupe alcényle en C₃ à C₆, alcényloxy en C₃ à C₆, alcynyle en C₃ à C₆, hydroxy, mercapto, un groupe cycloalkyle en C₃ à C₆ ou (cycloalkyle en C₃ à C₆)-(alkyle en C₁ à C₄) portant chacun éventuellement un substituant fluoro, chloro, bromo, alkyle en C₁ à C₄, alkoxy en C₁ à C₄, halogénalkyle en C₁ à C₄ ou halogénalkoxy en C₁ à C₄, un groupe phényle, phényloxy-(alkyle en C₁ à C₄) ou phényl-(alkyle en C₁ à C₄) portant chacun le cas échéant un substituant halogéno, cyano, nitro, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄ ou phényle éventuellement substitué par un radical halogéno ou alkyle en C₁ à C₄, ou le reste -S(O)ₙ¹R¹⁶,
R¹⁵ représente l'hydrogène, un groupe cyano, amino, un groupe alkyle en C₁ à C₄ ou alkoxy en C₁ à C₄ portant chacun le cas échéant un substituant fluoro, chloro, cyano, nitro, alkoxy en C₁ à C₄ ou amino, un groupe alcényle en C₃ à C₆, alcényloxy en C₃ à C₆, alcynyle en C₃ à C₆, alcynyloxy en C₃ à C₆, un groupe cycloalkyle en C₃ à C₆ ou (cycloalkyle en C₃ à C₆) - (alkyle en C₁ à C₄) portant chacun le cas échéant un substituant fluoro, chloro, bromo, alkyle en C₁ à C₄, alkoxy en C₁ à C₄, halogénalkyle en C₁ à C₄ ou halogénalkoxy en C₁ à C₄, un groupe phényle portant le cas échéant un substituant halogéno, cyano, nitro, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄ ou phényle éventuellement substitué par un halogène ou un radical alkyle en C₁ à C₄, un groupe benzyle éventuellement substitué par un radical alkyle en C₁ à C₄ ou l'un des restes -S(O)ₙ¹R¹⁶, -NR¹⁷R¹⁸ ou -N=CR¹⁹R²⁰,
R¹⁶ représente un groupe alkyle en C₁ à C₄ portant éventuellement un substituant fluoro, chloro, cyano ou nitro, un groupe alcényle en C₃ à C₆, un groupe alcanediényle en C₃ à C₆, un groupe alcynyle en C₃ à C₆, un groupe cycloalkyle en C₃ à C₆ portant éventuellement un substituant fluoro, chloro, bromo, alkyle en C₁ à C₄, alkoxy en C₁ à C₄, halogénalkyle en C₁ à C₄ ou halogénalkoxy en C₁ à C₄, ou un groupe phényle portant éventuellement un substituant halogéno, cyano, nitro, alkyle en C₁ à C₄, alkoxy en C₁ à C₄, alkylthio en C₁ à C₄, halogénalkyle en C₁ à C₄, halogénalkoxy en C₁ à C₄, halogénalkylthio en C₁ à C₄ ou phényle éventuellement substitué par un radical halogéno ou alkyle en C₁ à C₄,
n¹ représente l'un des nombres 0, 1 et 2,
R¹⁷ et R¹⁸ forment conjointement avec l'atome d'azote auquel ils sont liés un hétérocycle pentagonal à heptagonal saturé portant éventuellement un substituant halogéno, alkyle en C₁ à C₄, alkoxy en C₁ à C₄ ou alkylthio en C₁ à C₄ et ayant en plus de l'atome d'azote deux ou trois hétéroatomes identiques ou différents de la série azote, oxygène et soufre,
R¹⁹ représente l'hydrogène, un groupe alkyle en C₁ à C₄ portant éventuellement un substituant fluoro, chloro, cyano, nitro, amino ou alkoxy en C₁ à C₄, ou un groupe alkoxy en C₁ à C₄ et
R²⁰ représente l'hydrogène, un groupe alkyle en C₁ à C₄ portant éventuellement un substituant fluoro, chloro, cyano, nitro, amino ou alkoxy en C₁ à C₄, ou un groupe alkoxy en C₁ à C₄
à l'exception des composés

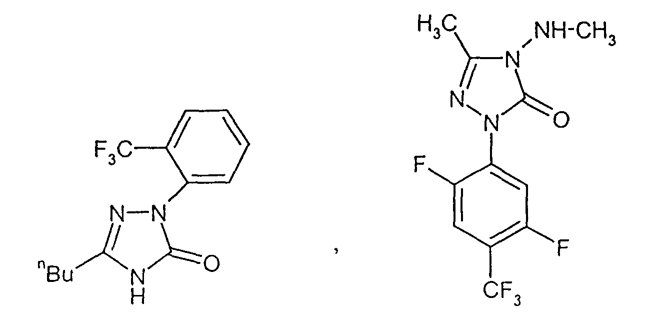

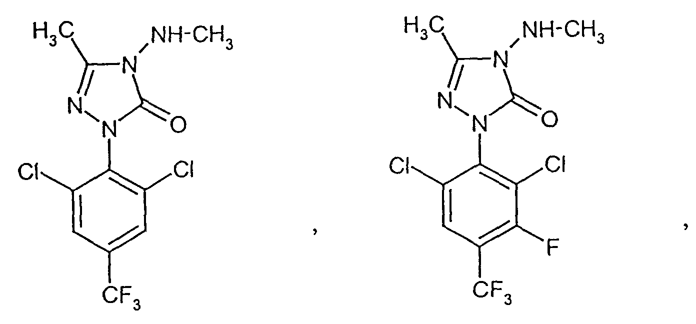

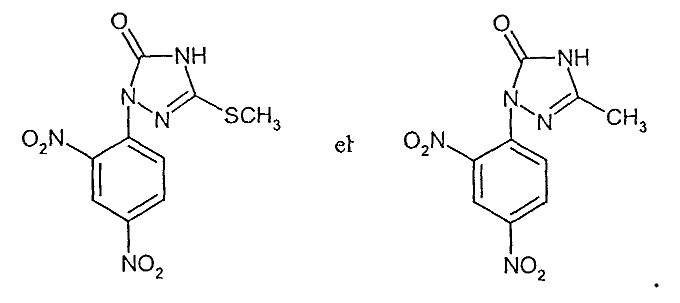

4. Composés de formule (Ia) suivant la revendication 1, dans laquelle
A¹ représente l'azote ou le groupe CR⁰,
R⁰ représente l'hydrogène, le fluor, le chlore, le brome, un groupe nitro, cyano, hydroxy, méthyle, éthyle, n-propyle, isopropyle, méthoxyméthyle, trifluorométhyle, difluorométhyle, chlorodifluorométhyle, dichlorofluorométhyle ou l'un des restes -CONH₂, -CSNH₂, -CONHCH₃, -CSNHCH₃, -CON(CH₃)₂ ou -CSN(CH₃)₂,
R¹¹ représente le fluor, le chlore, le brome, un groupe nitro, cyano, méthyle, éthyle, n-propyle, isopropyle, méthoxyméthyle, trifluorométhyle, difluorométhyle, chlorodifluorométhyle, dichlorofluorométhyle, trifluorométhoxy, difluorométhoxy, trifluoréthoxy ou l'un des restes -CONH₂, -CSNH₂, -CONHCH₃, -CSNHCH₃, -CON(CH₃)₂ et -CSN(CH₃)₂,
R¹² représente l'hydrogène, le fluor, le chlore, un groupe méthyle ou trifluorométhyle,
R¹³ est un groupe trifluorométhyle, difluorométhyle, chlorodifluorométhyle, dichlorofluorométhyle, trifluorométhoxy, difluorométhoxy, chlorodifluorométhoxy, trifluorométhylsulfényle, trifluorométhylsulfinyle ou trifluorométhylsulfonyle,
R¹⁴ représente le chlore, le brome, un groupe cyano, méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthoxyméthyle, méthoxyéthyle, éthoxyméthyle, éthoxyéthyle, trifluorométhyle, difluorométhyle, chlorodifluorométhyle, dichlorofluoro-méthyle, trifluoréthyle, tétrafluoréthyle, pentafluoréthyle, fluoropropyle, trifluorométhoxy, difluorométhoxy, chlorodifluorométhoxy, dichlorofluoro-méthoxy, trifluoréthoxy, tétrafluoréthoxy, pentafluoréthoxy, cyanométhyle, allyle, allyloxy, propargyle, hydroxy, mercapto, un groupe cyclopropyle, cyclopropylméthyle, cyclopentyle, cyclopentylméthyle, cyclohexyle ou cyclohexylméthyle dont chacun porte le cas échéant un substituant fluoro, chloro, bromo, méthyle, éthyle, méthoxy, trifluorométhyle ou trifluorométhoxy, un groupe phényle, phénoxyméthyle ou benzyle dont chacun porte le cas échéant un substituant fluoro, chloro, méthyle, méthoxy, trifluorométhyle, triflurométhoxy, méthylthio, trifluorométhylthio, nitro ou phényle éventuellement substitué par un radical fluoro, chloro ou méthyle, ou bien le reste -S(O)ₙ¹R¹⁶,
R¹⁵ représente l'hydrogène, un groupe cyano, amino, méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthoxyméthyle, méthoxyéthyle, éthoxyméthyle, éthoxyéthyle, difluorométhyle, chlorodifluorométhyle, dichlorofluorométhyle, trifluoréthyle, fluoropropyle, difluorométhoxy, chlorodifluorométhoxy, dichlorofluorométhoxy, cyanométhyle, allyle, allyloxy, propargyle, propargyloxy, butynyloxy, un groupe cyclopropyle, cyclopropylméthyle, cyclopentyle, cyclopentylméthyle, cyclohexyle ou cyclohexylméthyle dont chacun porte éventuellement un substituant fluoro, chloro, bromo, méthyle, éthyle, méthoxy, trifluorométhyle ou trifluorométhoxy, un groupe phényle portant éventuellement un substituant fluoro, chloro, méthyle, méthoxy, trifluorométhyle, trifluorométhoxy, méthylthio, triflurométhylthio, cyano, nitro ou phényle éventuellement substitué par un radical fluoro, chloro ou méthyle, un groupe benzyle portant éventuellement un substituant méthyle, éthyle, n-propyle, isopropyle, n-butyle, isobutyle, sec.-butyle ou tertiobutyle ou l'un des restes -S(O)ₙ¹R¹⁶, -NR¹⁷R¹⁸ ou -N=CR¹⁹R²⁰
R¹⁶ représente un groupe méthyle, éthyle, n-propyle, isopropyle, n-butyle, isobutyle, sec.-butyle, tertiobutyle, trifluorométhyle, difluorométhyle, chlorodifluorométhyle, dichlorofluorométhyle, trifluoréthyle, tétrafluoréthyle, pentafluoréthyle, fluoropropyle, allyle, allényle, propargyle, un groupe cyclopropyle, cyclopentyle ou cyclohexyle dont chacun porte éventuellement un substituant fluoro, chloro, bromo, méthyle, éthyle, méthoxy, trifluorométhyle ou trifluorométhoxy, ou bien un groupe phényle portant éventuellement un substituant fluoro, chloro, méthyle, méthoxy, trifluorométhyle, trifluorométhoxy, méthylthio, trifluorométhylthio, cyano, nitro ou phényle éventuellement substitué par un radical fluoro, chloro ou méthyle,
n¹ représente l'un des nombres 0, 1 et 2,
R¹⁷ et R¹⁸ forment conjointement avec l'atome d'azote auquel ils sont liés un groupe pipéridinyle, pipérazinyle ou morpholinyle dont chacun porte éventuellement un substituant méthyle, éthyle, méthoxy ou éthoxy,
R¹⁹ représente l'hydrogène, un groupe méthyle, éthyle, n-propyle, isopropyle, n-butyle, isobutyle, sec.-butyle, tertiobutyle, méthoxy ou éthoxy et
R²⁰ représente l'hydrogène, un groupe méthyle, éthyle, n-propyle, isopropyle, n-butyle, isobutyle, sec.-butyle, tertiobutyle, méthoxy ou éthoxy,
à l'exception des composés

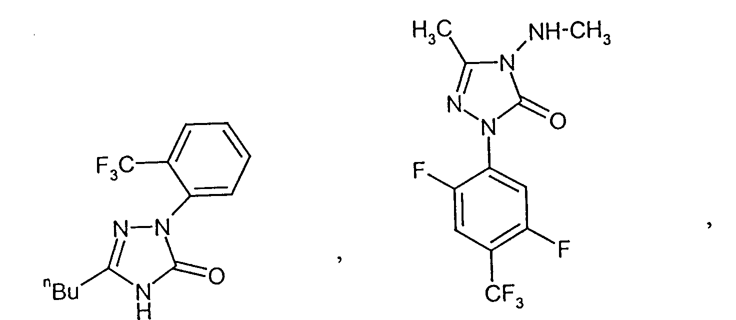

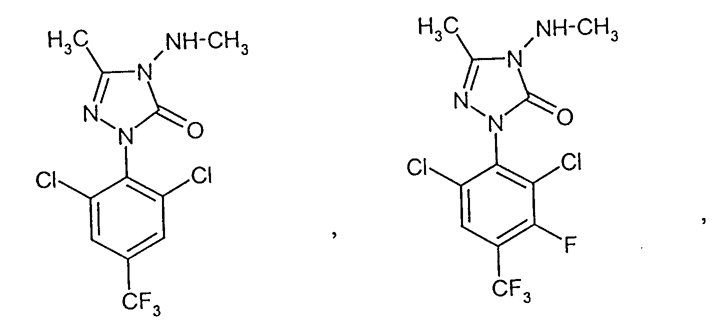

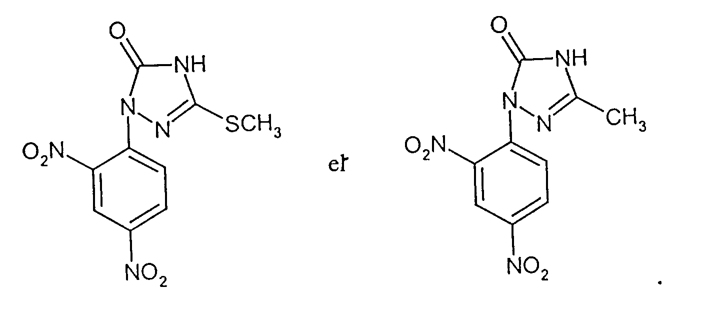

5. Compositions destinées à combattre des parasites animaux, caractérisées par une teneur en au moins un composé de formule (Ia) suivant la revendication 1.

6. Procédé pour combattre des parasites animaux, **caractérisé en ce qu**'on fait agir des composés de formule (Ia) suivant la revendication 1 sur les parasites et/ou sur leur milieu.

7. Utilisation de composés de formule (Ia) suivant la revendication 1 pour combattre des parasites animaux.

8. Procédé de préparation de compositions pesticides, **caractérisé en ce qu**'on mélange des composés de formule (Ia) suivant la revendication 1 avec des diluants et/ou des agents tensioactifs.

9. Utilisation de composés de formule (Ia) suivant la revendication 1 pour la préparation de compositions pesticides.
